# EUROPEAN PATENT SPECIFICATION

(11) **EP 4 349 821 B1**
(45) Date of publication and mention of the grant of the patent: **08.04.2026**
(21) Application number: 22811439.3
(22) Date of filing: 13.01.2022
(51) Int. Cl.: C07B 59/00, C07D 307/77, C07D 407/12, C07D 407/04, C07D 409/04, H10K 50/11, H10K 50/15, H10K 50/16, H10K 50/17, H10K 50/18, H10K 85/60

(54) **HETEROCYCLIC COMPOUND AND ORGANIC LIGHT-EMITTING DEVICE COMPRISING SAME**
HETEROCYCLISCHE VERBINDUNG UND ORGANISCHE LICHTEMITTIERENDE VORRICHTUNG DAMIT
COMPOSÉ HÉTÉROCYCLIQUE ET DISPOSITIF ÉLECTROLUMINESCENT ORGANIQUE LE COMPRENANT

(30) Priority: 24.05.2021 KR 20210066142
(43) Date of publication of application: 10.04.2024
(73) Proprietor: LT Materials Co., Ltd., Yongin-si, Gyeonggi-do 17118 (KR)
(72) Inventor: LEE, Dong-Jin, Yongin-si, Gyeonggi-do 17118 (KR); KIM, Ji-Un, Yongin-si, Gyeonggi-do 17118 (KR); JEONG, Won-Jang, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2022/000610
(87) International publication number: WO 2022/250244

(56) References cited:
- KR-A- 20210 023 773
- KR-B1- 101 789 998
- KR-B1- 102 076 958
- KR-B1- 102 228 768
- US-A1- 2018 208 836

## Description

### [Technical Field]

The present specification relates to a heterocyclic compound and an organic light emitting device comprising the same.

This application claims priority to and the benefit of Korean Patent Application No. 10-2021-0066142 filed in the Korean Intellectual Property Office on May 24, 2021.

### [Background Art]

An organic electroluminescent device is a kind of self-emitting type display device, and has an advantage in that the viewing angle is wide, the contrast is excellent, and the response speed is fast.

An organic light emitting device has a structure in which an organic thin film is disposed between two electrodes. When a voltage is applied to an organic light emitting device having the structure, electrons and holes injected from the two electrodes combine with each other in an organic thin film to make a pair, and then, emit light while being extinguished. The organic thin film may be composed of a single layer or multi layers, if necessary.

A material for the organic thin film may have a light emitting function, if necessary. For example, as the material for the organic thin film, it is also possible to use a compound, which may itself constitute a light emitting layer alone, or it is also possible to use a compound, which may serve as a host or a dopant of a host-dopant-based light emitting layer. In addition, as a material for the organic thin film, it is also possible to use a compound, which may perform a function such as hole injection, hole transport, electron blocking, hole blocking, electron transport or electron injection.

In order to improve the performance, service life, or efficiency of the organic light emitting device, there is a continuous need for developing a material for an organic thin film.

It is necessary to perform studies on an organic light emitting device comprising a compound having a chemical structure, which may satisfy conditions required for a material which is available for the organic light emitting device, for example, appropriate energy levels, electrochemical stability, thermal stability, and the like, and may perform various functions required for the organic light emitting device according to the substituent.

KR Patent no 102076958B1 discloses compounds which corresponds with compound of formula 1 and their use in organic light emitting devices.

KR Patent no 102228768B1 discloses compounds which corresponds with compound of formula 1 and their use in organic light emitting devices.

### [Disclosure]

### [Technical Problem]

The present application relates to a heterocyclic compound and an organic light emitting device comprising the same.

### [Technical Solution]

In an exemplary embodiment of the present application, provided is a heterocyclic compound represented by the following Chemical Formula 1.

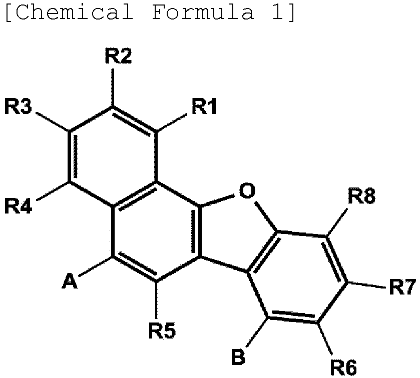

In Chemical Formula 1,
R1 to R8 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; and -SiRR'R", or two or more adjacent groups are bonded to each other to form a substituted or unsubstituted C6 to C60 aliphatic or aromatic hydrocarbon ring, or a substituted or unsubstituted C2 to C60 aliphatic or aromatic hetero ring,
A and B are each independently represented by the following Chemical Formula 1-1; or the following Chemical Formula 1-2, 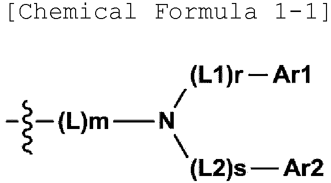 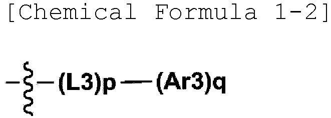
in Chemical Formulae 1-1 and 1-2,
L and L1 to L3 are the same as or different from each other, and are each independently a direct bond; a substituted or unsubstituted C6 to C60 arylene group; or a substituted or unsubstituted C2 to C60 heteroarylene group,
Ar1 to Ar3 are the same as or different from each other, and are each independently a substituted or unsubstituted C1 to C60 alkyl group; a substituted or unsubstituted C6 to C60 aryl group; a substituted or unsubstituted C2 to C60 heteroaryl group; -P(=O)RR'; or - SiRR'R",
p, m, r and s are each an integer from 0 to 4,
q is an integer from 1 to 6,
when p, m, r, s and q are each 2 or higher, substituents in the parenthesis are the same as or different from each other,
a deuterium content of the heterocyclic compound of Chemical Formula 1 is 1% to 100%, and
R, R' and R" are the same as or different from each other, and are each independently a substituted or unsubstituted C1 to C60 alkyl group; a substituted or unsubstituted C6 to C60 aryl group; or a substituted or unsubstituted C2 to C60 heteroaryl group.

Further, according to an exemplary embodiment of the present application, provided is an organic light emitting device comprising: a first electrode; a second electrode provided to face the first electrode; and an organic material layer having one or more layers provided between the first electrode and the second electrode, in which one or more layers of the organic material layer comprise the heterocyclic compound represented by Chemical Formula 1.

### [Advantageous Effects]

The compound described in the present specification can be used as a material for the organic material layer of the organic light emitting device. The compound can serve as a hole injection material, a hole transport material, a light emitting material, an electron transport material, an electron injection material, an electron blocking material, a hole blocking material, and the like in an organic light emitting device. In particular, the compound can be used as a light emitting auxiliary material or hole transport material for an organic light emitting device.

In addition, when the heterocyclic compound represented by Chemical Formula 1 is used for an organic light emitting device, the driving voltage of the device can be lowered, the light efficiency of the device can be improved, and the service life characteristics of the device can be improved due to the thermal stability of the compound.

The deuterium content of the heterocyclic compound of the present invention satisfies 1% to 100%, and since the single bond dissociation energy of carbon and deuterium is higher than the single bond dissociation energy of carbon and hydrogen, the stability of the total molecules is enhanced as the deuterium content of the heterocyclic compound of Chemical Formula 1 according to the present application satisfies the above range, so that there is an effect that the service life of the device is improved.

### [Description of Drawings]

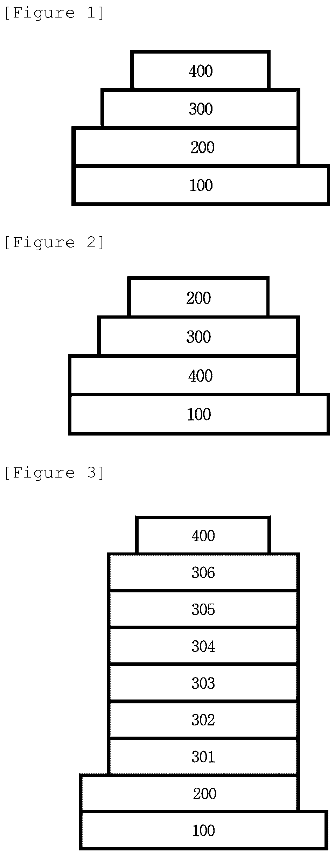

FIGS. 1 to 4 each are views schematically illustrating a stacking structure of an organic light emitting device according to an exemplary embodiment of the present application.

### [Explanation of Reference Numerals and Symbols]

- 100:: Substrate
- 200:: Positive electrode
- 300:: Organic material layer
- 301:: Hole injection layer
- 302:: Hole transport layer
- 303:: Light emitting layer
- 304:: Hole blocking layer
- 305:: Electron transport layer
- 306:: Electron injection layer
- 307:: Light emitting auxiliary layer
- 400:: Negative electrode

### [Best Mode]

Hereinafter, the present application will be described in detail.

In the present specification, "when a substituent is not indicated in the structure of a chemical formula or compound" means that a hydrogen atom is bonded to a carbon atom. However, since deuterium (²H) is an isotope of hydrogen, some hydrogen atoms may be deuterium.

In an exemplary embodiment of the present application, "when a substituent is not indicated in the structure of a chemical formula or compound" may mean that all the positions that may be reached by the substituent are hydrogen or deuterium. That is, deuterium is an isotope of hydrogen, and some hydrogen atoms may be deuterium which is an isotope, and in this case, the content of deuterium may be 0% to 100%.

In an exemplary embodiment of the present application, in "the case where a substituent is not indicated in the structure of a chemical formula or compound", when the content of deuterium is 0%, the content of hydrogen is 100%, and all the substituents do not explicitly exclude deuterium such as hydrogen, hydrogen and deuterium may be mixed and used in the compound.

In an exemplary embodiment of the present application, deuterium is one of the isotopes of hydrogen, is an element that has a deuteron composed of one proton and one neutron as a nucleus, and may be represented by hydrogen-2, and the element symbol may also be expressed as D or ²H.

In an exemplary embodiment of the present application, the isotope means an atom with the same atomic number (Z), but different mass numbers (A), and the isotope may be interpreted as an element which has the same number of protons, but different number of neutrons.

In an exemplary embodiment of the present application, when the total number of substituents of a basic compound is defined as T1 and the number of specific substituents among the substituents is defined as T2, the meaning of the content T% of the specific substituent may be defined as T2/T1×100 = T%.

That is, in an example, the deuterium content of 20% in a phenyl group represented by 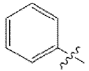 may be represented by 20% when the total number of substituents that the phenyl group can have is 5 (T1 in the formula) and the number of deuteriums among the substituents is 1 (T2 in the formula). That is, a deuterium content of 20% in the phenyl group may be represented by the following structural formula.

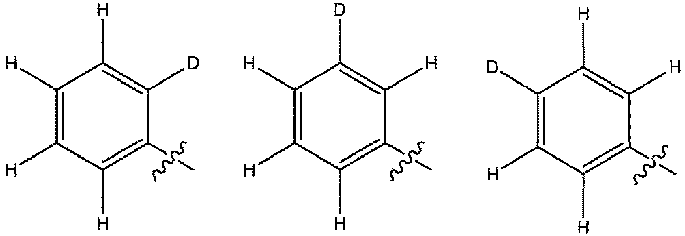

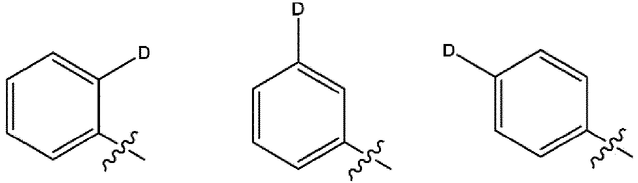

Further, in an exemplary embodiment of the present application, "a phenyl group having a deuterium content of 0%" may mean a phenyl group that does not comprise a deuterium atom, that is, has five hydrogen atoms.

In the present specification, the halogen may be fluorine, chlorine, bromine or iodine.

In the present specification, the alkyl group comprises a straight-chain or branched-chain having 1 to 60 carbon atoms, and may be additionally substituted with another substituent. The number of carbon atoms of the alkyl group may be 1 to 60, specifically 1 to 40, and more specifically 1 to 20. Specific examples thereof comprise a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like, but are not limited thereto.

In the present specification, the alkenyl group comprises a straight-chain or branched-chain having 2 to 60 carbon atoms, and may be additionally substituted with another substituent. The number of carbon atoms of the alkenyl group may be 2 to 60, specifically 2 to 40, and more specifically 2 to 20. Specific examples thereof comprise a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like, but are not limited thereto.

In the present specification, the alkynyl group comprises a straight-chain or branched-chain having 2 to 60 carbon atoms, and may be additionally substituted with another substituent. The number of carbon atoms of the alkynyl group may be 2 to 60, specifically 2 to 40, and more specifically 2 to 20.

In the present specification, an alkoxy group may be straight-chained, branched, or cyclic. The number of carbon atoms of the alkoxy group is not particularly limited, but is preferably 1 to 20. Specific examples thereof comprise methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and the like, but are not limited thereto.

In the present specification, the cycloalkyl group comprises a monocycle or polycycle having 3 to 60 carbon atoms, and may be additionally substituted with another substituent. Here, the polycycle means a group in which a cycloalkyl group is directly linked to or fused with another cyclic group. Here, another cyclic group may also be a cycloalkyl group, but may also be another kind of cyclic group, for example, a heterocycloalkyl group, an aryl group, a heteroaryl group, and the like. The number of carbon atoms of the cycloalkyl group may be 3 to 60, specifically 3 to 40, and more specifically 5 to 20. Specific examples thereof comprise a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, and the like, but are not limited thereto.

In the present specification, the heterocycloalkyl group comprises O, S, Se, N, or Si as a heteroatom, comprises a monocycle or polycycle having 2 to 60 carbon atoms, and may be additionally substituted with another substituent. Here, the polycycle means a group in which a heterocycloalkyl group is directly linked to or fused with another cyclic group. Here, another cyclic group may also be a heterocycloalkyl group, but may also be another kind of cyclic group, for example, a cycloalkyl group, an aryl group, a heteroaryl group, and the like. The number of carbon atoms of the heterocycloalkyl group may be 2 to 60, specifically 2 to 40, and more specifically 3 to 20.

In the present specification, the aryl group comprises a monocycle or polycycle having 6 to 60 carbon atoms, and may be additionally substituted with another substituent. Here, the polycycle means a group in which an aryl group is directly linked to or fused with another cyclic group. Here, another cyclic group may also be an aryl group, but may also be another kind of cyclic group, for example, a cycloalkyl group, a heterocycloalkyl group, a heteroaryl group, and the like. The number of carbon atoms of the aryl group may be 6 to 60, specifically 6 to 40, and more specifically 6 to 25. Specific examples of the aryl group comprise a phenyl group, a biphenyl group, a terphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, an indenyl group, an acenaphthylenyl group, a 2,3-dihydro-1H-indenyl group, a fused cyclic group thereof, and the like, but are not limited thereto.

In the present specification, a fluorenyl group may be substituted, and adjacent substituents may be bonded to each other to form a ring.

When the fluorenyl group is substituted, the substituent may be the following structure, but is not limited thereto.

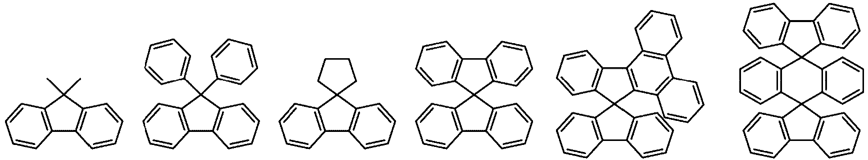

In the present specification, the heteroaryl group comprises S, O, Se, N, or Si as a heteroatom, comprises a monocycle or polycycle having 2 to 60 carbon atoms, and may be additionally substituted with another substituent. Here, the polycycle means a group in which a heteroaryl group is directly linked to or fused with another cyclic group. Here, another cyclic group may also be a heteroaryl group, but may also be another kind of cyclic group, for example, a cycloalkyl group, a heterocycloalkyl group, an aryl group, and the like. The number of carbon atoms of the heteroaryl group may be 2 to 60, specifically 2 to 40, and more specifically 3 to 25. Specific examples of the heteroaryl group comprise a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a quinozolilyl group, a naphthyridyl group, an acridinyl group, a phenanthridinyl group, an imidazopyridinyl group, a diaza naphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi (dibenzosilole) group, a dihydrophenazinyl group, a phenoxazinyl group, a phenanthridyl group, a thienyl group, an indolo[2,3-a]carbazolyl group, an indolo[2,3-b]carbazolyl group, an indolinyl group, a 10,11-dihydro-dibenzo[b,f]azepin group, a 9,10-dihydroacridinyl group, a phenanthrazinyl group, a phenothiazinyl group, a phthalazinyl group, a naphthylidinyl group, a phenanthrolinyl group, a benzo[c][1,2,5]thiadiazolyl group, a 5,10-dihydrodibenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group, and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and the number of carbon atoms thereof is not particularly limited, but is preferably 1 to 30. Specific examples of the amine group comprise a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, and the like, but are not limited thereto.

In the present specification, an arylene group means that there are two bonding positions in an aryl group, that is, a divalent group. The above-described description on the aryl group may be applied to the arylene group, except that the arylene groups are each a divalent group. Further, a heteroarylene group means that there are two bonding positions in a heteroaryl group, that is, a divalent group. The above-described description on the heteroaryl group may be applied to the heteroarylene group, except for a divalent heteroarylene group.

In the present specification, a phosphine oxide group is represented by -P(=O) (R101) (R102), and R101 and R102 are the same as or different from each other, and may be each independently a substituent composed of at least one of hydrogen; deuterium; a halogen group; an alkyl group; an alkenyl group; an alkoxy group; a cycloalkyl group; an aryl group; and a heterocyclic group. Specific examples of the phosphine oxide group comprise a diphenylphosphine oxide group, dinaphthylphosphine oxide group, and the like, but are not limited thereto.

In the present specification, a silyl group comprises Si and is a substituent to which the Si atom is directly linked as a radical, and is represented by - Si(R104) (R105) (R106), and R104 to R106 are the same as or different from each other, and may be each independently a substituent composed of at least one of hydrogen; deuterium; a halogen group; an alkyl group; an alkenyl group; an alkoxy group; a cycloalkyl group; an aryl group; and a heterocyclic group. Specific examples of the silyl group comprise a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but are not limited thereto.

In the present specification, the "adjacent" group may mean a substituent substituted with an atom directly linked to an atom in which the corresponding substituent is substituted, a substituent disposed to be sterically closest to the corresponding substituent, or another substituent substituted with an atom in which the corresponding substituent is substituted. For example, two substituents substituted at the ortho position in a benzene ring and two substituents substituted with the same carbon in an aliphatic ring may be interpreted as groups which are "adjacent" to each other.

Structures exemplified by the above-describe cycloalkyl group, aryl group, cycloheteroalkyl group and heteroaryl group may be applied, except that an aliphatic or aromatic hydrocarbon ring, or an aliphatic or aromatic hetero ring which adjacent groups may form is not a monovalent group.

In the present specification, the term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more substituents are substituted, the two or more substituents may be the same as or different from each other.

In the present specification, "substituted or unsubstituted" means being substituted with one or more substituents selected from the group consisting of deuterium; a cyano group; a halogen group; a C1 to C60 straight-chained or branched alkyl; a C2 to C60 straight-chained or branched alkenyl; a C2 to C60 straight-chained or branched alkynyl; a C3 to C60 monocyclic or polycyclic cycloalkyl; a C2 to C60 monocyclic or polycyclic heterocycloalkyl; a C6 to C60 monocyclic or polycyclic aryl; a C2 to C60 monocyclic or polycyclic heteroaryl; -SiRR'R"; -P(=O)RR'; a C1 to C20 alkylamine; a C6 to C60 monocyclic or polycyclic arylamine; and a C2 to C60 monocyclic or polycyclic heteroarylamine, or unsubstituted or substituted with a substituent to which two or more substituents selected among the exemplified substituents are linked, and R, R' and R" are the same as or different from each other, and are each independently a substituted or unsubstituted C1 to C60 alkyl group; a substituted or unsubstituted C6 to C60 aryl group; or a substituted or unsubstituted C2 to C60 heteroaryl group.

In an exemplary embodiment of the present application, provided is the heterocyclic compound represented by Chemical Formula 1.

In an exemplary embodiment of the present application, Chemical Formula 1 may be represented by the following Chemical Formula 2 or the following Chemical Formula 3.

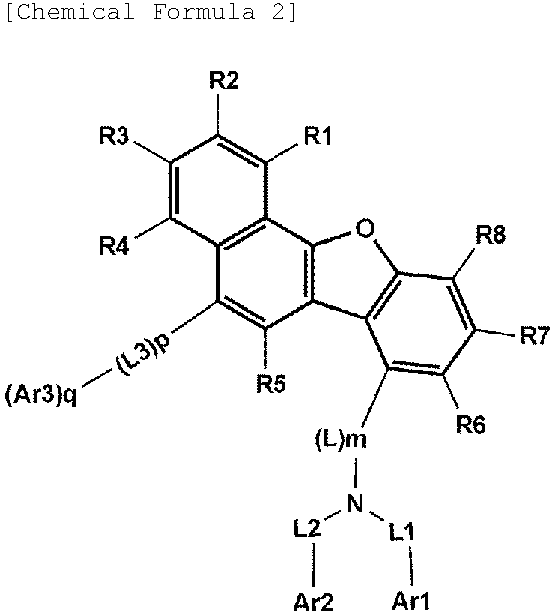

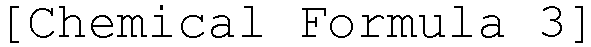

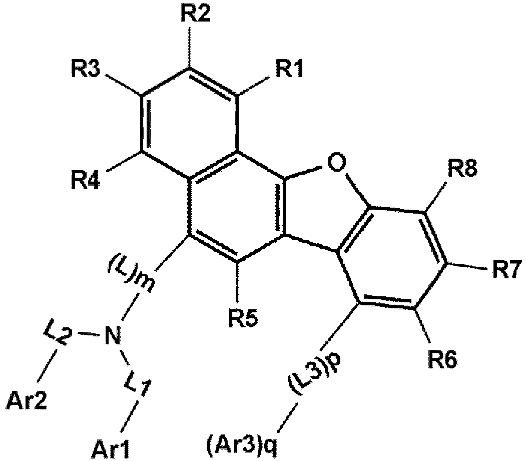

In Chemical Formulae 2 and 3,
the definition of each substituent is the same as the definition in Chemical Formula 1.

In an exemplary embodiment of the present application, R1 to R8 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; and -SiRR'R", or two or more adjacent groups may be bonded to each other to form a substituted or unsubstituted C6 to C60 aliphatic or aromatic hydrocarbon ring, or a substituted or unsubstituted C2 to C60 aliphatic or aromatic hetero ring.

In another exemplary embodiment, R1 to R8 are the same as or different from each other, and are each independently selected from the group consisting of hydrogen; deuterium; a substituted or unsubstituted C1 to C60 alkyl group; a substituted or unsubstituted C6 to C60 aryl group; a substituted or unsubstituted C2 to C60 heteroaryl group; -P(=O)RR'; and -SiRR'R", or two or more adjacent groups may be bonded to each other to form a substituted or unsubstituted C6 to C60 aliphatic or aromatic hydrocarbon ring, or a substituted or unsubstituted C2 to C60 aliphatic or aromatic hetero ring.

In still another exemplary embodiment, R1 to R8 are the same as or different from each other, and may be each independently selected from the group consisting of hydrogen; deuterium; a substituted or unsubstituted C1 to C40 alkyl group; a substituted or unsubstituted C6 to C40 aryl group; a substituted or unsubstituted C2 to C40 heteroaryl group; -P(=O)RR'; and -SiRR'R".

In yet another exemplary embodiment, R1 to R8 are the same as or different from each other, and may be each independently selected from the group consisting of hydrogen; deuterium; a C1 to C40 alkyl group; a C6 to C40 aryl group; a C2 to C40 heteroaryl group; -P(=O)RR'; and - SiRR'R".

In yet another exemplary embodiment, R1 to R8 are the same as or different from each other, and may be each independently hydrogen; or deuterium.

In an exemplary embodiment of the present application, L and L1 to L3 are the same as or different from each other, and may be each independently a direct bond; a substituted or unsubstituted C6 to C60 arylene group; or a substituted or unsubstituted C2 to C60 heteroarylene group.

In another exemplary embodiment, L and L1 to L3 are the same as or different from each other, and may be each independently a direct bond; a substituted or unsubstituted C6 to C40 arylene group; or a substituted or unsubstituted C2 to C40 heteroarylene group.

In still another exemplary embodiment, L and L1 to L3 are the same as or different from each other, and may be each independently a direct bond; or a substituted or unsubstituted C6 to C40 arylene group.

In yet another exemplary embodiment, L and L1 to L3 are the same as or different from each other, and may be each independently a direct bond; or a substituted or unsubstituted C6 to C20 arylene group.

In yet another exemplary embodiment, L and L1 to L3 are the same as or different from each other, and may be each independently a direct bond; or a C6 to C20 arylene group which is unsubstituted or substituted with deuterium.

In yet another exemplary embodiment, L and L1 to L3 are the same as or different from each other, and may be each independently a direct bond; a C6 to C10 monocyclic arylene group which is unsubstituted or substituted with deuterium; or a C10 to C20 polycyclic arylene group which is unsubstituted or substituted with deuterium.

In yet another exemplary embodiment, L and L1 to L3 are the same as or different from each other, and may be each independently a direct bond; a phenylene group which is unsubstituted or substituted with deuterium; or a biphenylene group which is unsubstituted or substituted with deuterium.

In an exemplary embodiment of the present application, Ar1 to Ar3 are the same as or different from each other, and may be each independently a substituted or unsubstituted C1 to C60 alkyl group; a substituted or unsubstituted C6 to C60 aryl group; a substituted or unsubstituted C2 to C60 heteroaryl group; -P(=O)RR'; or - SiRR'R".

In another exemplary embodiment, Ar1 to Ar3 are the same as or different from each other, and may be each independently a substituted or unsubstituted C6 to C60 aryl group; or a substituted or unsubstituted C2 to C60 heteroaryl group.

In still another exemplary embodiment, Ar1 to Ar3 are the same as or different from each other, and may be each independently a substituted or unsubstituted C6 to C40 aryl group; or a substituted or unsubstituted C2 to C40 heteroaryl group.

In yet another exemplary embodiment, Ar1 to Ar3 are the same as or different from each other, and may be each independently a substituted or unsubstituted phenyl group; a substituted or unsubstituted biphenyl group; a substituted or unsubstituted naphthyl group; a substituted or unsubstituted terphenyl group; a substituted or unsubstituted dimethyl fluorenyl group; a substituted or unsubstituted spirobifluorenyl group; a substituted or unsubstituted diphenyl fluorenyl group; a substituted or unsubstituted triphenylenyl group; a substituted or unsubstituted phenanthrenyl group; a substituted or unsubstituted dibenzofuran group; or a substituted or unsubstituted dibenzothiophene group.

In yet another exemplary embodiment, Ar1 to Ar3 are the same as or different from each other, and may be each independently a C6 to C40 aryl group which is unsubstituted or substituted with one or more substituents selected from the group consisting of deuterium, a C1 to C40 alkyl group and a C6 to C40 aryl group; a substituted or unsubstituted spirobifluorenyl group; or a C2 to C40 heteroaryl group which is unsubstituted or substituted with deuterium.

In yet another exemplary embodiment, Ar1 to Ar3 are the same as or different from each other, and may be each independently a C6 to C40 aryl group which is unsubstituted or substituted with one or more substituents selected from the group consisting of deuterium, a C1 to C40 alkyl group and a C6 to C40 aryl group; or a C2 to C40 heteroaryl group which is unsubstituted or substituted with deuterium.

In yet another exemplary embodiment, Ar1 to Ar3 are the same as or different from each other, and may be each independently a C6 to C20 aryl group which is unsubstituted or substituted with one or more substituents selected from the group consisting of deuterium, a C1 to C20 alkyl group and a C6 to C20 aryl group; or a C2 to C20 heteroaryl group which is unsubstituted or substituted with deuterium.

In yet another exemplary embodiment, Ar1 to Ar3 are the same as or different from each other, and may be each independently a phenyl group which is unsubstituted or substituted with deuterium; a biphenyl group which is unsubstituted or substituted with deuterium; a naphthyl group which is unsubstituted or substituted with deuterium; a terphenyl group which is unsubstituted or substituted with deuterium; a dimethyl fluorenyl group which is unsubstituted or substituted with deuterium; a spirobifluorenyl group which is unsubstituted or substituted with deuterium; a diphenyl fluorenyl group which is unsubstituted or substituted with deuterium; a triphenylenyl group which is unsubstituted or substituted with deuterium; a phenanthrenyl group which is unsubstituted or substituted with deuterium; a dibenzofuran group which is unsubstituted or substituted with deuterium; or a dibenzothiophene group which is unsubstituted or substituted with deuterium.

In an exemplary embodiment of the present application, the deuterium content of the heterocyclic compound of Chemical Formula 1 may be 1% to 100%.

In another exemplary embodiment, the deuterium content of the heterocyclic compound of Chemical Formula 1 may be 1% to 100%, preferably 5% to 90%, and more preferably 10% to 80%.

In an exemplary embodiment of the present application, the deuterium content of the heterocyclic compound of Chemical Formula 1 satisfies the above range, the photochemical characteristics of a compound which comprises deuterium and a compound which does not comprise deuterium are almost similar, but when deposited on a thin film, the deuterium-containing material tends to be packed with a narrower intermolecular distance.

Accordingly, when an electron only device (EOD) and a hole only device (HOD) are manufactured and the current density thereof according to voltage is confirmed, it can be confirmed that the compound of Chemical Formula 1 according to the present application, which comprises deuterium, exhibits a much more balanced charge transport characteristics than the compound which does not comprise deuterium.

Further, when the surface of a thin film is observed using an atomic force microscope (AFM), it can be confirmed that the thin film made of a compound comprising deuterium is deposited with a more uniform surface without any aggregated portion.

Additionally, since the single bond dissociation energy of carbon and deuterium is higher than the single bond dissociation energy of carbon and hydrogen, the stability of the total molecules is enhanced as the deuterium content of the heterocyclic compound of Chemical Formula 1 according to the present application satisfies the above range, so that there is an effect that the service life of the device is improved.

In an exemplary embodiment of the present application, Chemical Formula 1 may be expressed while being divided into the units of the following Structural Formulae A to C.

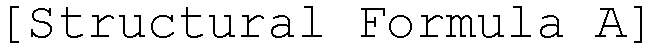

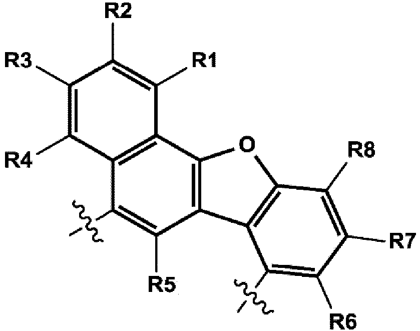

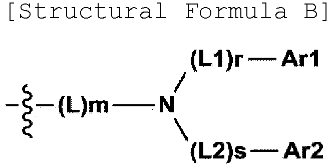

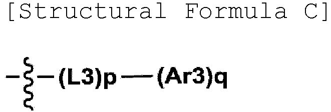

In Structural Formulae A to C, the definition of each substituent is the same as the definition in Chemical Formula 1, and 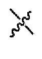 may mean a position at which Structural Formulae A to C are linked to each other.

In an exemplary embodiment of the present application, the deuterium content of at least one of Structural Formula A; Structural Formula B; and Structural Formula C in Chemical Formula 1 may be 1% to 100%.

In an exemplary embodiment of the present application, the deuterium content of at least one of Structural Formula A; Structural Formula B; and Structural Formula C in Chemical Formula 1 may be 50% to 100%.

In an exemplary embodiment of the present application, the deuterium content of Structural Formula A; Structural Formula B; Structural Formula C; Structural Formulae A and B; Structural Formulae A and C; Structural Formulae B and C; or Structural Formulae A to C in Chemical Formula 1 may be 1% to 100%.

In an exemplary embodiment of the present application, the deuterium content of Structural Formula A; Structural Formula B; Structural Formula C; Structural Formulae A and B; Structural Formulae A and C; Structural Formulae B and C; or Structural Formulae A to C in Chemical Formula 1 may be 100%.

In an exemplary embodiment of the present application, the deuterium content of Structural Formula A in Chemical Formula 1 may be 0%.

In an exemplary embodiment of the present application, the deuterium content of Structural Formula A in Chemical Formula 1 may be 100%.

In an exemplary embodiment of the present application, the deuterium content of Structural Formula A in Chemical Formula 1 may be 0% to 100%.

In an exemplary embodiment of the present application, the deuterium content of Structural Formula A in Chemical Formula 1 may be 1% to 100%.

In an exemplary embodiment of the present application, the deuterium content of Structural Formula B in Chemical Formula 1 may be 0%.

In an exemplary embodiment of the present application, the deuterium content of Structural Formula B in Chemical Formula 1 may be 100%.

In an exemplary embodiment of the present application, the deuterium content of Structural Formula B in Chemical Formula 1 may be 0% to 100%.

In an exemplary embodiment of the present application, the deuterium content of Structural Formula B in Chemical Formula 1 may be 1% to 100%.

In an exemplary embodiment of the present application, the deuterium content of Structural Formula C in Chemical Formula 1 may be 0%.

In an exemplary embodiment of the present application, the deuterium content of Structural Formula C in Chemical Formula 1 may be 100%.

In an exemplary embodiment of the present application, the deuterium content of Structural Formula C in Chemical Formula 1 may be 1% to 100%.

In an exemplary embodiment of the present application, the deuterium content of Structural Formulae A to C in Chemical Formula 1 may be 100%.

In an exemplary embodiment of the present application, the deuterium content of Structural Formula A; Structural Formula B; or Structural Formula C in Chemical Formula 1 may be 100%.

In an exemplary embodiment of the present application, the deuterium content of Structural Formulae A and B; Structural Formulae A and C; or Structural Formulae B and C in Chemical Formula 1 may be 100%.

In an exemplary embodiment of the present application, the deuterium content of Structural Formula A; Structural Formula B; and Structural Formula C in Chemical Formula 1 may be 100%.

The deuterium content of Structural Formulae A to C may mean a ratio of a position capable of having a substituent with respect to the entire structure of Structural Formulae A to C to be substituted with deuterium, and comprises a case where two or more substituents are linked.

In another expression, the content of deuterium of Structural Formulae A to C may mean the ratio of hydrogen substituted with deuterium in the entire structure of Structural Formulae A to C.

As an example, the fact that the deuterium content of Structural Formula A is 100% may mean that in Structural Formula A, R1 to R8 are all deuterium, or in Structural Formula A, when R1 to R8 have different substituents, all the positions capable of being substituted with the substituent are substituted with deuterium.

In an exemplary embodiment of the present application, Chemical Formula 1 may be expressed while being divided into the structures of Structural Formulae A to C, and in Structural Formulae A to C, the definition of each substituent is the same as the definition in Chemical Formula 1, 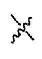 means a position at which Structural Formulae A to C are linked to each other, and is provided a heterocyclic compound in which the deuterium content of Structural Formula A; Structural Formula B; Structural Formula C; Structural Formulae A and B; Structural Formulae A and C; or Structural Formulae B and C in Chemical Formula 1 is 100%.

According to an exemplary embodiment of the present application, Chemical Formula 1 may be represented by any one of the following compounds, but is not limited thereto.

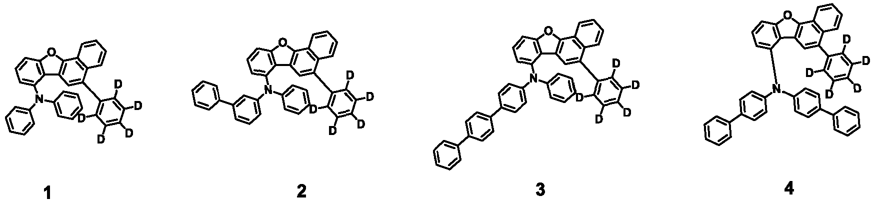

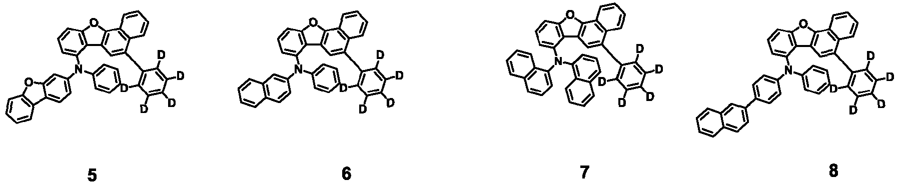

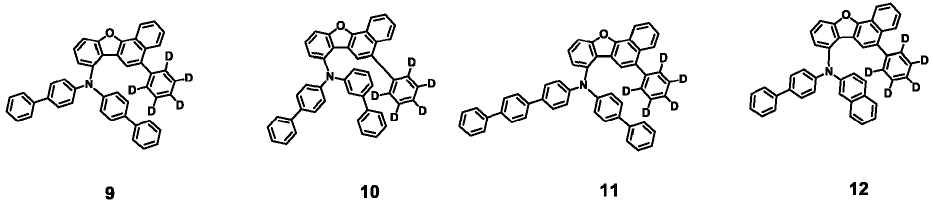

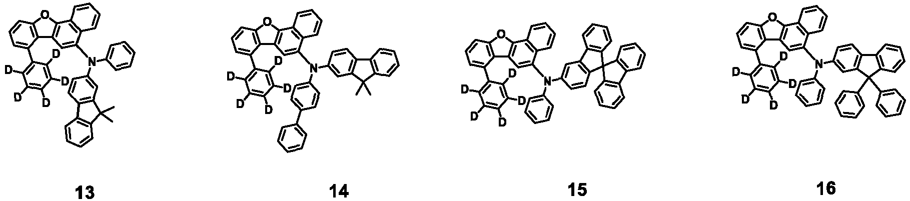

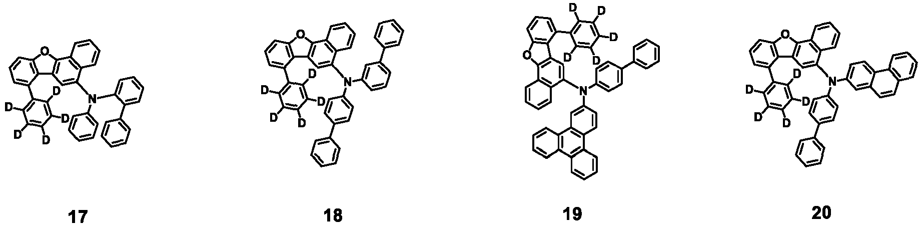

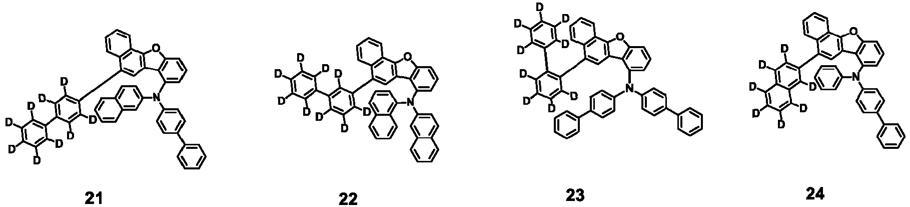

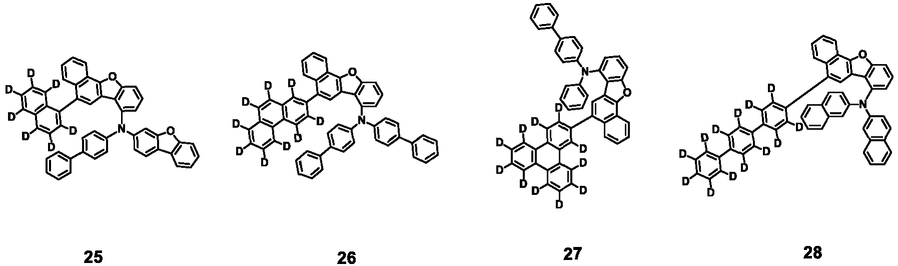

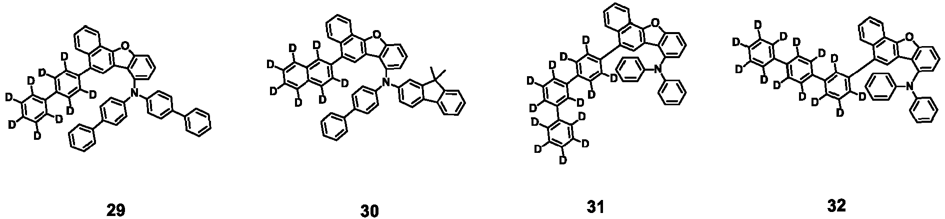

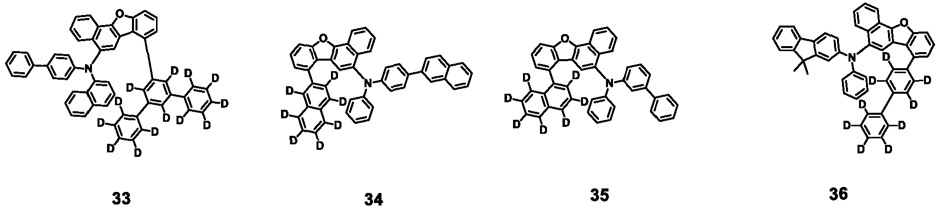

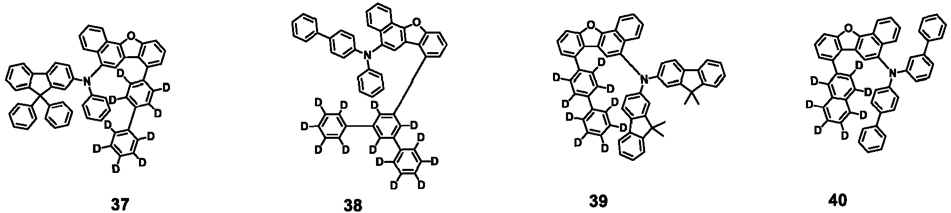

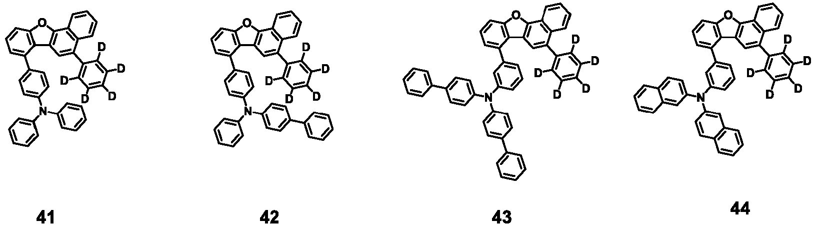

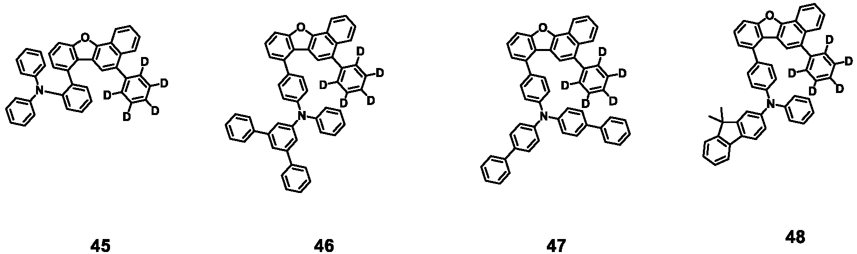

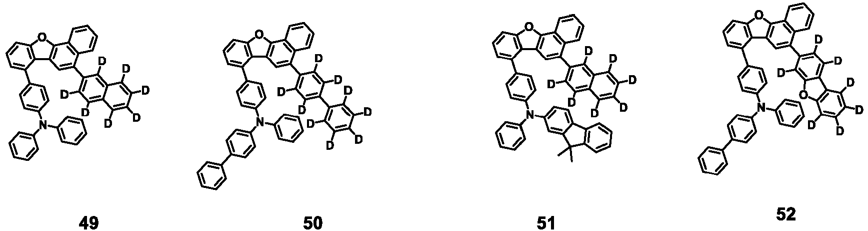

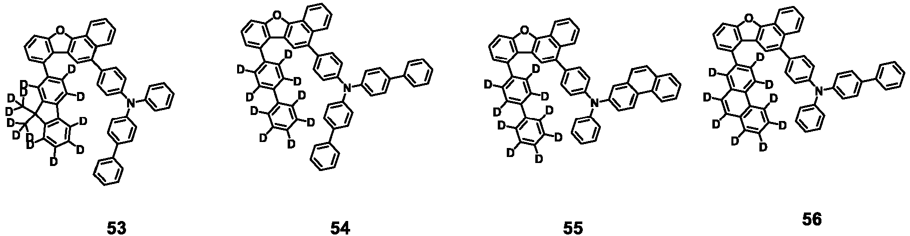

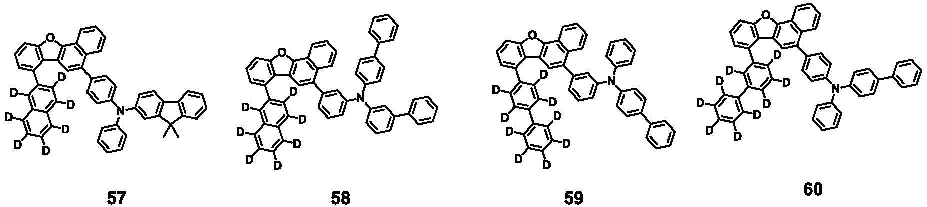

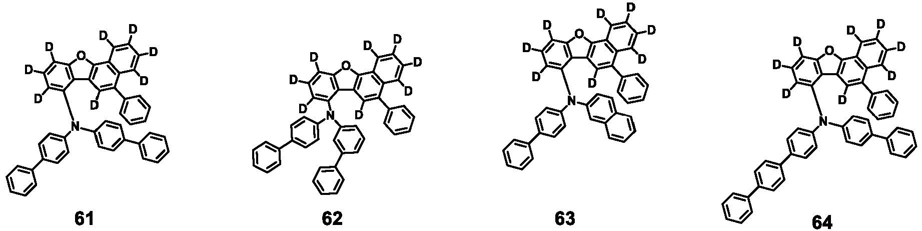

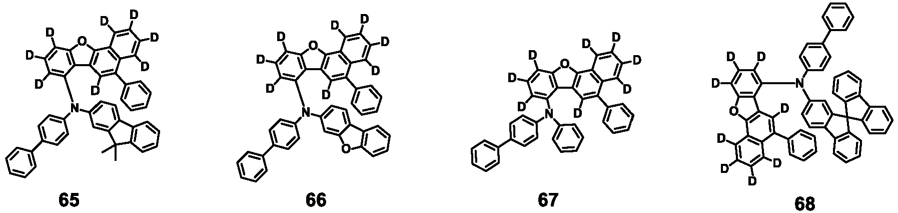

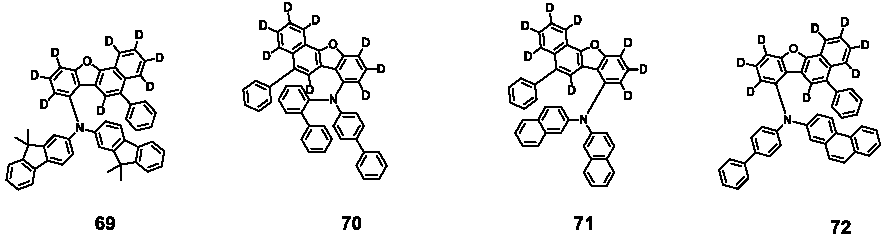

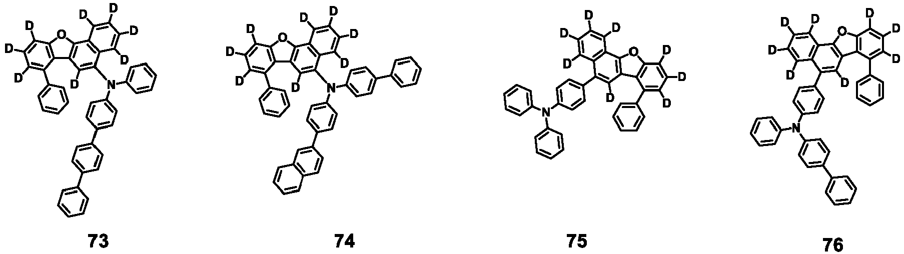

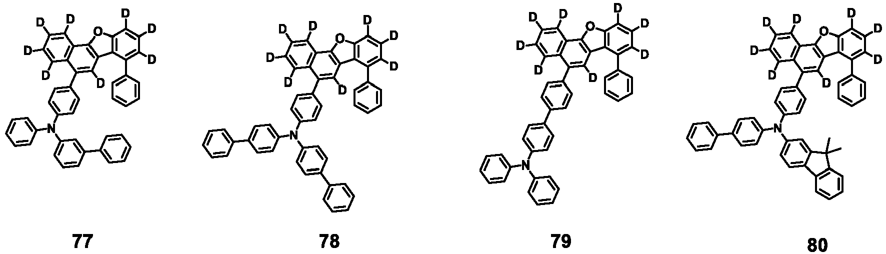

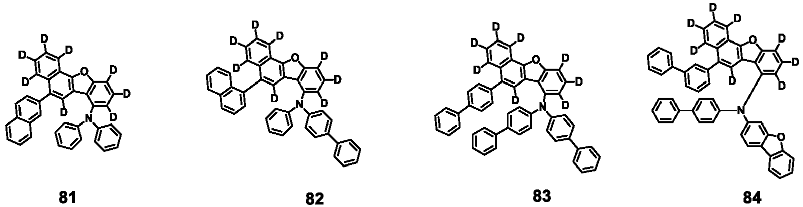

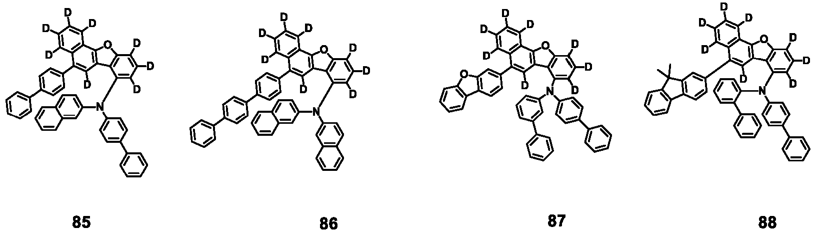

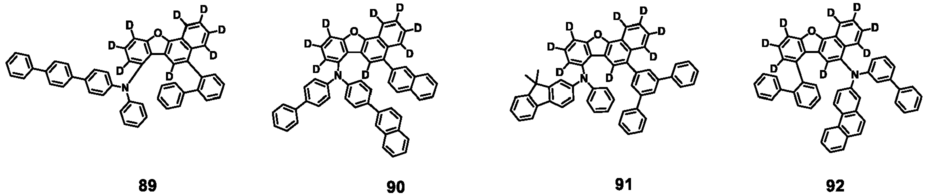

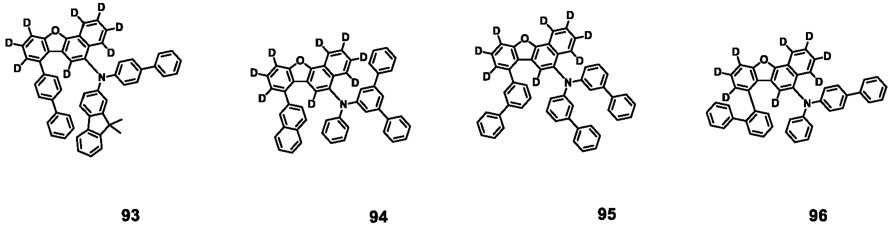

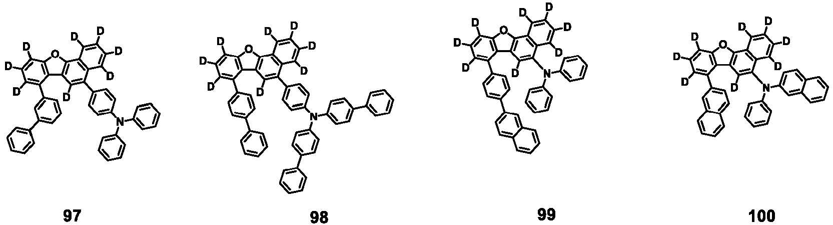

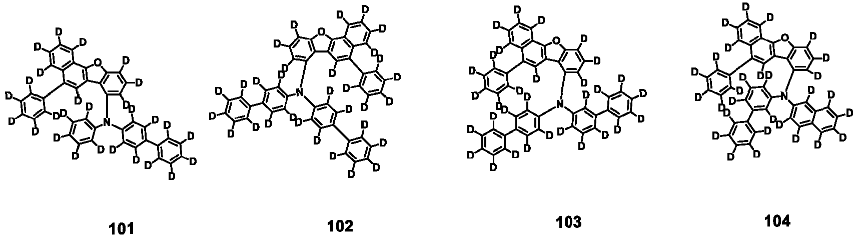

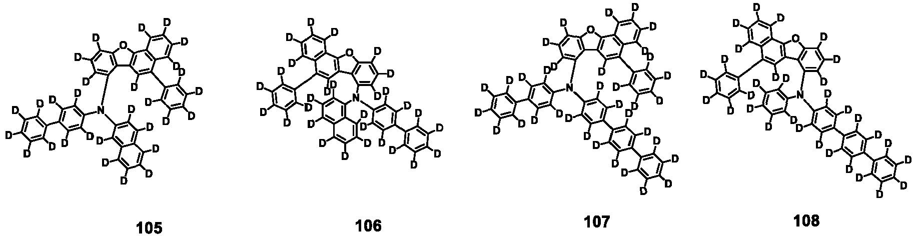

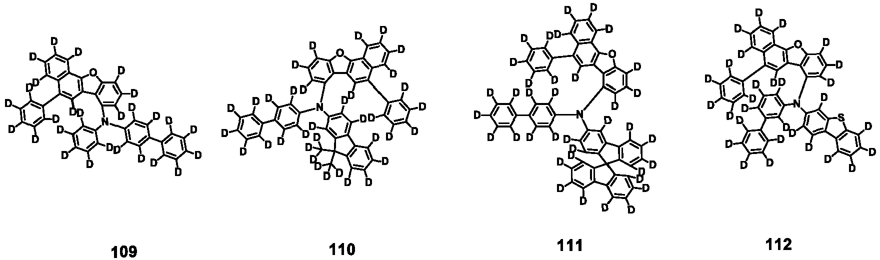

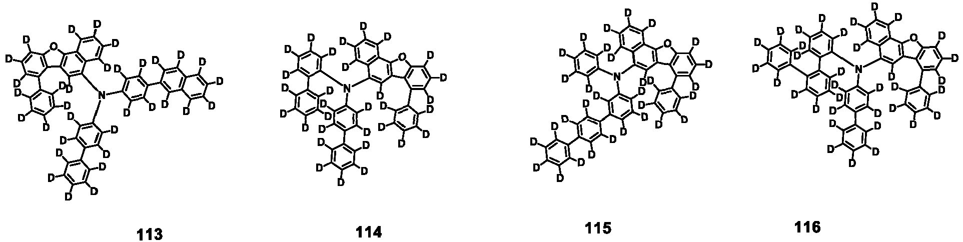

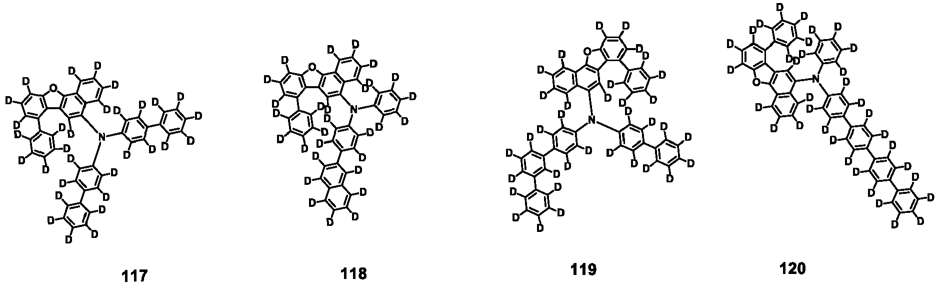

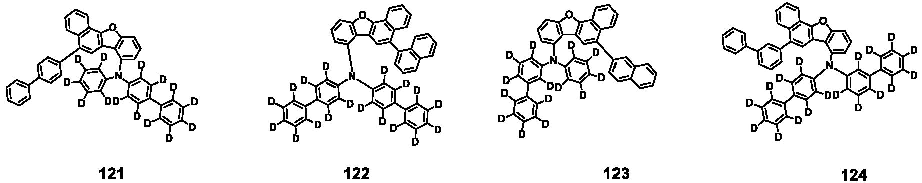

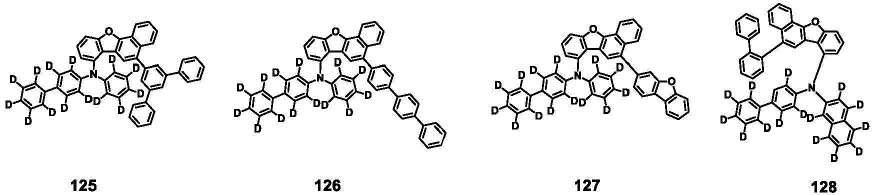

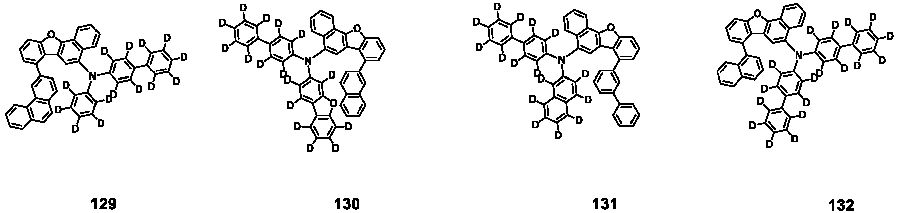

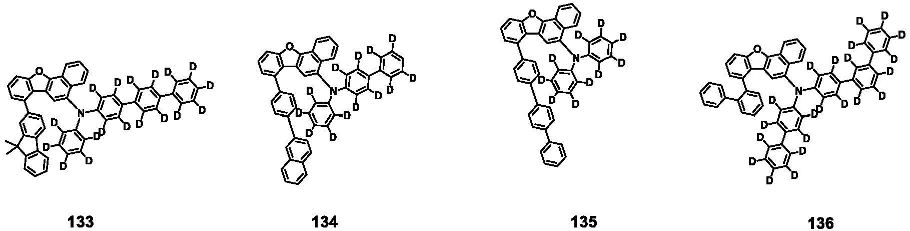

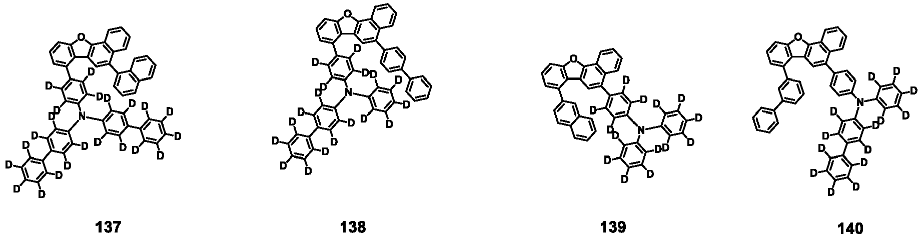

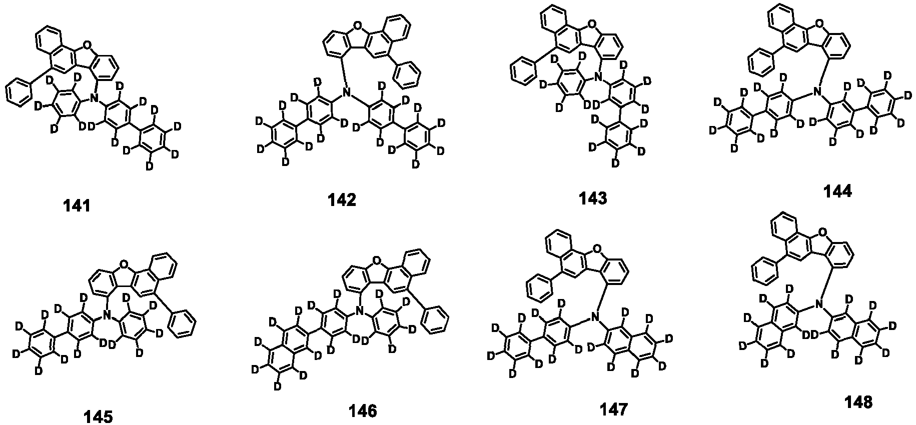

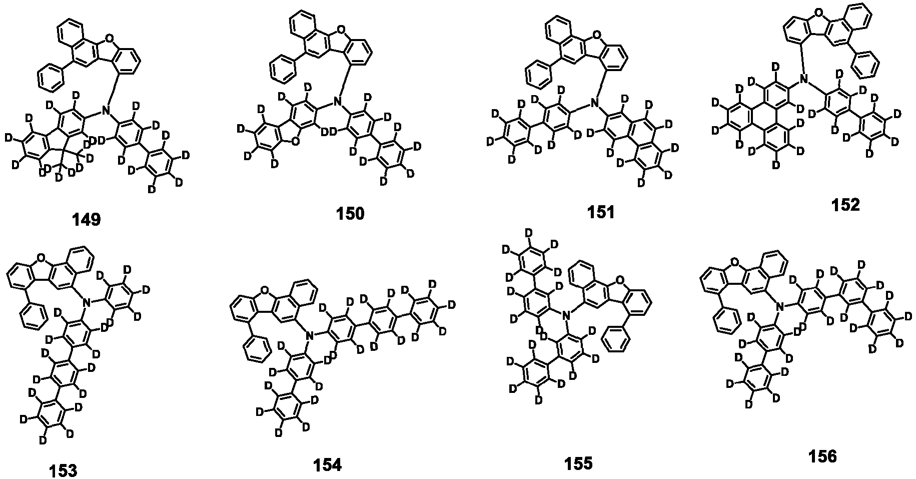

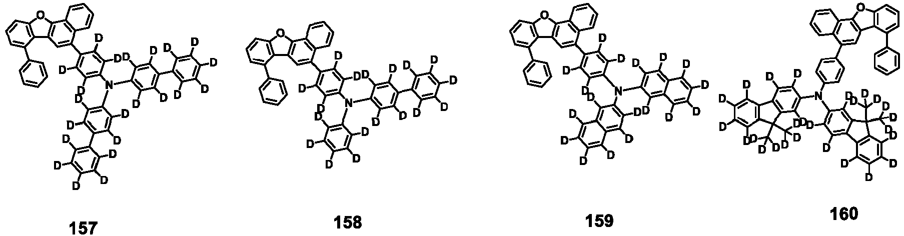

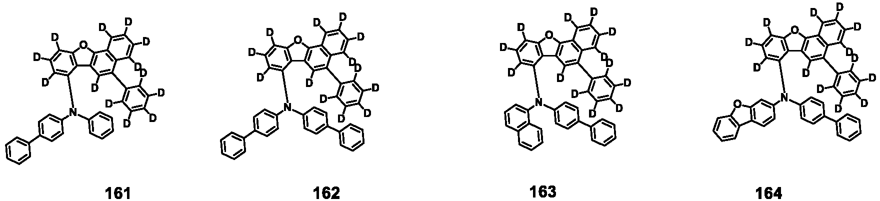

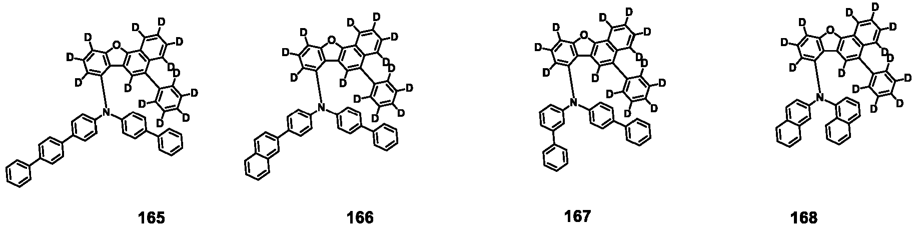

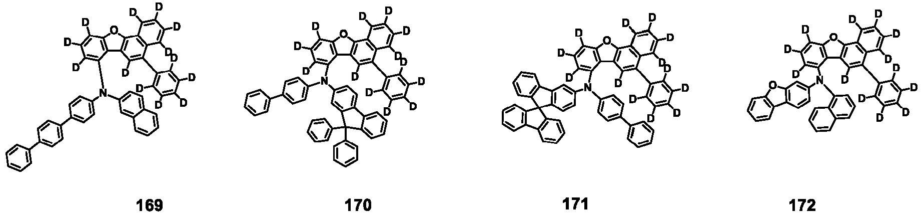

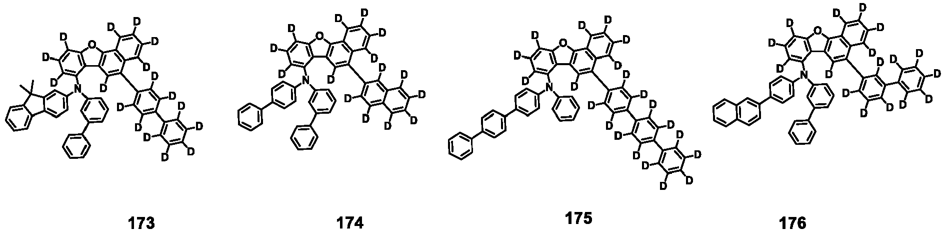

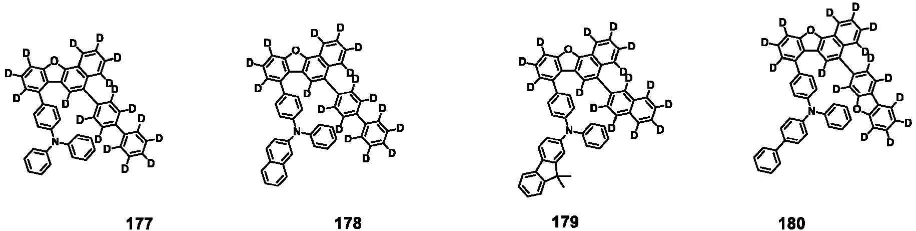

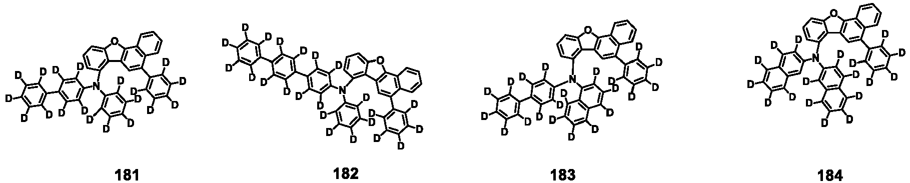

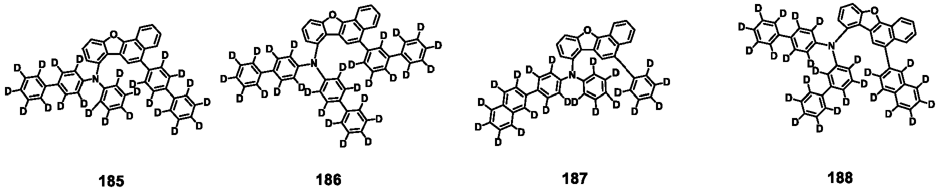

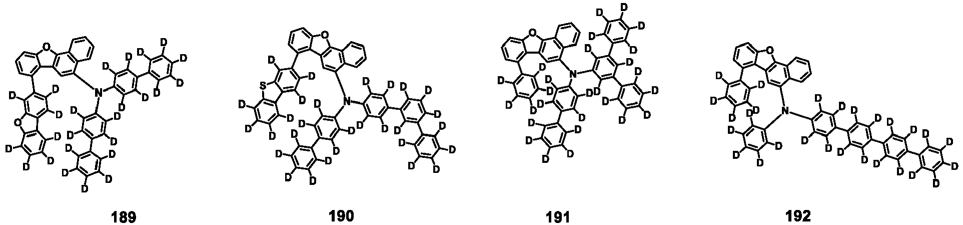

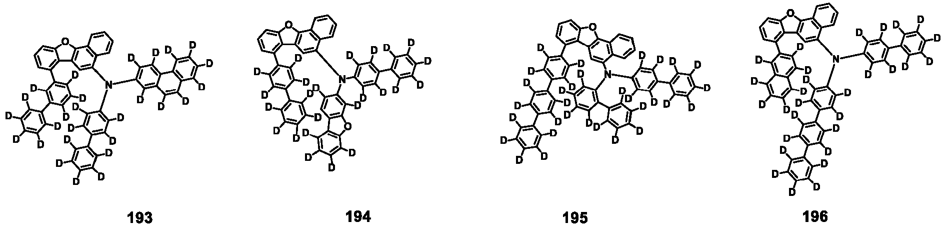

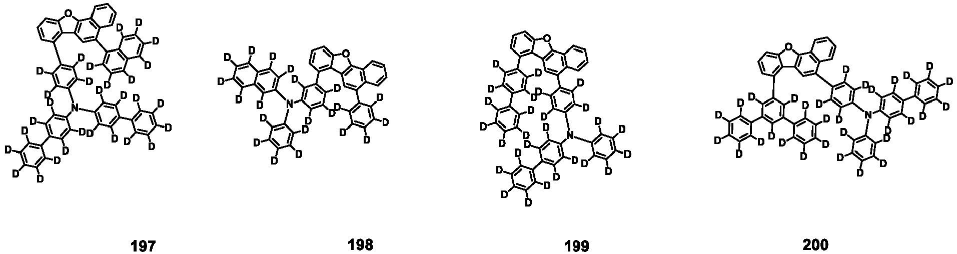

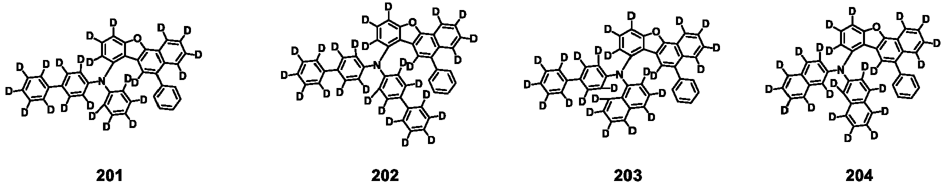

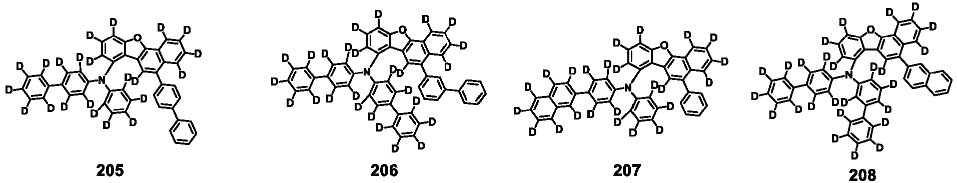

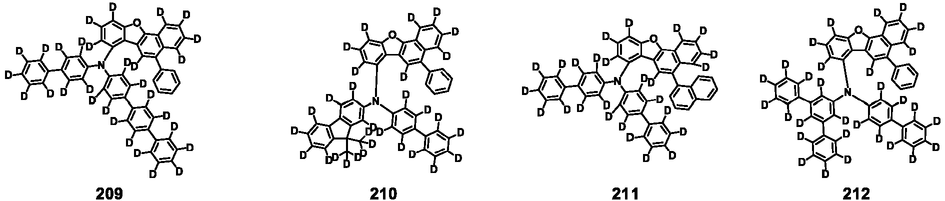

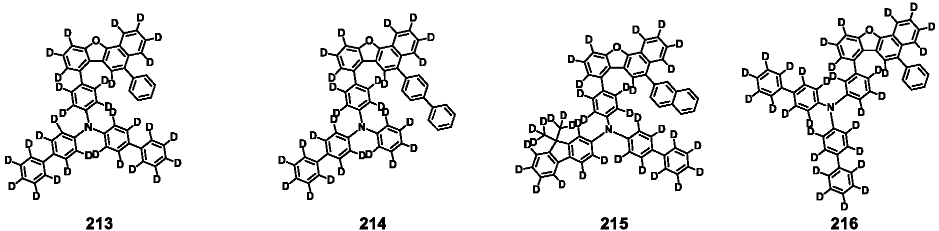

Further, various substituents may be introduced into the structure of Chemical Formula 1 to synthesize a compound having inherent characteristics of a substituent introduced. For example, it is possible to synthesize a material which satisfies conditions required for each organic material layer by introducing a substituent usually used for a hole injection layer material, a hole transport layer material, a light emitting layer material, an electron transport layer material, and a charge generation layer material, which are used for preparing an organic light emitting device, into the core structure.

In addition, it is possible to finely adjust an energy band gap by introducing various substituents into the structure of Chemical Formula 1, and meanwhile, it is possible to improve characteristics at the interface between organic materials and diversify the use of the material.

Furthermore, in an exemplary embodiment of the present application, provided is an organic light emitting device comprising a first electrode; a second electrode provided to face the first electrode; and an organic material layer having one or more layers provided between the first electrode and the second electrode, in which one or more layers of the organic material layer comprise the heterocyclic compound according to Chemical Formula 1.

In another exemplary embodiment, provided is an organic light emitting device comprising: a first electrode; a second electrode provided to face the first electrode; and an organic material layer having one or more layers provided between the first electrode and the second electrode, in which one or more layers of the organic material layer comprise one heterocyclic compound according to Chemical Formula 1.

In still another exemplary embodiment, provided is an organic light emitting device comprising: a first electrode; a second electrode provided to face the first electrode; and an organic material layer having one or more layers provided between the first electrode and the second electrode, in which one or more layers of the organic material layer comprise two heterocyclic compounds according to Chemical Formula 1.

The specific content on the heterocyclic compound represented by Chemical Formula 1 is the same as that described above.

In an exemplary embodiment of the present application, the first electrode may be a positive electrode, and the second electrode may be a negative electrode.

In another exemplary embodiment, the first electrode may be a negative electrode, and the second electrode may be a positive electrode.

In an exemplary embodiment of the present application, the organic light emitting device may be a blue organic light emitting device, and the heterocyclic compound according to Chemical Formula 1 may be used as a material for the blue organic light emitting device. For example, the heterocyclic compound according to Chemical Formula 1 may be included in a host of a hole transport layer, a light emitting auxiliary layer or a light emitting layer of the blue organic light emitting device.

In an exemplary embodiment of the present application, the organic light emitting device may be a green organic light emitting device, and the heterocyclic compound according to Chemical Formula 1 may be used as a material for the green organic light emitting device. For example, the heterocyclic compound according to Chemical Formula 1 may be included in a host of a hole transport layer, a light emitting auxiliary layer or a light emitting layer of the green organic light emitting device.

In an exemplary embodiment of the present application, the organic light emitting device may be a red organic light emitting device, and the heterocyclic compound according to Chemical Formula 1 may be used as a material for the red organic light emitting device. For example, the heterocyclic compound according to Chemical Formula 1 may be included in a host of a hole transport layer, a light emitting auxiliary layer or a light emitting layer of the red organic light emitting device.

The organic light emitting device of the present invention may be manufactured using typical manufacturing methods and materials of an organic light emitting device, except that the above-described heterocyclic compound is used to form an organic material layer having one or more layers.

The heterocyclic compound may be formed as an organic material layer by not only a vacuum deposition method, but also a solution application method when an organic light emitting device is manufactured. Here, the solution application method means spin coating, dip coating, inkjet printing, screen printing, a spray method, roll coating, and the like, but is not limited thereto.

The organic material layer of the organic light emitting device of the present invention may be composed of a single-layered structure, but may be composed of a multilayered structure in which two or more organic material layers are stacked. For example, the organic light emitting device of the present invention may have a structure comprising a hole injection layer, a hole transport layer, a light emitting layer, an electron transport layer, an electron injection layer, and the like as organic material layers. However, the structure of the organic light emitting device is not limited thereto, and may comprise a fewer number of organic material layers.

In the organic light emitting device of the present invention, the organic material layer may comprise a light emitting layer, and the light emitting layer may comprise the heterocyclic compound.

In another organic light emitting device, the organic material layer comprises a light emitting layer, the light emitting layer comprises a host material, and the host material may comprise the heterocyclic compound.

In the organic light emitting device of the present invention, provided is an organic light emitting device in which the organic material layer comprises a light emitting auxiliary layer and the light emitting auxiliary layer comprises the heterocyclic compound.

In an exemplary embodiment of the present application, a light emitting auxiliary layer applied to the organic light emitting device is a layer capable of forming a high light emitting efficiency in the light emitting layer by making a balance between the moving speeds of holes and electrons.

As another example, the organic material layer comprising the heterocyclic compound comprises the heterocyclic compound represented by Chemical Formula 1 as a host, and the heterocyclic compound may be used with an iridium-based dopant.

In the organic light emitting device of the present invention, the organic material layer comprises an electron injection layer or an electron transport layer, and the electron injection layer or electron transport layer may comprise the heterocyclic compound.

In another organic light emitting device, the organic material layer comprises an electron blocking layer or a hole blocking layer, and the electron blocking layer or the hole blocking layer may comprise the heterocyclic compound.

In the organic light emitting device of the present invention, the organic material layer comprises a hole transport layer, and the hole transport layer may comprise the heterocyclic compound.

The organic light emitting device of the present invention may further comprise one or two or more layers selected from the group consisting of a light emitting layer, a hole injection layer, a hole transport layer, an electron injection layer, an electron transport layer, an electron blocking layer, and a hole blocking layer.

FIGS. 1 to 4 exemplify the stacking sequence of the electrodes and the organic material layer of the organic light emitting device according to an exemplary embodiment of the present application. However, the scope of the present application is not intended to be limited by these drawings, and the structure of the organic light emitting device known in the art may also be applied to the present application.

According to FIG. 1, an organic light emitting device in which a positive electrode 200, an organic material layer 300, and a negative electrode 400 are sequentially stacked on a substrate 100 is illustrated. However, the organic light emitting device is not limited only to such a structure, and as in FIG. 2, an organic light emitting device in which a negative electrode, an organic material layer, and a positive electrode are sequentially stacked on a substrate may also be implemented.

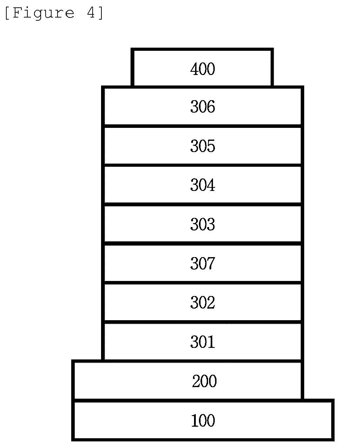

FIGS. 3 and 4 exemplify a case where an organic material layer is a multilayer. The organic light emitting device according to FIG. 3 comprises a hole injection layer 301, a hole transport layer 302, a light emitting layer 303, a hole blocking layer 304, an electron transport layer 305 and an electron injection layer 306, and the organic light emitting device according to FIG. 4 comprises a hole injection layer 301, a hole transport layer 302, a light emitting auxiliary layer 307, a light emitting layer 303, a hole blocking layer 304, an electron transport layer 305 and an electron injection layer 306. However, the scope of the present application is not limited by the stacking structure as described above, and if necessary, the other layers except for the light emitting layer may be omitted, and another necessary functional layer may be further added.

An organic material layer comprising the compound of Chemical Formula 1 may additionally comprise other materials, if necessary.

In the organic light emitting device according to an exemplary embodiment of the present application, materials other than the heterocyclic compound of Chemical Formula 1 will be exemplified below, but these materials are illustrative only and are not for limiting the scope of the present application, and may be replaced with materials publicly known in the art.

As a positive electrode material, materials having a relatively high work function may be used, and a transparent conductive oxide, a metal or a conductive polymer, and the like may be used. Specific examples of the positive electrode material comprise: a metal such as vanadium, chromium, copper, zinc, and gold, or an alloy thereof; a metal oxide such as zinc oxide, indium oxide, indium tin oxide (ITO), and indium zinc oxide (IZO); a combination of a metal and an oxide, such as ZnO:Al or SnO₂:Sb; a conductive polymer such as poly(3-methylthiophene), poly[3,4-(ethylene-1,2-dioxy)thiophene] (PEDOT), polypyrrole, and polyaniline; and the like, but are not limited thereto.

As a material for the negative electrode, materials having a relatively low work function may be used, and a metal, a metal oxide, or a conductive polymer, and the like may be used. Specific examples of the negative electrode material comprise: a metal such as magnesium, calcium, sodium, potassium, titanium, indium, yttrium, lithium, gadolinium, aluminum, silver, tin, and lead, or an alloy thereof; a multi-layer structured material, such as LiF/Al or LiO₂/Al; and the like, but are not limited thereto.

As a hole injection material, a publicly-known hole injection material may also be used, and it is possible to use, for example, a phthalocyanine compound such as copper phthalocyanine disclosed in US Patent No. 4,356,429 or starburst-type amine derivatives described in the document [Advanced Material, 6, p. 677 (1994)], for example, tris(4-carbazoyl-9-ylphenyl)amine (TCTA), 4,4',4"-tris[phenyl(m-tolyl)amino]triphenylamine (m-MTDATA), 1,3,5-tris[4-(3-methylphenylphenylamino)phenyl]benzene (m-MTDAPB), polyaniline/dodecylbenzenesulfonic acid or poly(3,4-ethylenedioxythiophene)/poly(4-styrenesulfonate), which is a soluble conductive polymer, polyaniline/camphor sulfonic acid or polyaniline/poly(4-styrene-sulfonate), and the like.

As a hole transporting material, a pyrazoline derivative, an arylamine-based derivative, a stilbene derivative, a triphenyldiamine derivative, and the like may be used, and a low-molecular weight or polymer material may also be used.

As an electron transporting material, it is possible to use an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene and a derivative thereof, a diphenoquinone derivative, a metal complex of 8-hydroxyquinoline and a derivative thereof, and the like, and a low-molecular weight material and a polymer material may also be used.

As an electron injection material, for example, LiF is representatively used in the art, but the present application is not limited thereto.

As a light emitting material, a red, green, or blue light emitting material may be used, and if necessary, two or more light emitting materials may be mixed and used. In this case, two or more light emitting materials are deposited or used as an individual supply source, or premixed to be deposited and used as one supply source. Further, a fluorescent material may also be used as the light emitting material, but may also be used as a phosphorescent material. As the light emitting material, it is also possible to use alone a material which emits light by combining holes and electrons each injected from a positive electrode and a negative electrode, but materials in which a host material and a dopant material are involved in light emission together may also be used.

When hosts of the light emitting material are mixed and used, the same series of hosts may also be mixed and used, and different series of hosts may also be mixed and used. For example, two or more materials selected from n-type host materials or p-type host materials may be used as a host material for a light emitting layer.

The organic light emitting device according to an exemplary embodiment of the present application may be a top emission type, a bottom emission type, or a dual emission type according to the material to be used.

The heterocyclic compound according to an exemplary embodiment of the present application may act even in organic electronic devices comprising organic solar cells, organic photoconductors, organic transistors, and the like, based on the principle similar to those applied to organic light emitting devices.

### [Mode for Invention]

Hereinafter, the present specification will be described in more detail through Examples, but these Examples are provided only for exemplifying the present application, and are not intended to limit the scope of the present application.

### <Preparation Example 1> Preparation of Compound 1

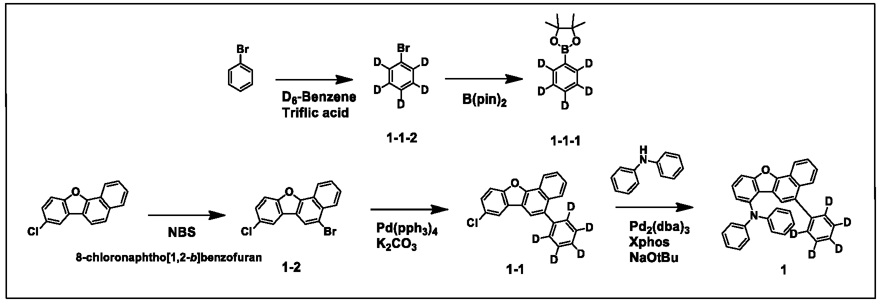

### <Preparation of Compound 1-1-2>

100 g (637 mmol, 1 eq) of bromobenzene, 1000 ml (10 T) of benzene-D₆, and 393 ml (4459 mmol, 7 eq) of triflic acid were put into a flask, and the resulting mixture was stirred at 60°C. After 4 hours, the reaction was terminated by adding 1000 ml of distilled water dropwise thereto. After the organic layer was extracted, moisture was removed using MgSO₄. The concentrated organic layer was purified by being allowed to pass through silica gel. 98 g (yield 95%) of Compound 1-1-2 was obtained.

### <Preparation of Compound 1-1-1>

98 g (605 mmol, 1 eq) of Compound 1-1-2, 183 g (726 mmol, 1.2 eq) of B₂(pin)₂, 22 g (30 mmol, 0.05 eq) of Pd(dppf)Cl₂, 177 g (1815 mmol, 3 eq) of KoAc, and 1000 ml (10 T) of 1,4-dioxane were put into a flask, and the resulting mixture was stirred at 120°C. After 4 hours, the reaction solution was filtered with celite, and then concentrated. The concentrated solution was dissolved in DCM, and the resulting solution was allowed to pass through silica. The solution was concentrated, MeOH was added dropwise thereto, and then the resulting mixture was stirred, and filtered to obtain 108 g (yield: 85%) of Compound 1-1-1.

### <Preparation of Compound 1-2>

100 g (397 mmol, 1 eq) of 8-chloronaphtho[1,2-b]benzofuran, 77 g (1.1 eq), and 1000 ml (10 T) of DMF were added dropwise to a flask, and the resulting mixture was stirred at 120°C. A large amount of solid was produced in the reaction solution after 2 hours. MeOH was added dropwise thereto, and then the resulting mixture was stirred, and filtered to obtain 120 g (yield: 91%) of Compound 1-2.

### <Preparation of Compound 1-1>

120 g (362 mmol, 1 eq) of Compound 1-2, 75 g (1 eq) of Compound 1-1-1, 21 g (0.05 eq) of Pd(PPh₃)₄, 150 g (3 eq) of K₂CO₃, 1200 ml of 1,4-dioxane, and 300 ml of water were added dropwise to a flask, and the resulting mixture was refluxed. After 4 hours, the reaction solution was extracted, the organic layer was adsorbed onto silica and purified with adsorption column chromatography. The resulting solution was concentrated, MeOH was added dropwise thereto, and then the resulting solid was filtered to obtain 108 g (yield 90%) of Compound 1-1.

### <Preparation of Compound 1>

10 g (30 mmol, 1 eq) of Compound 1-1, 5 g (1 eq) of diphenylamine, 1.3 g (0.05 eq) of Pd₂(dba)₃, 1.4 g (0.1 eq) of Xphos, 8.6 g (3 eq) of NaOtBu, and 100 ml of xylene were added dropwise to a flask, and the resulting mixture was refluxed. After 4 hours, the reaction solution was filtered with celite, adsorbed onto silica and purified with adsorption column chromatography. The solution was concentrated, EA and MeOH were added dropwise thereto, and then the resulting solid was filtered to obtain 10 g (yield 73%) of Compound 1.

A target compound in the following Table 1 was obtained by performing synthesis in the same manner as in Preparation Example 1, except that Compound A in the following Table 1 was used instead of diphenylamine in Preparation Example 1.

**[Table 1]**

| Compound (yield) | Compound A | Target compound |
|---|---|---|
| 2 (88%) | 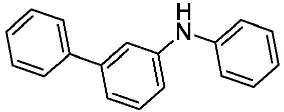 | 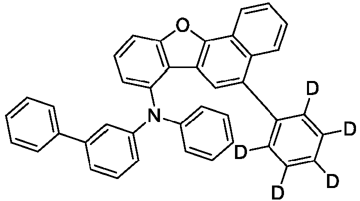 |
| 3 (78%) | 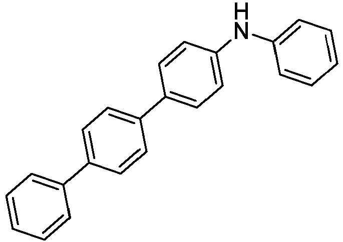 | 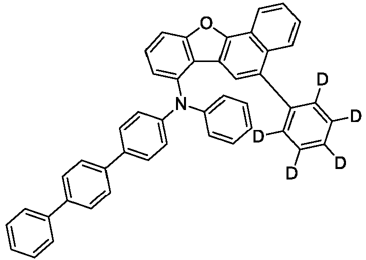 |
| 4 (77%) | 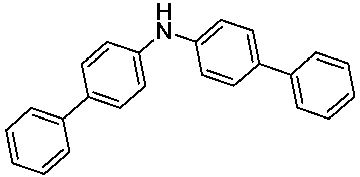 | 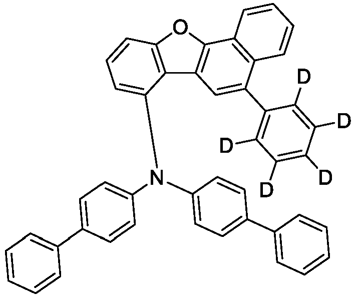 |
| 5 (89%) | 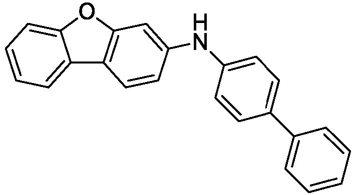 | 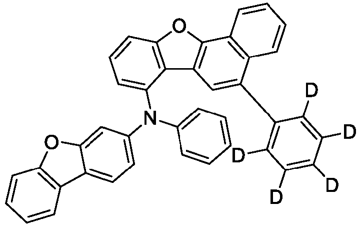 |
| 6 (87%) | 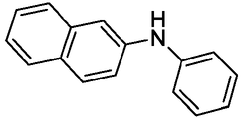 | 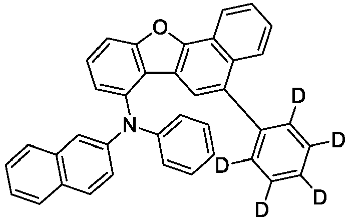 |
| 7 (79%) | 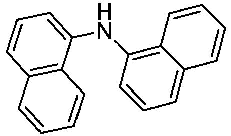 | 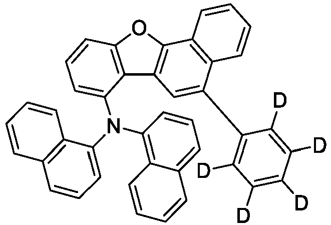 |
| 8 (90%) | 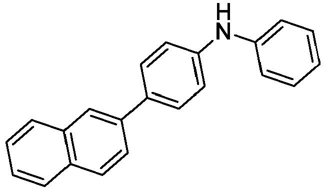 | 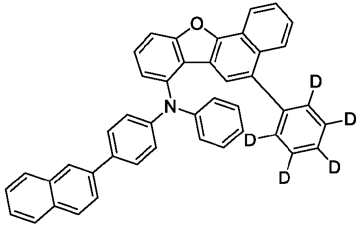 |
| 9 (78%) | 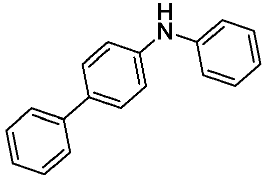 | 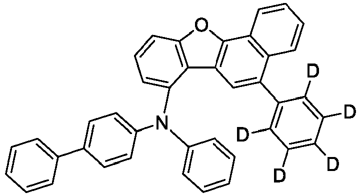 |
| 10 (77%) | 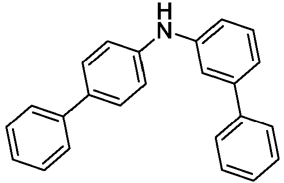 | 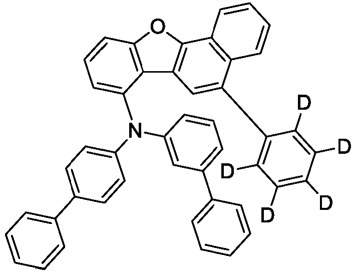 |
| 11 (86%) | 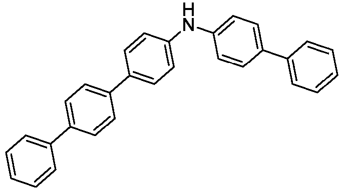 | 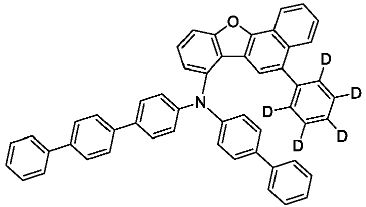 |
| 12 (85%) | 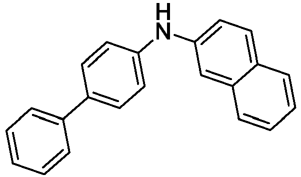 | 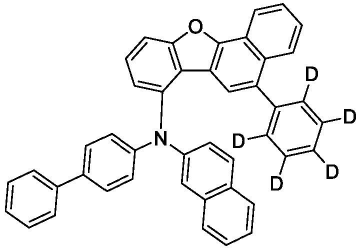 |

A target compound in the following Table 2 was obtained by performing synthesis in the same manner as in Preparation Example 1, except that Compound A in the following Table 2 was used instead of bromobenzene in Preparation Example 1 and Compound B in the following Table 2 was used instead of diphenylamine in Preparation Example 1.

**[Table 2]**

| Compound (yield) | Compound A | Compound B | Target compound |
|---|---|---|---|
| 21 (81%) | 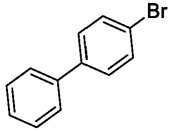 | 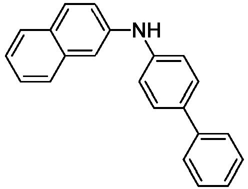 | 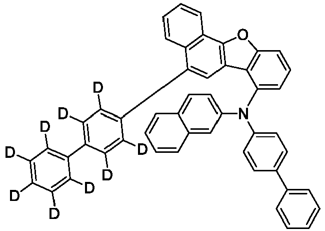 |
| 22 (78%) | 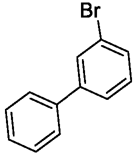 | 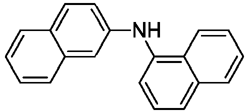 | 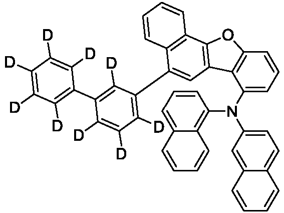 |
| 23 (81%) | 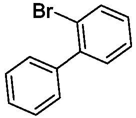 | 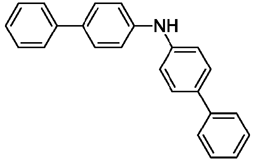 | 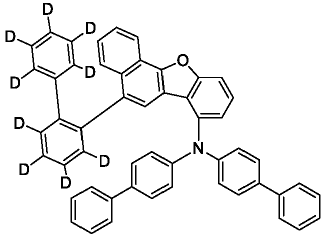 |
| 24 (82%) | 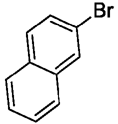 | 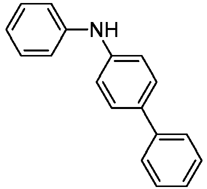 | 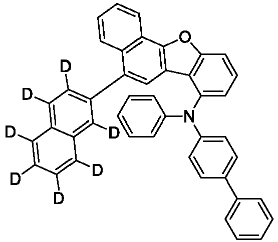 |
| 25 (85%) | 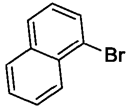 | 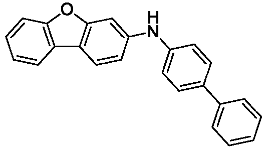 | 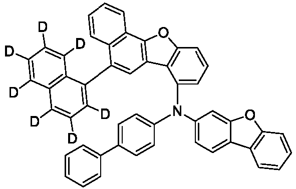 |
| 26 (78%) | 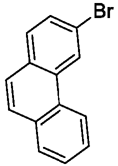 | 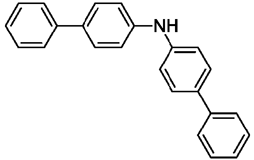 | 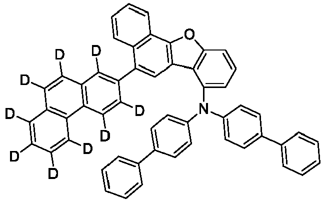 |
| 27 (72%) | 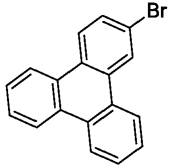 | 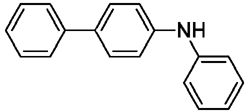 | 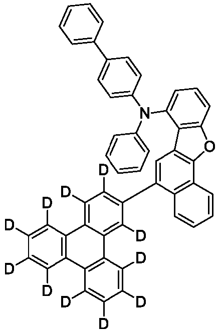 |
| 28 (91%) | 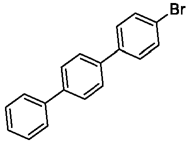 | 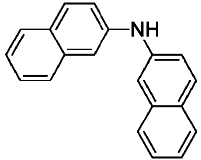 | 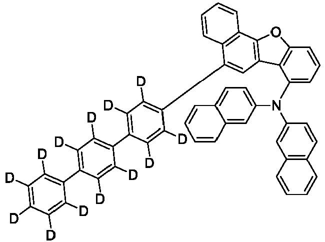 |
| 29 (85%) | 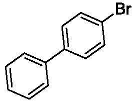 | 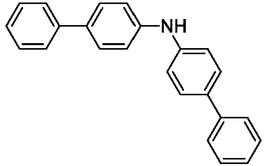 | 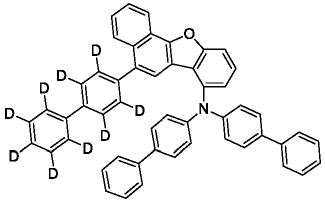 |
| 30 (93%) | 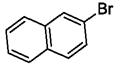 | 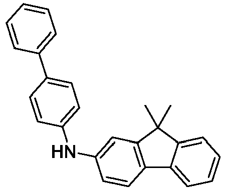 | 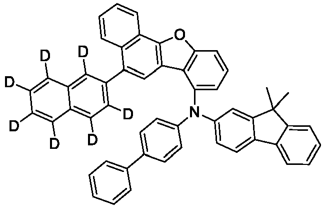 |
| 31 (77%) | 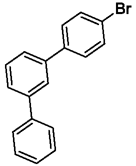 | 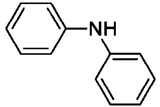 | 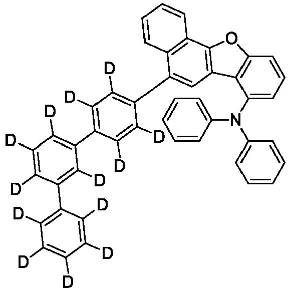 |
| 32 (75%) | 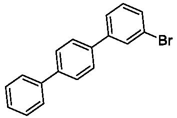 | 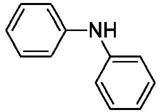 | 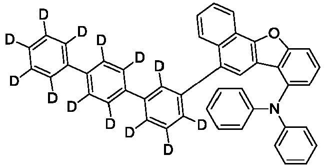 |

### <Preparation Example 2> Preparation of Compound 13

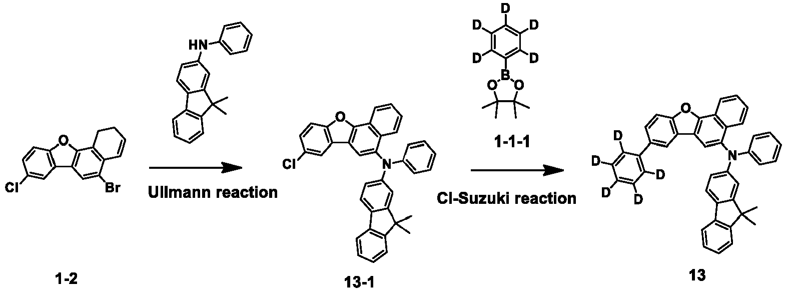

### <Preparation of Compound 13-1>

10 g (30 mmol, 1 eq) of Compound 1-2, 1 eq of 9,9-dimethyl-N-phenyl-9H-fluoren-2-amine, 0.05 eq of Pd(OAc)₂, 1 eq of xantphos, 3 eq of NaOtBu, and 10 T of toluene were added dropwise to a flask, and the resulting mixture was refluxed. After 4 hours, the reaction solution was filtered with celite, adsorbed onto silica, and then purified with an adsorption column chromatography purification method. The resulting solution was concentrated, MeOH was added dropwise thereto, and the resulting mixture was filtered to obtain 14 g (yield 87%) of Compound 13-1.

### <Preparation of Compound 13>

14 g (26 mmol, 1 eq) of Compound 13-1, 1.05 eq of Compound 1-1-1 prepared in Preparation Example 1, 0.05 eq of Pd₂(dba)₃, 1 eq of Xphos, 3 eq of K₂CO₃, 150 ml of 1,4-dioxane, and 30 ml of water were added dropwise to a flask, and then the resulting mixture was refluxed. After 3 hours, the reaction solution was filtered with celite, concentrated and adsorbed onto silica, and then purified with an adsorption column chromatography purification method. The resulting solution was concentrated, EA and MeOH were added dropwise thereto, and a solid was filtered while the resulting mixture was stirred. 12 g (yield 80%) of Compound 13 was obtained.

A target compound in the following Table 3 was obtained by performing synthesis in the same manner as in Preparation Example 2, except that Compound A in the following Table 3 was used instead of 9,9-dimethyl-N-phenyl-9H-fluoren-2-amine in Preparation Example 2.

**[Table 3]**

| Compound (yield) | Compound A | Target compound |
|---|---|---|
| 14 (75%) | 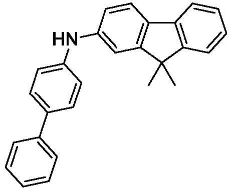 | 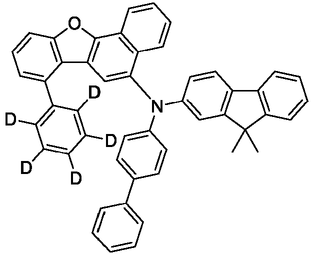 |
| 15 (72%) | 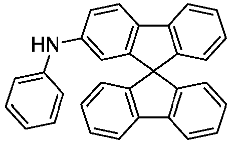 | 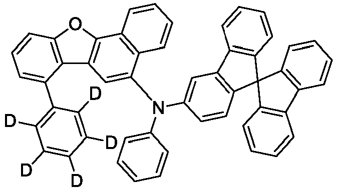 |
| 16 (73%) | 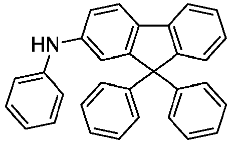 | 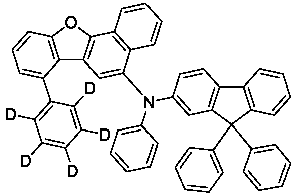 |
| 17 (81%) | 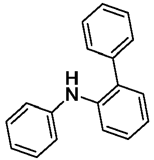 | 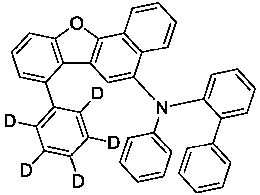 |
| 18 (69%) | 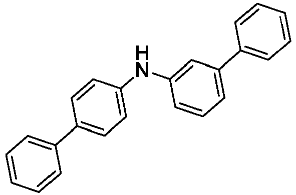 | 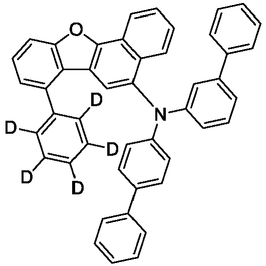 |
| 19 (88%) | 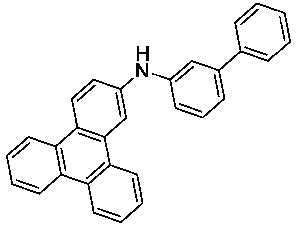 | 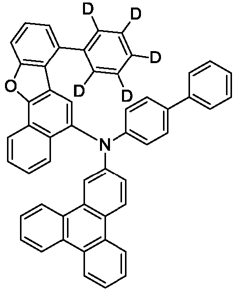 |
| 20 (90%) | 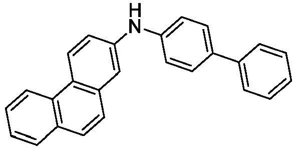 | 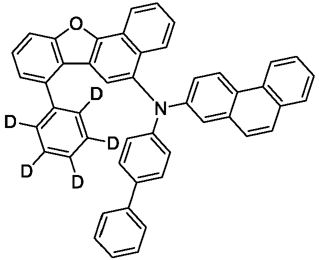 |

An intermediate used instead of Compound 1-1-1 was obtained using Compound A in the following Table 4 instead of bromobenzene in Preparation Example 1, the intermediate was used instead of Compound 1-1-1 in Preparation Example 2, and a target compound in the following Table 4 was obtained using Compound B in the following Table 4 instead of 9,9-dimethyl-N-phenyl-9H-fluoren-2-amine.

**[Table 4]**

| Compound (yield) | Compound A | Compound B | Target compound |
|---|---|---|---|
| 33 (72%) | 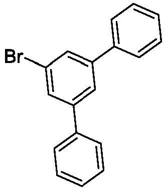 | 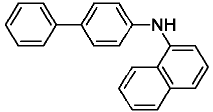 | 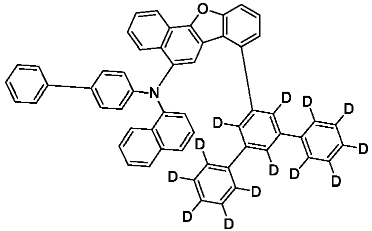 |
| 34 (77%) | 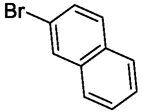 | 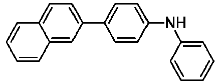 | 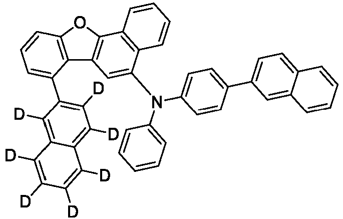 |
| 35 (81%) | 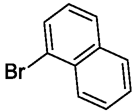 | 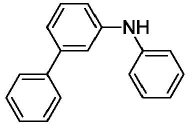 | 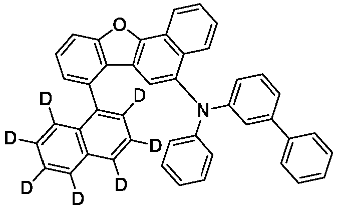 |
| 36 (89%) | 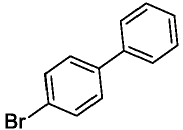 | 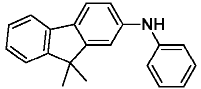 | 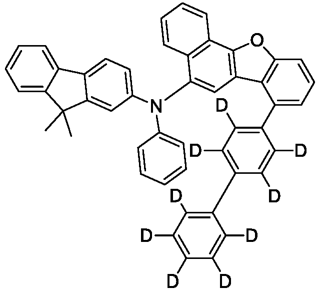 |
| 37 (85%) | 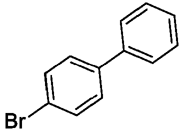 | 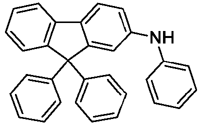 | 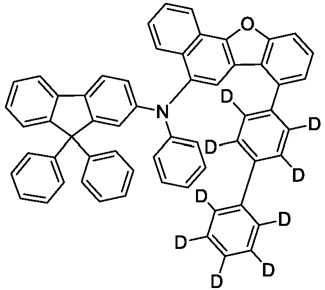 |
| 38 (68%) | 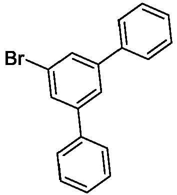 | 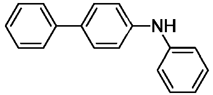 | 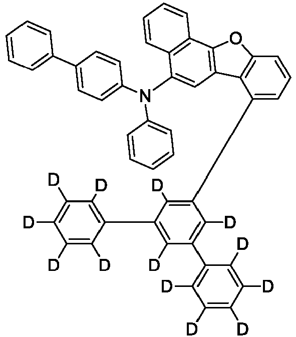 |
| 39 (75%) | 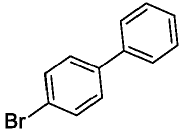 | 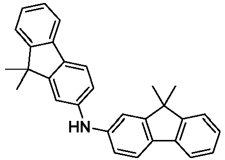 | 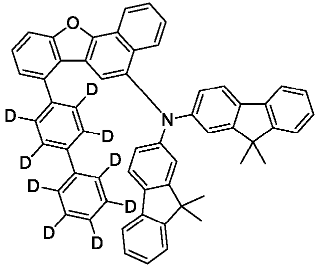 |
| 40 (76%) | 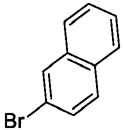 | 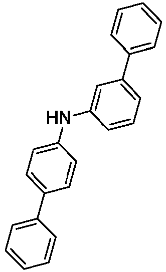 | 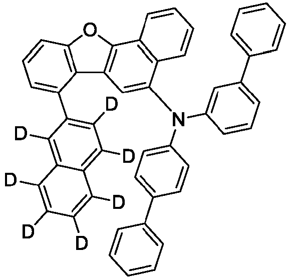 |

The preparation methods of Preparation Examples 1 and 2 are well-known name reactions, and in order to simplify the description of Preparation Examples 3 to 16, the preparation methods of compounds are defined as shown in the following Table 5.

**[Table 5]**

| **Preparation method** | **Name reaction** |
|---|---|
| **Preparation method of Compound 1-1 in Preparation Example 1** | Br-Suzuki reaction |
| **Preparation method of Compound 1 in Preparation Example 1** | Buchwald reaction |
| **Preparation method of Compound 13-1 in Preparation Example 2** | Ullmann reaction |
| **Preparation method of Compound 13 in Preparation Example 2** | Cl-Suzuki reaction |

### <Preparation Example 3> Preparation of Compound 41

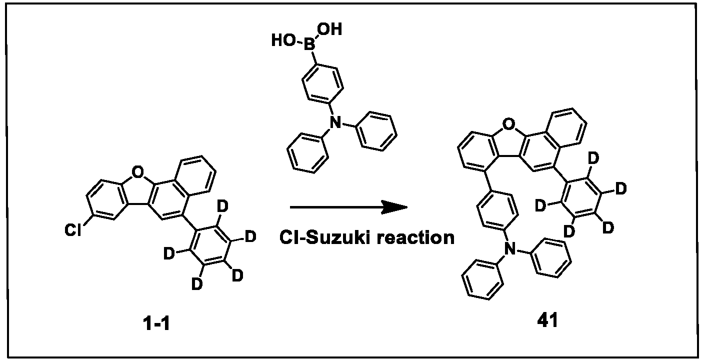

### <Preparation of Compound 41>

Compound 41 was obtained by a Cl-Suzuki reaction using Compound 1-1 prepared in Preparation Example 1 and 4-(diphenylamino)phenylboronic acid.

Compound A in the following Table 6 was prepared in the same manner as in the preparation method of Compound 1-1-1 in Preparation Example 1, an intermediate was prepared using Compound A in the following Table 6 instead of Compound 1-1-1 in the preparation method of Compound 1-1 in Preparation Example 1, and a target compound in the following Table 6 was obtained using the intermediate instead of Compound 1-1 and using Compound B in the following Table 6 instead of 4-(diphenylamino)phenylboronic acid in Preparation Example 3.

**[Table 6]**

| Compound (yield) | Compound A | Compound B | Target compound |
|---|---|---|---|
| 42 (75%) | 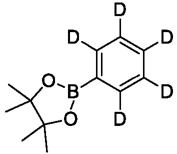 | 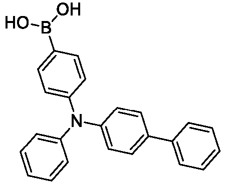 | 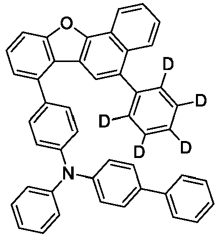 |
| 43 (77%) | 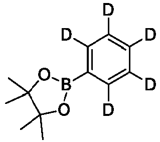 | 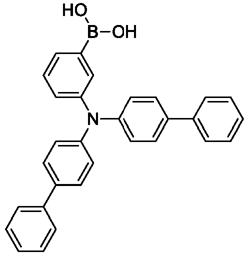 | 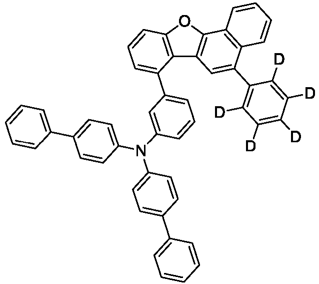 |
| 44 (68%) | 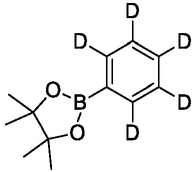 | 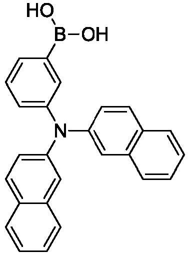 | 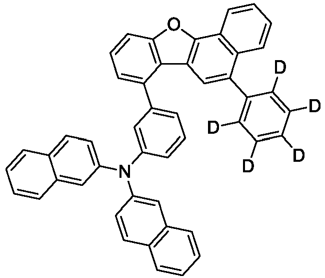 |
| 45 (74%) | 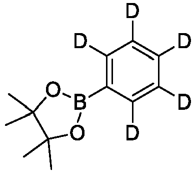 | 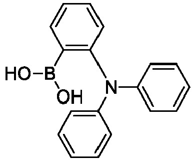 | 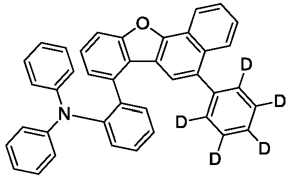 |
| 46 (81%) | 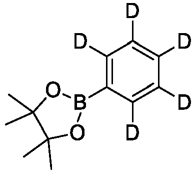 | 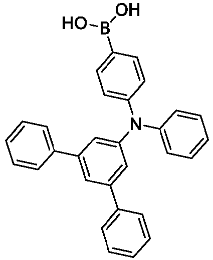 | 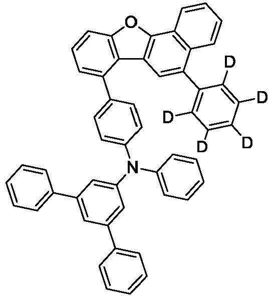 |
| 47 (85%) | 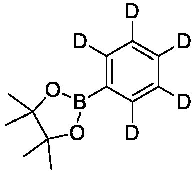 | 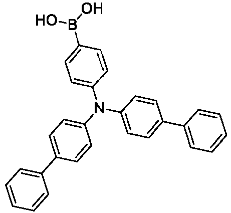 | 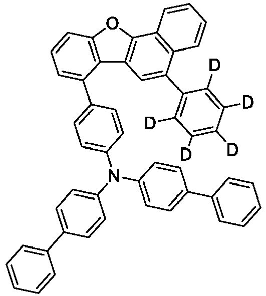 |
| 48 (90%) | 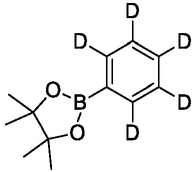 | 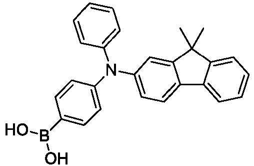 | 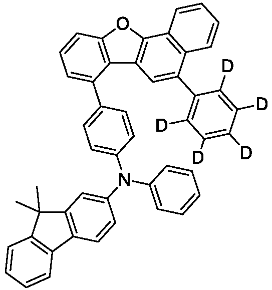 |
| 49 (86%) | 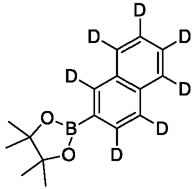 | 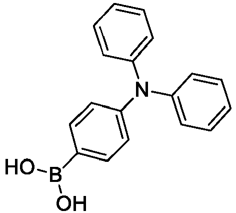 | 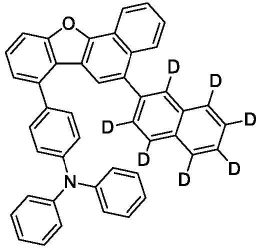 |
| 50 (88%) | 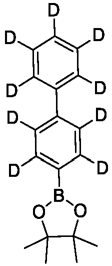 | 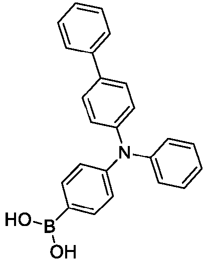 | 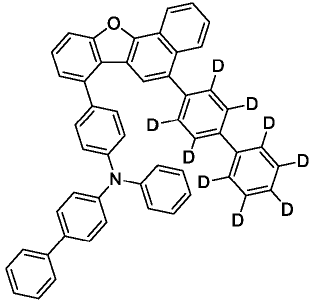 |
| 51 (79%) | 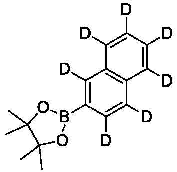 | 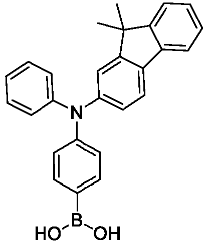 | 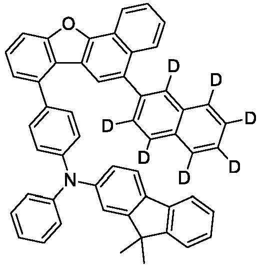 |
| 52 (70%) | 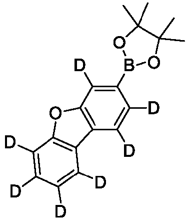 | 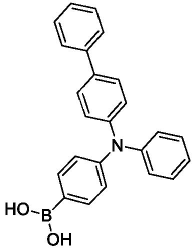 | 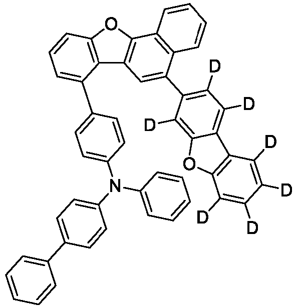 |

### <Preparation Example 4> Preparation of Compound 53

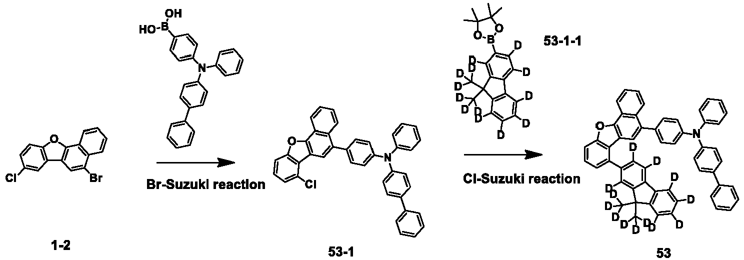

### <Preparation of Compound 53-1-1>

Compound 53-1-1 instead of Compound 1-1-1 was obtained using 2-bromo-9,9-dimethyl-9H-fluorene instead of bromobenzene in Preparation Example 1.

### <Preparation of Compound 53-1>

Compound 53-1 was obtained by a Br-Suzuki reaction using Compound 1-2 prepared in Preparation Example 1 and (4-([1,1'-biphenyl]-4-yl(phenyl)amino)phenyl)boronic acid.

### <Preparation of Compound 53>

Compound 53 was obtained by a Cl-suzuki reaction using Compound 53-1 and Compound 53-1-1.

Compound A in the following Table 7 was obtained by the preparation method of Compound 1-1-1 in Preparation Example 1, and Compound A in the following Table 7 was used instead of Compound 53-1-1 in Preparation Example 4 and Compound B in the following Table 7 was used instead of (4-([1,1'-biphenyl]-4-yl(phenyl)amino)phenyl)boronic acid in Preparation Example 4 to obtain a target compound in the following Table 7.

**[Table 7]**

| Compound (yield) | Compound A | Compound B | Target compound |
|---|---|---|---|
| 54 (72%) | 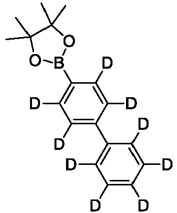 | 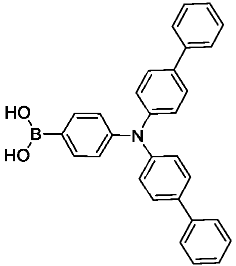 | 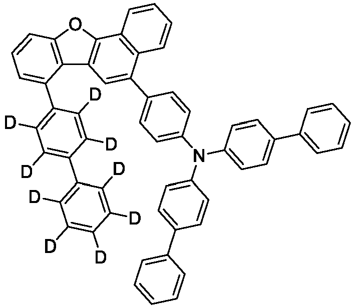 |
| 55 (80%) | 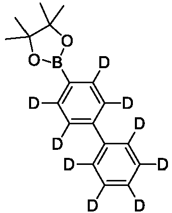 | 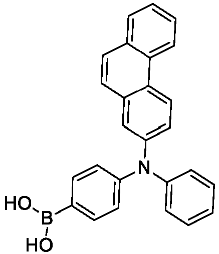 | 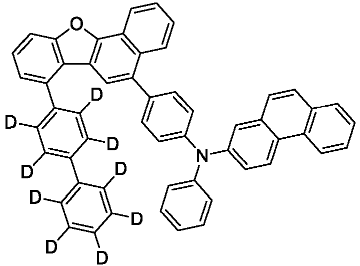 |
| 56 (86%) | 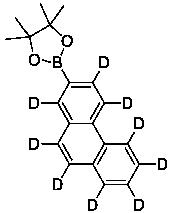 | 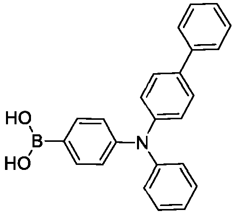 | 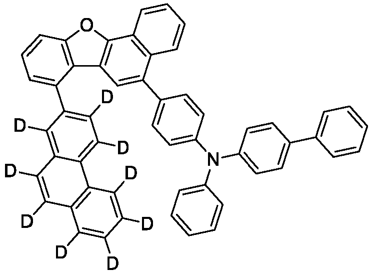 |
| 57 (84%) | 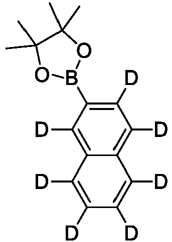 | 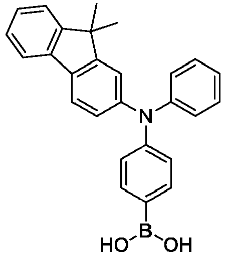 | 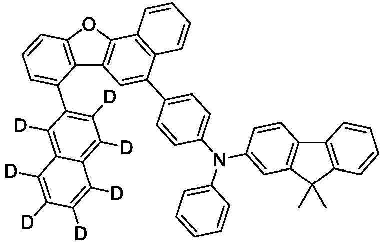 |
| 58 (78%) | 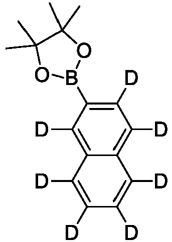 | 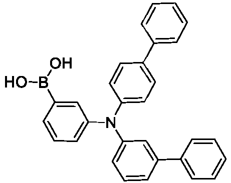 | 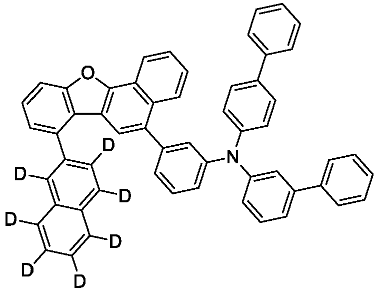 |
| 59 (86%) | 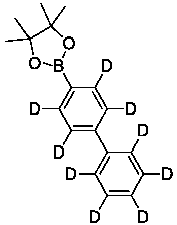 | 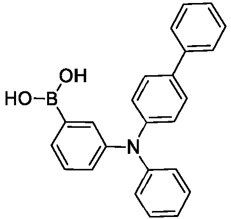 | 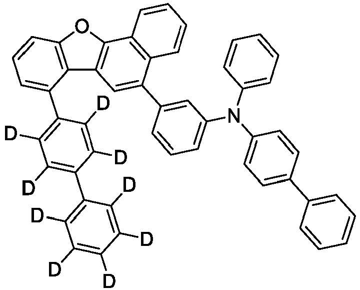 |
| 60 (82%) | 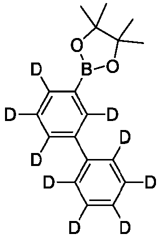 | 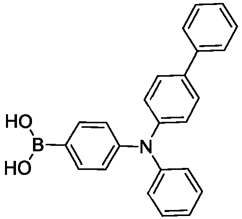 | 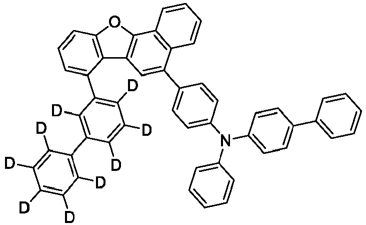 |

### <Preparation Example 5> Preparation of Compound 61

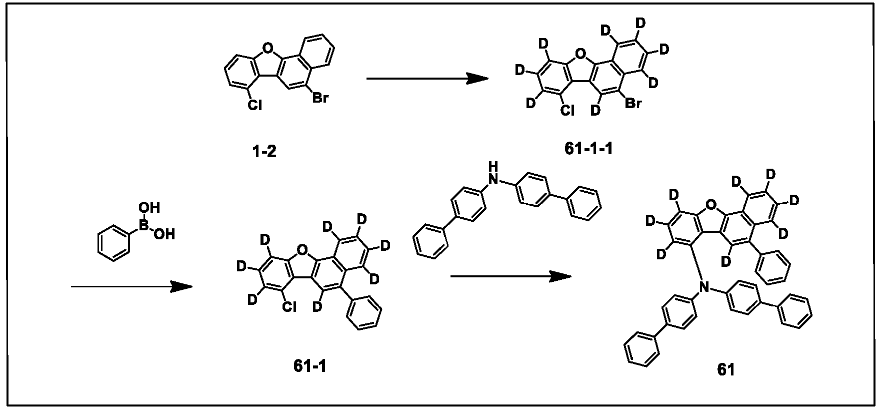

### <Preparation of Compound 61-1-1>

Compound 61-1-1 instead of Compound 1-1-2 was obtained using Compound 1-2 prepared in Preparation Example 1 instead of bromobenzene in Preparation Example 1.

### <Preparation of Compound 61-1>

Compound 61-1 was obtained by a Br-Suzuki reaction using Compound 61-1-1 and phenylboronic acid.

### <Preparation of Compound 61>

Compound 61 was obtained by a Buchwald reaction using Compound 61-1 and di([1,1'-biphenyl]-4-yl)amine.

Compound A in the following Table 8 was used instead of phenylboronic acid in Preparation Example 5 and Compound B was used instead of di([1,1'-biphenyl]-4-yl)amine in Preparation Example 5 to obtain a target compound in the following Table 8. Compound 61-1-1 was commonly used to obtain the target compound in the following Table 8.

**[Table 8]**

| Compound (yield) | Compound A | Compound B | Target compound |
|---|---|---|---|
| 62 (90%) | 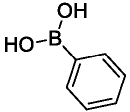 | 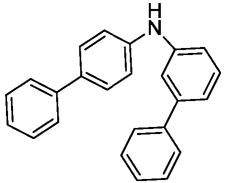 | 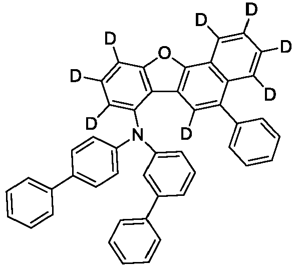 |
| 63 (88%) | 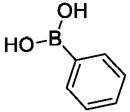 | 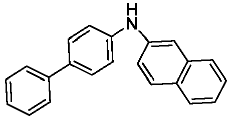 | 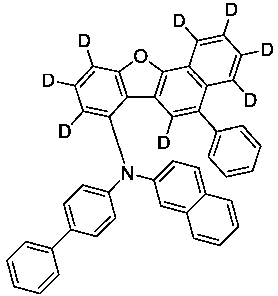 |
| 64 (86%) | 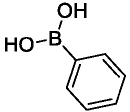 | 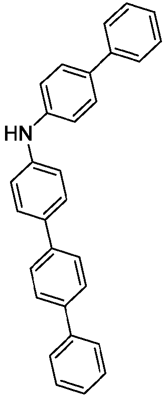 | 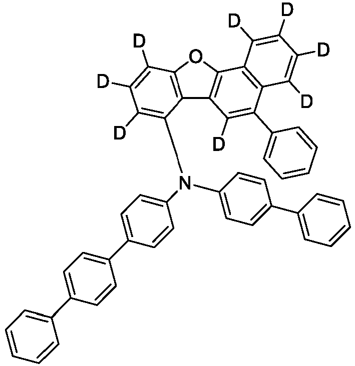 |
| 65 (91%) | 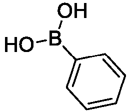 | 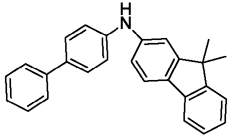 | 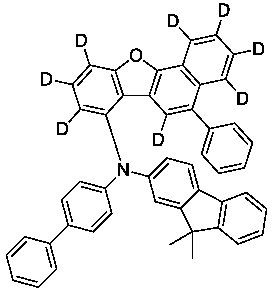 |
| 66 (79%) | 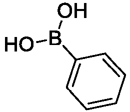 | 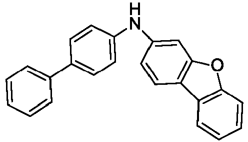 | 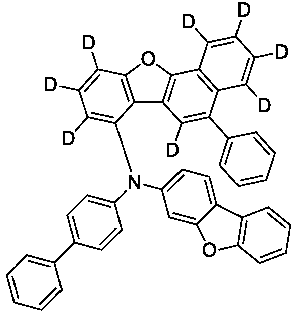 |
| 67 (80%) | 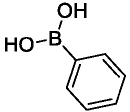 | 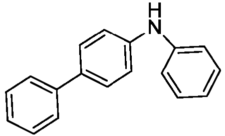 | 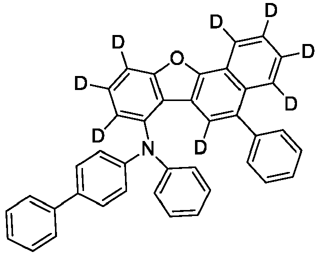 |
| 68 (85%) | 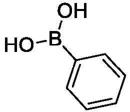 | 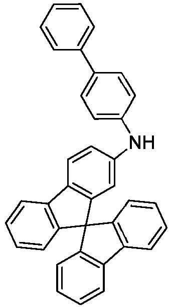 | 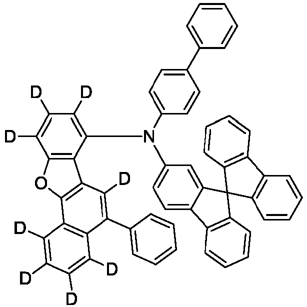 |
| 69 (79%) | 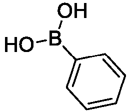 | 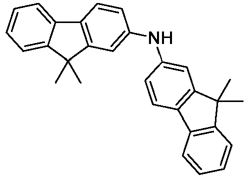 | 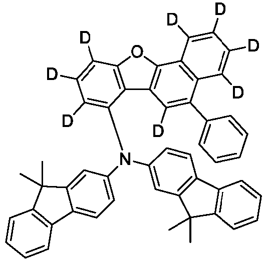 |
| 70 (76%) | 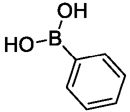 | 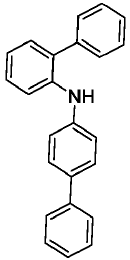 | 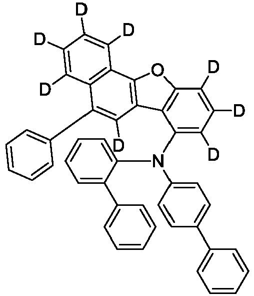 |
| 71 (70%) | 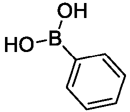 | 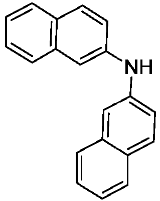 | 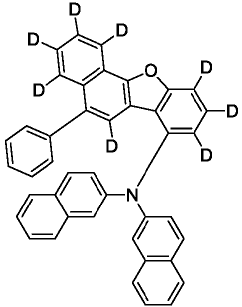 |
| 72 (69%) | 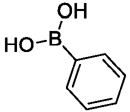 | 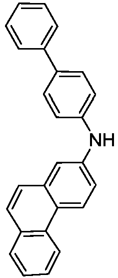 | 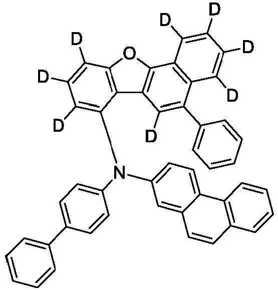 |
| 81 (80%) | 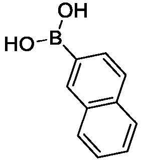 | 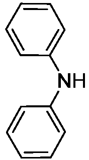 | 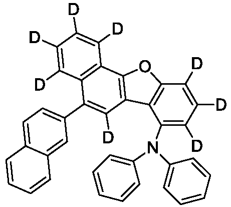 |
| 82 (77%) | 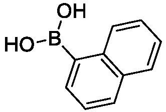 | 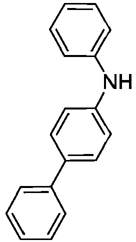 | 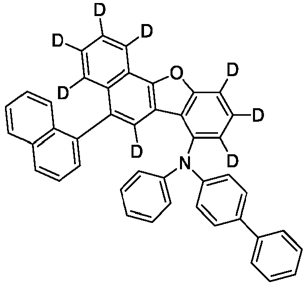 |
| 83 (76%) | 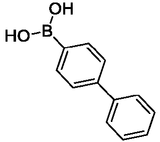 | 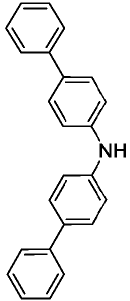 | 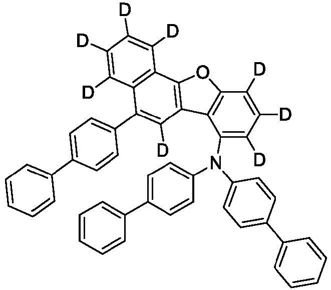 |
| 84 (86%) | 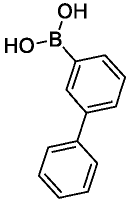 | 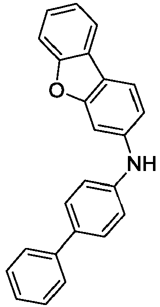 | 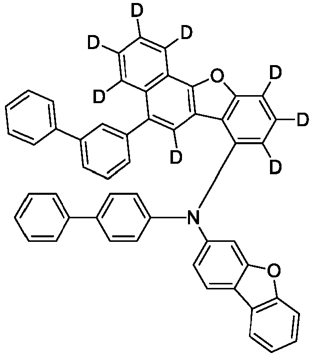 |
| 85 (77%) | 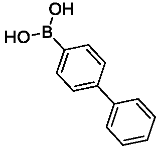 | 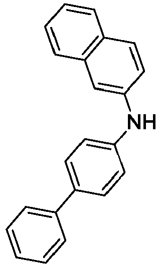 | 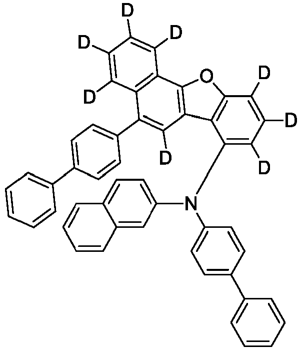 |
| 86 (89%) | 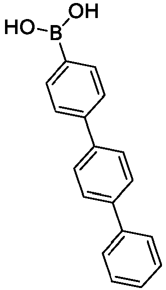 | 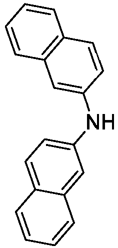 | 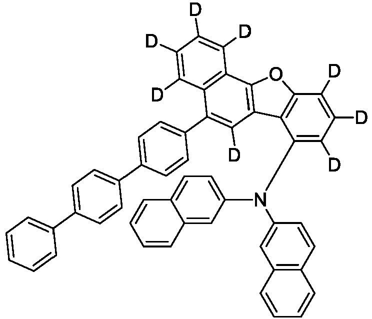 |
| 87 (72%) | 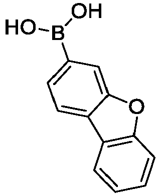 | 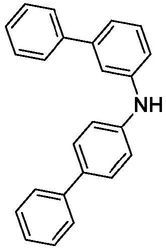 | 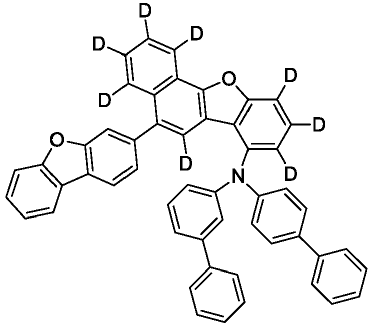 |
| 88 (80%) | 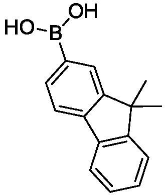 | 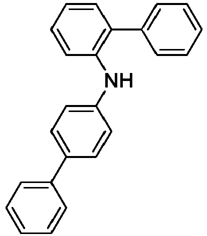 | 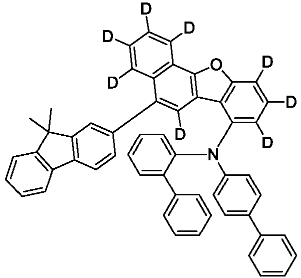 |
| 89 (81%) | 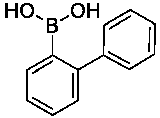 | 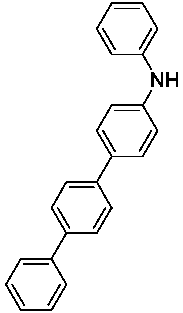 | 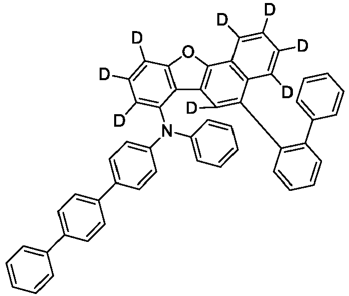 |
| 90 (68%) | 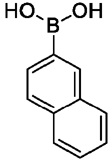 | 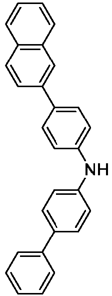 | 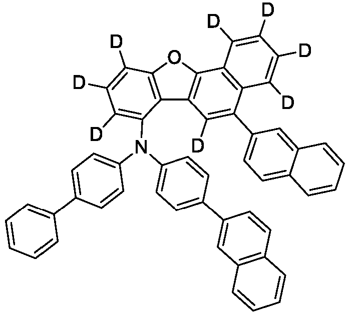 |
| 91 (90%) | 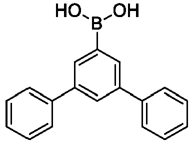 | 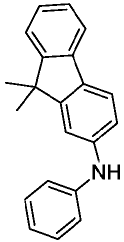 | 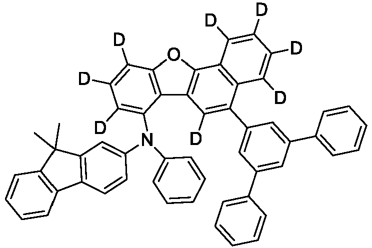 |

### <Preparation Example 6> Preparation of Compound 73

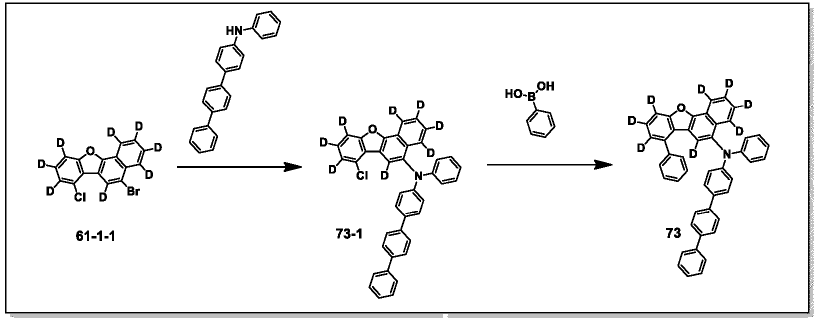

### <Preparation of Compound 73-1>

Compound 73-1 was obtained using Compound 61-1-1 prepared in Preparation Example 5 instead of Compound 1-2 and using N-phenyl-[1,1':4',1''-terphenyl]-4-amine instead of 9,9-dimethyl-N-phenyl-9H-fluoren-2-amine in the preparation method of Compound 13-1 in Preparation Example 2.

### <Preparation of Compound 73>

Compound 73 was obtained using Compound 73-1 instead of Compound 13-1 and using phenylboronic acid instead of Compound 1-1-1 in the preparation method of Compound 13 in Preparation Example 2.

A target compound in the following Table 9 was obtained using Compound A in the following Table 9 instead of N-phenyl-[1,1':4',1''-terphenyl]-4-amine in Preparation Example 6 and using Compound B in the following Table 9 instead of phenylboronic acid in Preparation Example 6.

**[Table 9]**

| Compound (yield) | Compound A | Compound B | Target compound |
|---|---|---|---|
| 73 (%72) | 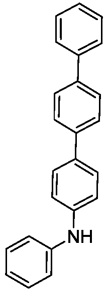 | 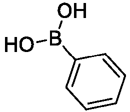 | 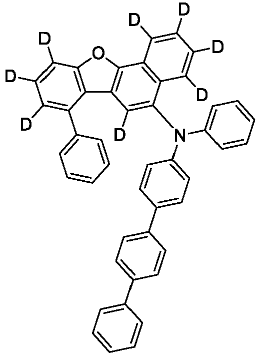 |
| 74 (75%) | 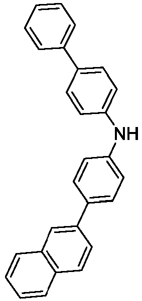 | 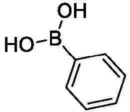 | 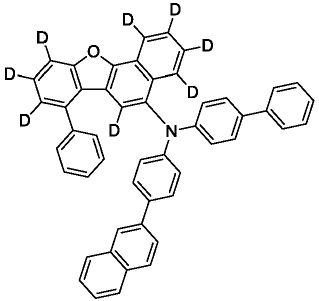 |
| 92 (81%) | 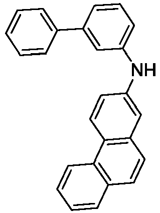 | 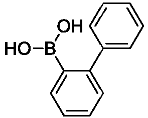 | 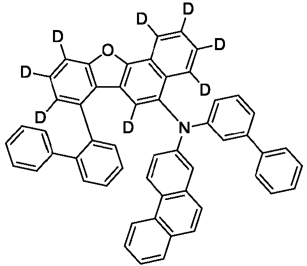 |
| 93 (90%) | 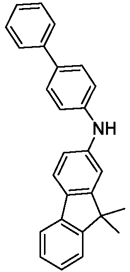 | 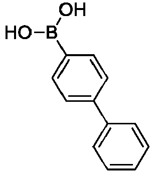 | 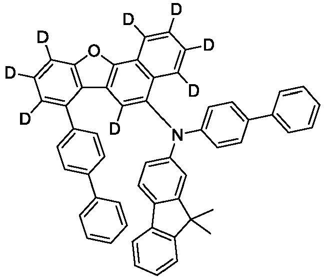 |
| 94 (88%) | 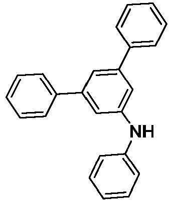 | 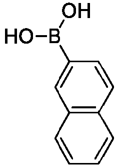 | 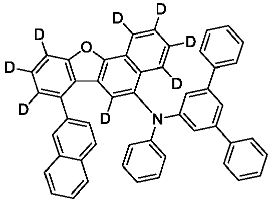 |
| 95 (79%) | 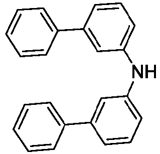 | 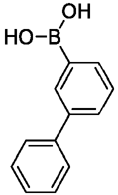 | 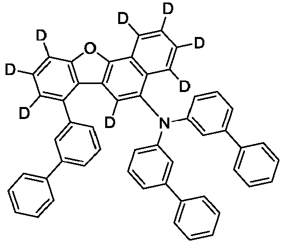 |
| 96 (91%) | 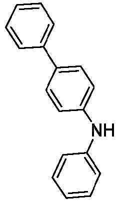 | 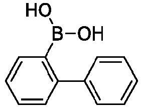 | 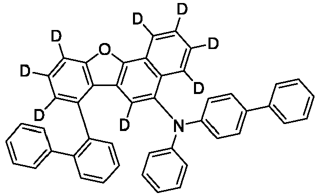 |
| 99 (89%) | 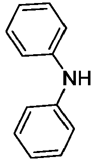 | 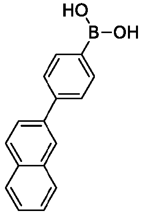 | 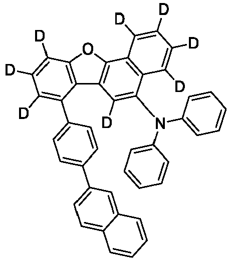 |
| 100 (75%) | 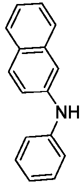 | 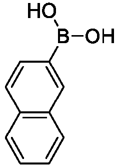 | 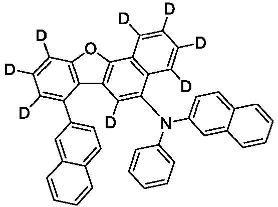 |

### <Preparation Example 7> Preparation of Compound 97

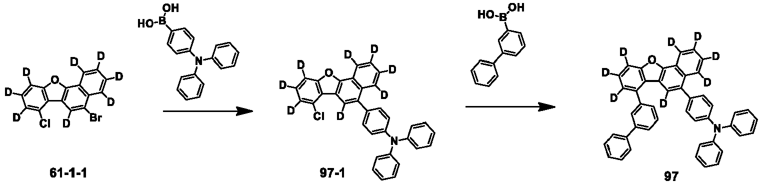

### <Preparation of Compound 97-1>

Compound 97-1 was obtained by a Br-Suzuki reaction using Compound 61-1-1 prepared in Preparation Example 5 and 4-(diphenylamino)phenyl)boronic acid.

### <Preparation of Compound 97>

Compound 97 was obtained by a Cl-Suzuki reaction using Compound 97-1 and [1,1'-biphenyl]-3-ylboronic acid.

A target compound in the following Table 10 was obtained using Compound A in the following Table 10 instead of 4-(diphenylamino)phenylboronic acid in Preparation Example 7 and using Compound B instead of [1,1'-biphenyl]-3-ylboronic acid in Preparation Example 7.

**[Table 10]**

| Compound (yield) | Compound A | Compound B | Target compound |
|---|---|---|---|
| 98 (86%) | 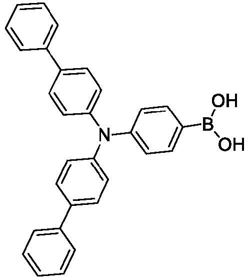 | 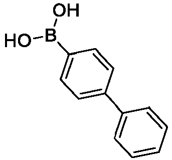 | 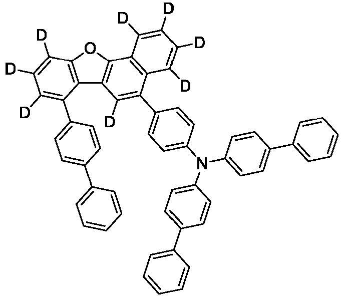 |
| 75 (68%) | 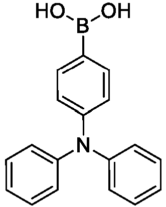 | 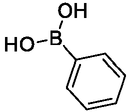 | 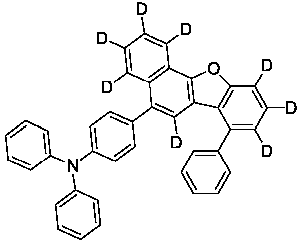 |
| 76 (72%) | 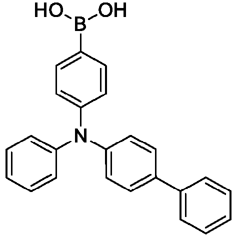 | 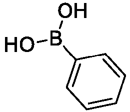 | 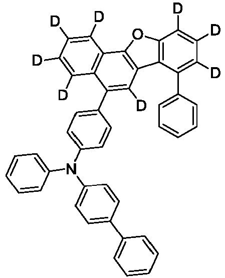 |
| 77 (75%) | 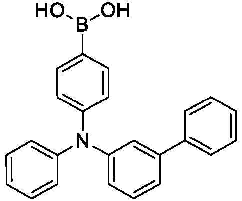 | 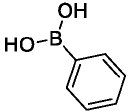 | 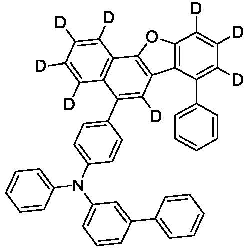 |
| 78 (77%) | 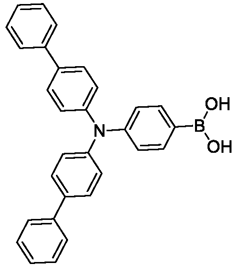 | 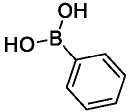 | 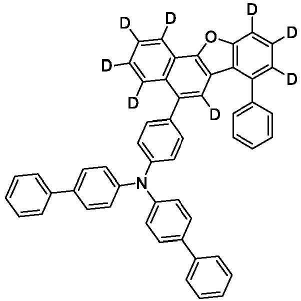 |
| 79 (79%) | 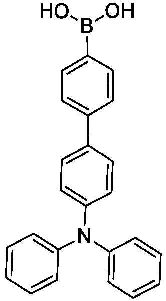 | 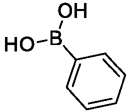 | 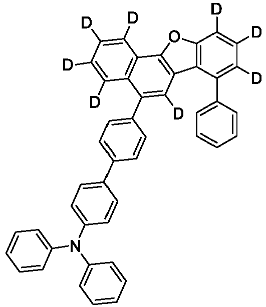 |
| 80 (81%) | 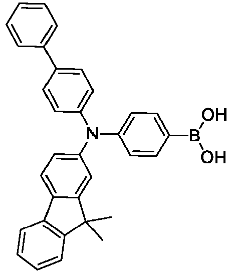 | 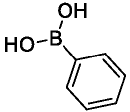 | 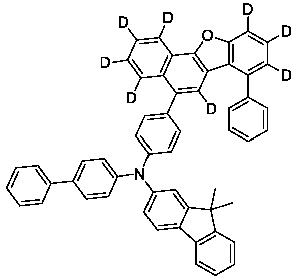 |

### <Preparation Example 8> Preparation of Compound 121

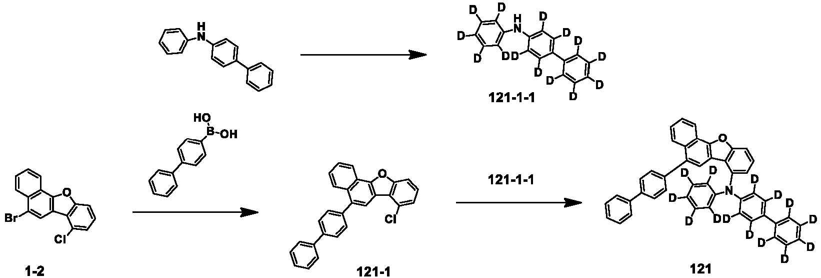

### <Preparation of Compound 121-1-1>

Compound 121-1-1 was obtained using N-phenyl-[1,1'-biphenyl]-4-amine instead of bromobenzene in the preparation method of Compound 1-1-2 in Preparation Example 1.

### <Preparation of Compound 121-1>

Compound 121-1 was obtained by a Br-Suzuki reaction using Compound 1-2 prepared in Preparation Example 1 and [1,1'-biphenyl]-4-ylboronic acid.

### <Preparation of Compound 121>

Compound 121 was obtained by a Buchwald reaction using Compound 121-1 and Compound 121-1-1.

A target compound in the following Table 11 was obtained using Compound A in the following Table 11 instead of [1,1'-biphenyl]-4-ylboronic acid in Preparation Example 8 and using Compound B in the following Table 11 instead of N-phenyl-[1,1'-biphenyl]-4-amine in Preparation Example 8.

**[Table 11]**

| Compound (yield) | Compound A | Compound B | Target compound |
|---|---|---|---|
| 122 (81%) | 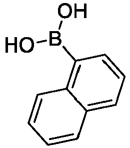 | 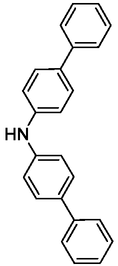 | 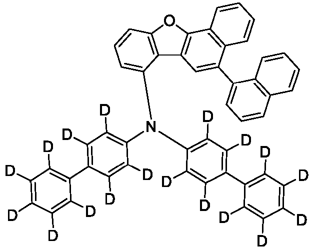 |
| 123 (78%) | 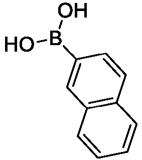 | 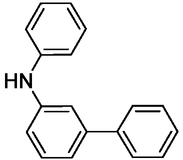 | 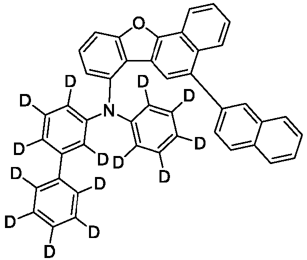 |
| 124 (79%) | 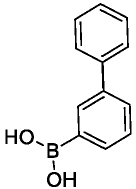 | 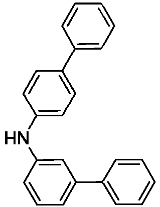 | 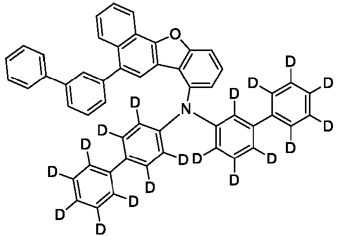 |
| 125 (81%) | 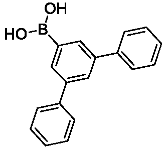 | 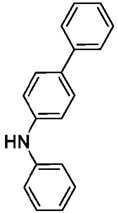 | 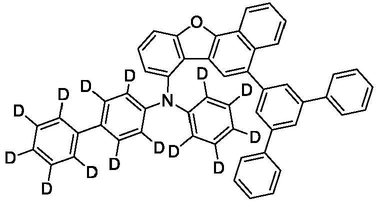 |
| 126 (85%) | 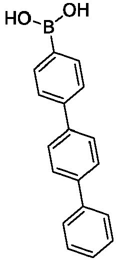 | 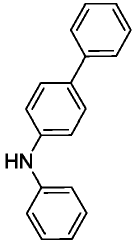 | 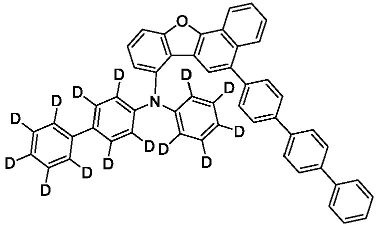 |
| 127 (88%) | 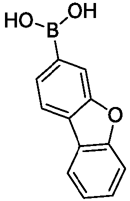 | 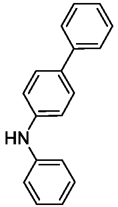 | 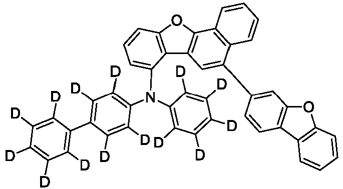 |
| 128 (79%) | 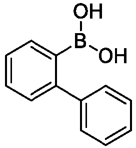 | 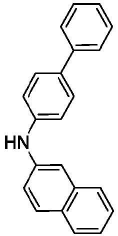 | 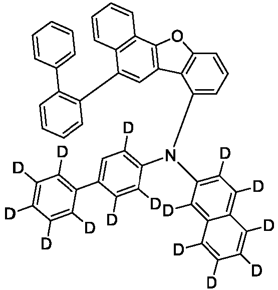 |
| 141 (86%) | 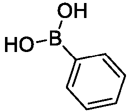 | 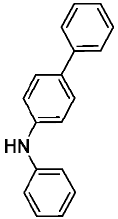 | 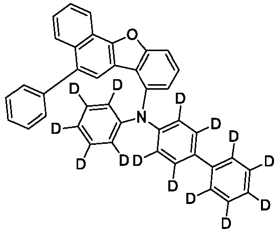 |
| 142 (85%) | 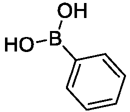 | 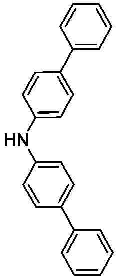 | 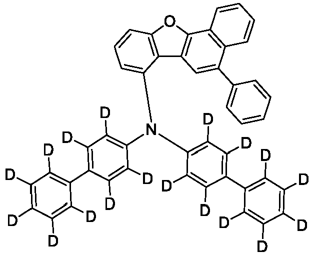 |
| 143 (91%) | 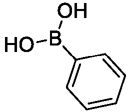 | 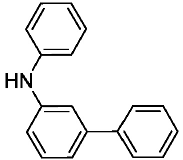 | 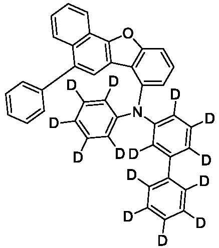 |
| 144 (80%) | 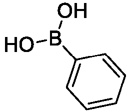 | 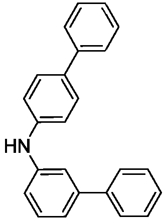 | 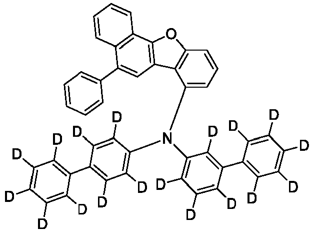 |
| 145 (79%) | 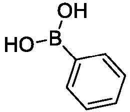 | 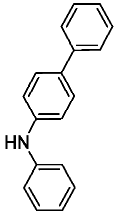 | 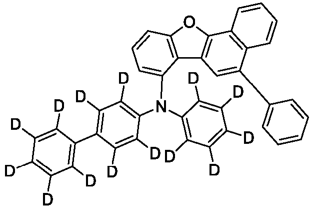 |
| 146 (76%) | 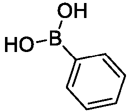 | 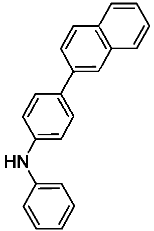 | 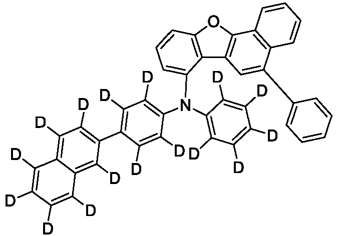 |
| 147 (72%) | 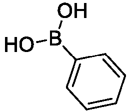 | 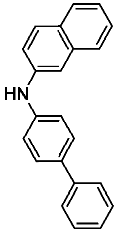 | 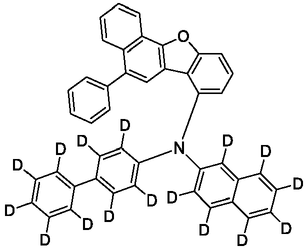 |
| 148 (71%) | 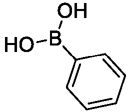 | 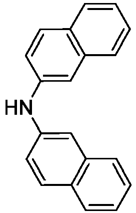 | 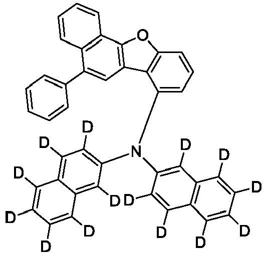 |
| 149 (76%) | 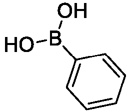 | 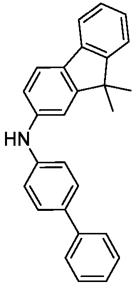 | 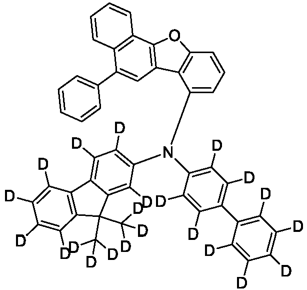 |
| 150 (89%) | 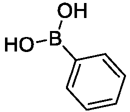 | 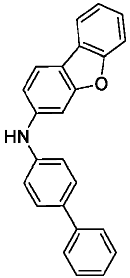 | 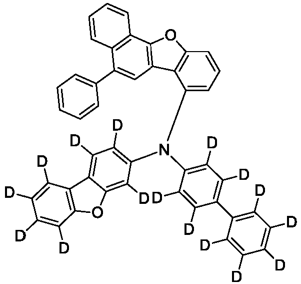 |
| 151 (81%) | 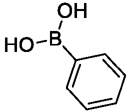 | 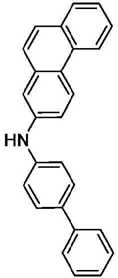 | 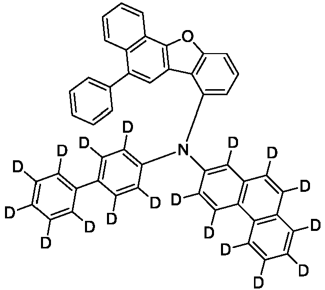 |
| 152 (76%) | 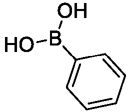 | 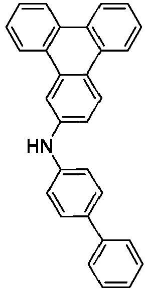 | 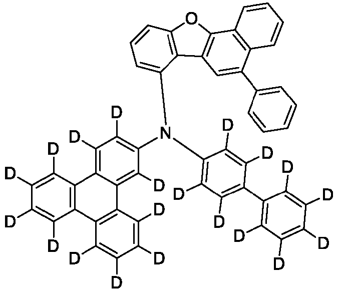 |

Compound A in the following Table 12 is prepared by the synthesis method of Compound 121-1 in Preparation Example 8, and a target compound in the following Table 12 was obtained using Compound A in the following Table 12 instead of 9,9-dimethyl-N-phenyl-9H-fluoren-2-amine in the preparation method in Preparation Example 2 and using Compound B in the following Table 12 instead of Compound 1-1-1 in the preparation method in Preparation Example 2.

**[Table 12]**

| Compound (yield) | Compound A | Compound B | Target compound |
|---|---|---|---|
| 129 (76%) | 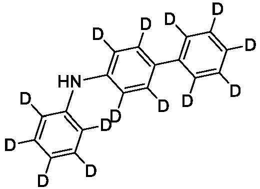 | 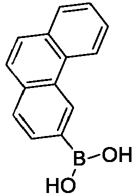 | 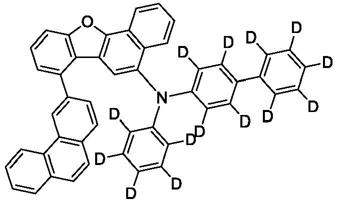 |
| 130 (69%) | 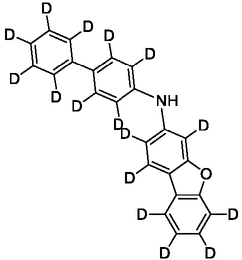 | 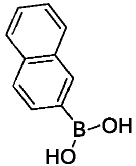 | 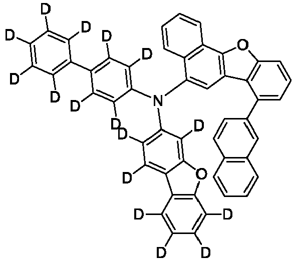 |
| 131 (81%) | 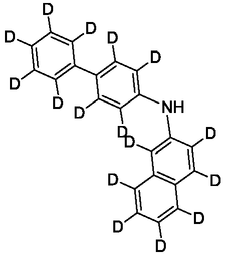 | 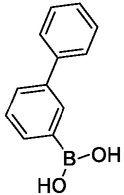 | 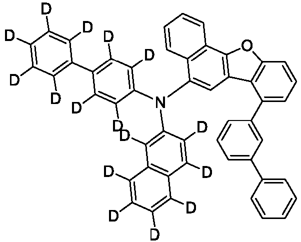 |
| 132 (80%) | 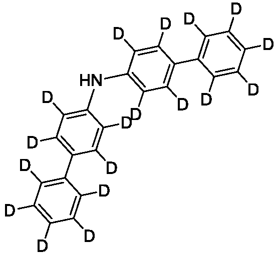 | 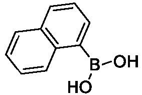 | 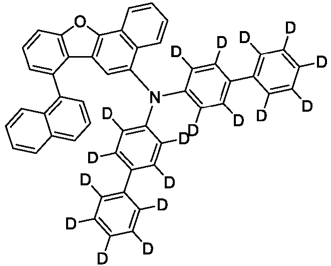 |
| 133 (79%) | 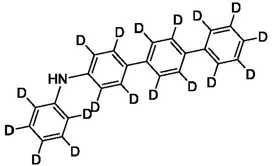 | 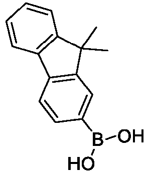 | 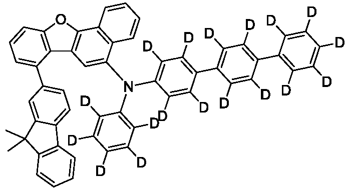 |
| 134 (77%) | 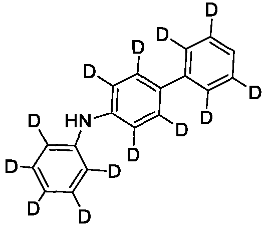 | 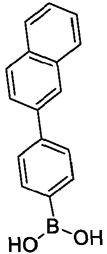 | 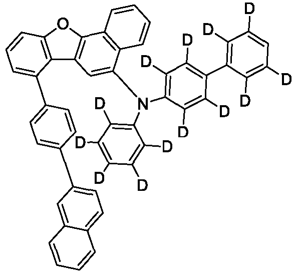 |
| 135 (75%) | 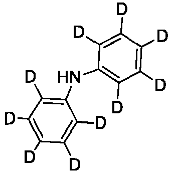 | 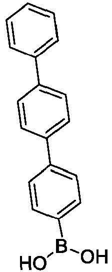 | 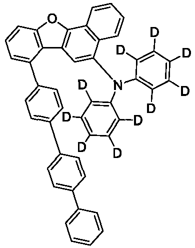 |
| 136 (79%) | 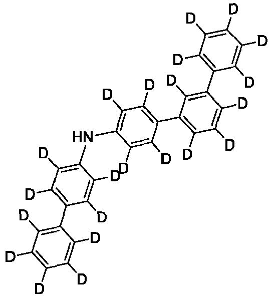 | 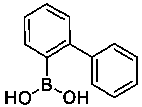 | 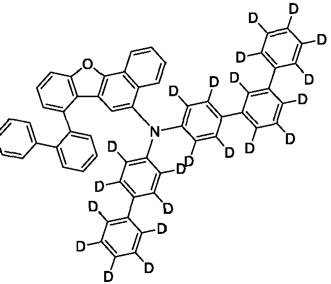 |
| 153 (75%) | 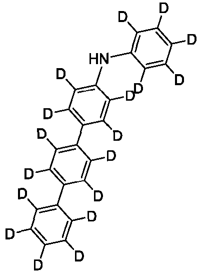 | 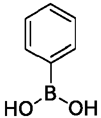 | 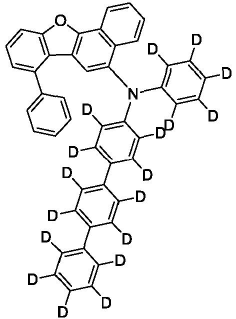 |
| 154 (68%) | 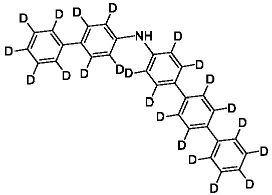 | 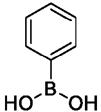 | 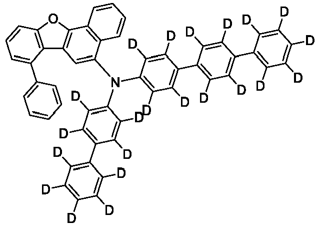 |
| 155 (67%) | 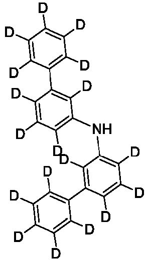 | 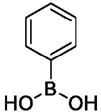 | 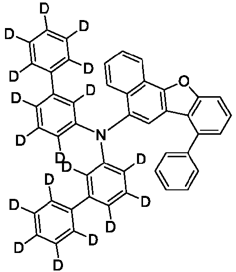 |
| 156 (75%) | 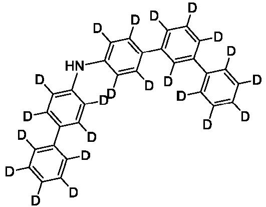 | 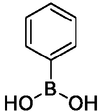 | 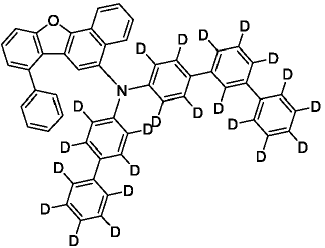 |

### <Preparation Example 9> Preparation of Compound 137

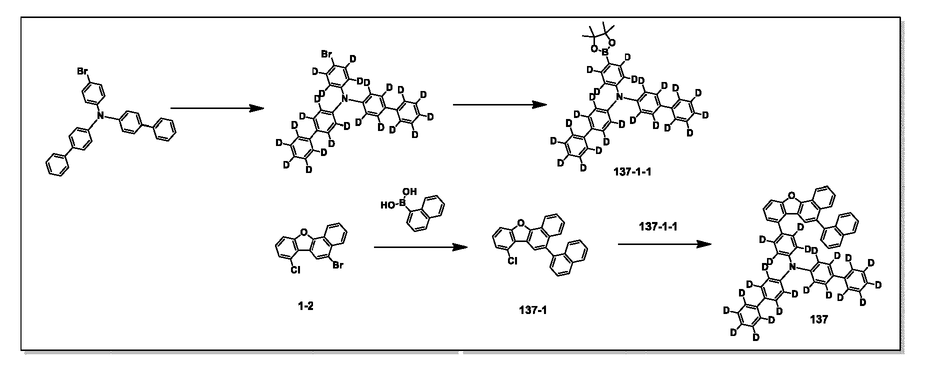

### <Preparation of Compound 137-1-1>

Compound 137-1-1 instead of Compound 1-1-1 was obtained using N-([1,1'-biphenyl]-4-yl)-N-(4-bromophenyl)-[1,1'-biphenyl]-4-amine instead of bromobenzene in Preparation Example 1.

### <Preparation of Compound 137-1>

Compound 137-1 was obtained by a Br-Suzuki reaction using Compound 1-2 prepared in Preparation Example 1 and naphthalen-1-ylboronic acid.

### <Compound 137>

Compound 137 was obtained by a Cl-Suzuki reaction using Compound 137-1 and Compound 137-1-1.

A target compound in the following Table 13 was obtained using Compound A in the following Table 13 instead of N-([1,1'-biphenyl]-4-yl)-N-(4-bromophenyl)-[1,1'-biphenyl]-4-amine in Preparation Example 9 and using Compound B instead of naphthalen-1-ylboronic acid in Preparation Example 9.

**[Table 13]**

| Compound (yield) | Compound A | Compound B | Target compound |
|---|---|---|---|
| 138 (75%) | 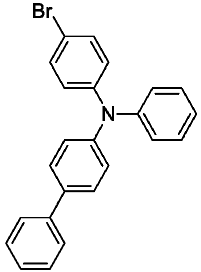 | 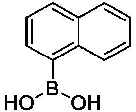 | 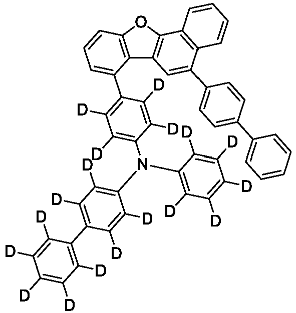 |
| 139 (76%) | 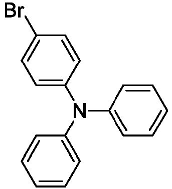 | 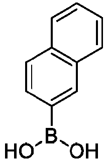 | 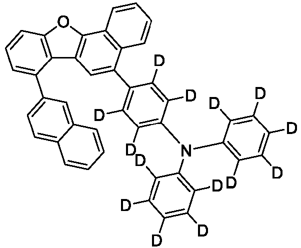 |
| 140 (77%) | 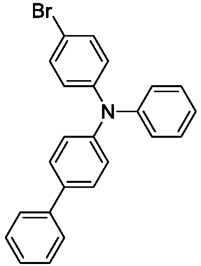 | 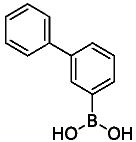 | 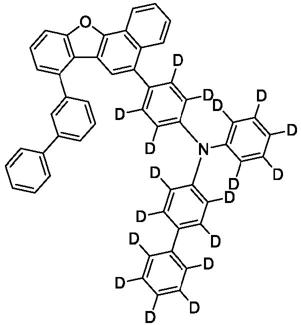 |
| 157 (90%) | 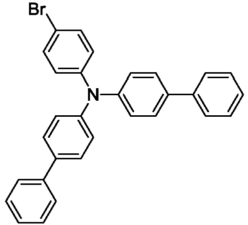 | 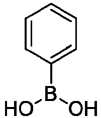 | 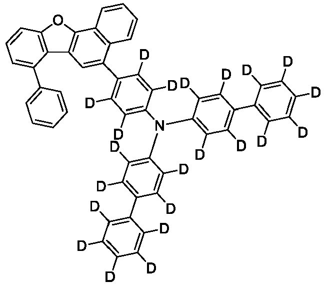 |
| 158 (86%) | 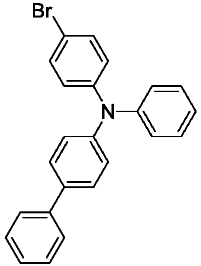 | 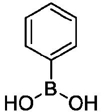 | 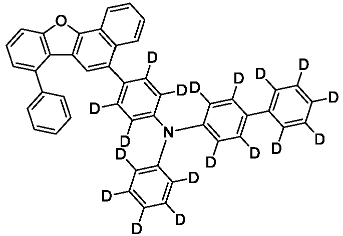 |
| 159 (75%) | 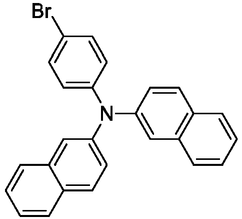 | 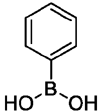 | 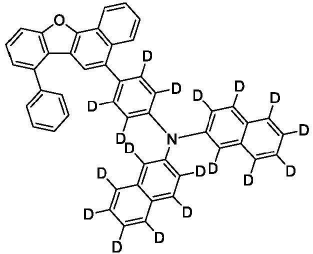 |
| 160 (77%) | 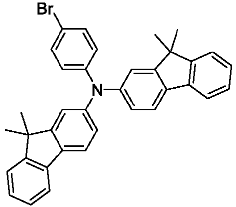 | 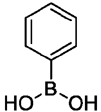 | 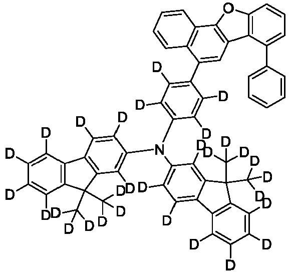 |

### <Preparation Example 10> Preparation of Compound 102

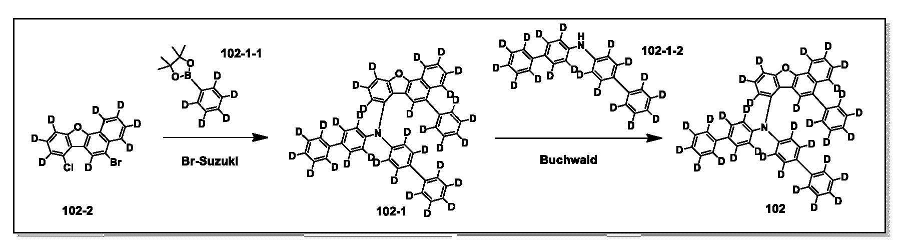

The starting material for the preparation of Compound 102, Compound 102-2, Compound 102-1-1, and Compound 102-1-2 are all compounds having a history of preparation in the above Preparation Examples.

In Preparation Example 10, when Compound A in the following Table 14 is used instead of bromobenzene used to produce Compound 102-1-1 and Compound B in the following Table 14 is used instead of di([1,1'-biphenyl]-4-yl)amine used to produce Compound 102-1-2, an intermediate is formed, and a target compound in the following Table 14 can be obtained instead of Target Compound 102 in Preparation Example 10.

**[Table 14]**

| Compound (yield) | Compound A | Compound B | Target compound |
|---|---|---|---|
| 102 (68%) | 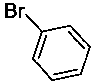 | 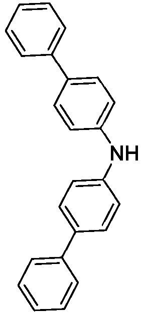 | 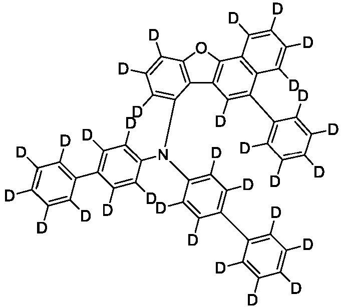 |
| 103 (71%) | 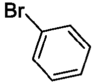 | 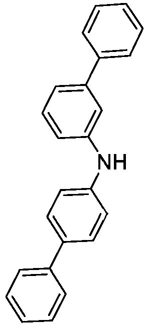 | 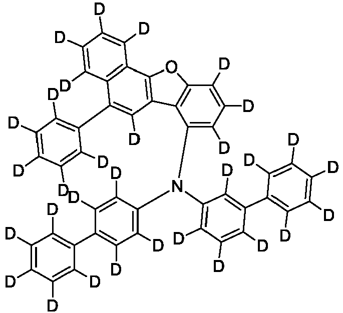 |
| 104 (89%) | 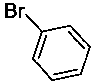 | 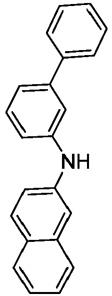 | 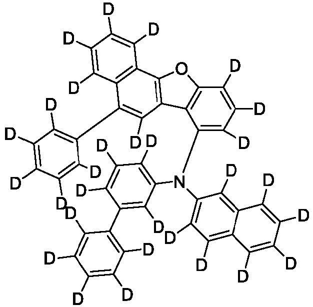 |
| 105 (78%) | 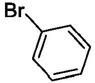 | 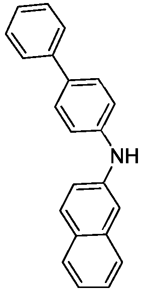 | 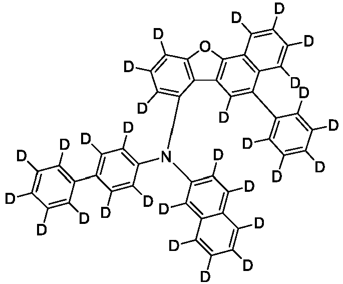 |
| 106 (76%) | 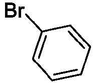 | 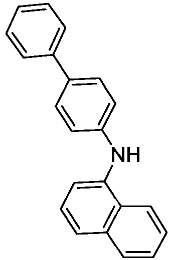 | 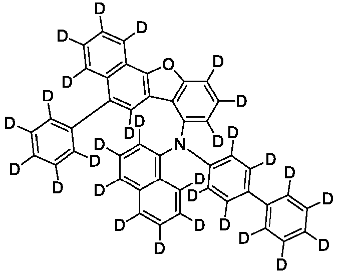 |
| 107 (73%) | 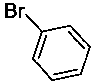 | 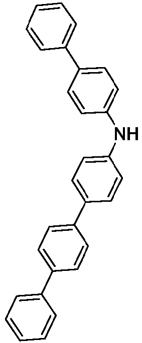 | 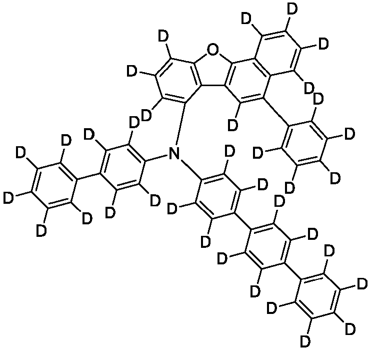 |
| 108 (80%) | 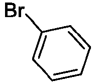 | 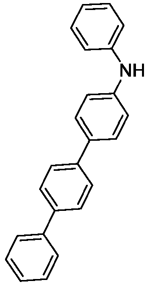 | 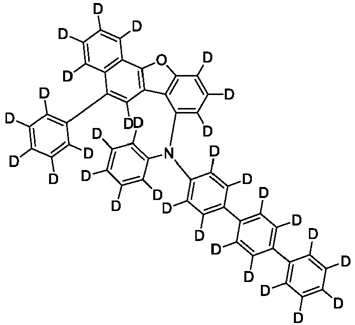 |
| 109 (90%) | 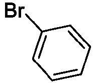 | 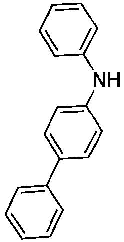 | 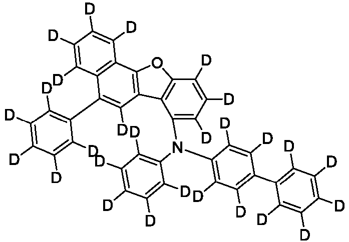 |
| 110 (86%) | 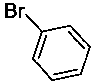 | 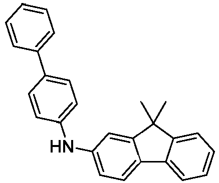 | 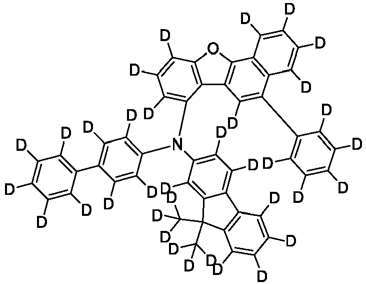 |
| 111 (77%) | 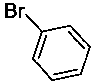 | 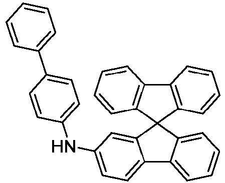 | 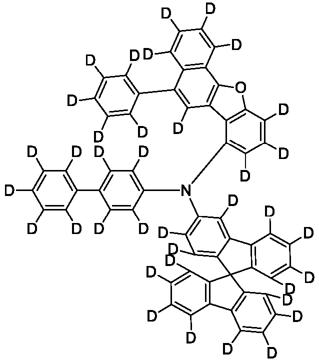 |
| 112 (71%) | 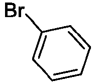 | 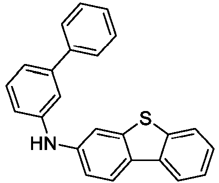 | 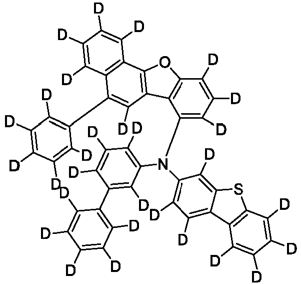 |

### <Preparation Example 11> Preparation of Compound 113

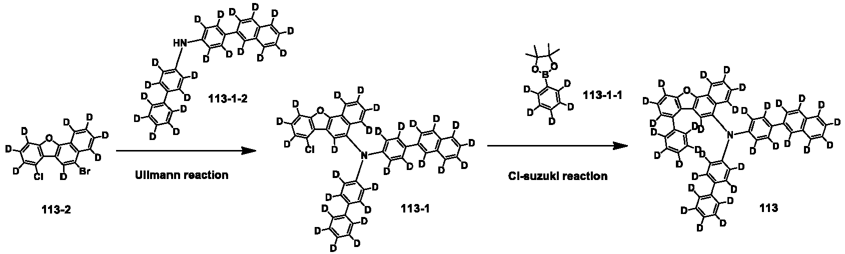

Preparation Example 11 can also be described as in Preparation Example 10.

Compound A in the following Table 15 is used instead of N-phenyl-[1,1'-biphenyl]-4-amine in Preparation Example 8, and Compound B is used instead of bromobenzene in Preparation Example 1 to form an intermediate and produce a target compound in the following Table 15 according to Preparation Example 11.

**[Table 15]**

| Compound (yield) | Compound A | Compound B | Target compound |
|---|---|---|---|
| 113 (86%) | 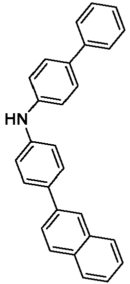 | 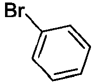 | 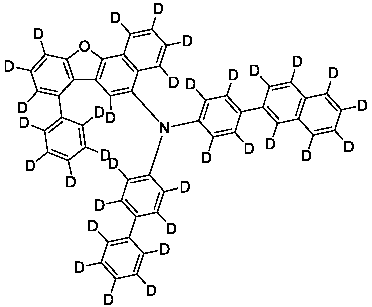 |
| 114 (85%) | 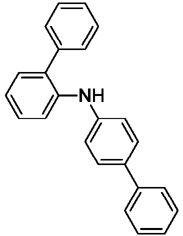 | 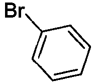 | 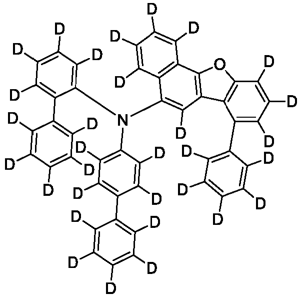 |
| 115 (79%) | 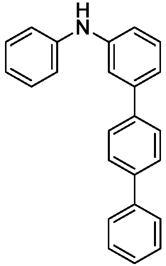 | 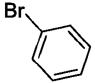 | 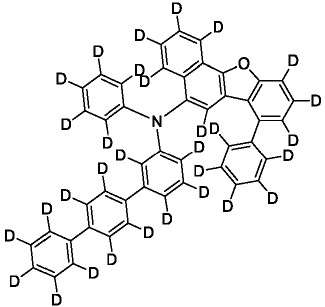 |
| 116 (80%) | 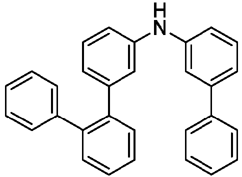 | 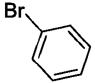 | 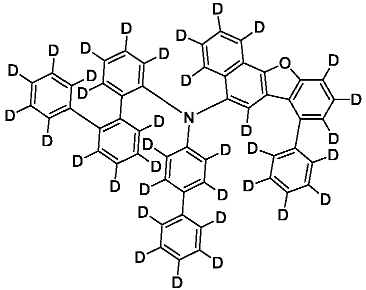 |
| 117 (83%) | 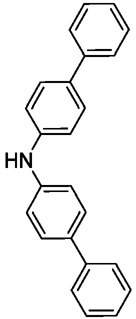 | 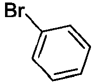 | 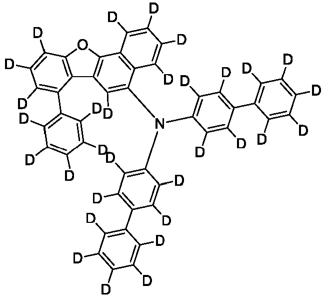 |
| 118 (75%) | 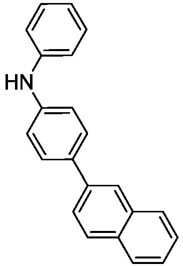 | 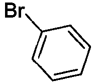 | 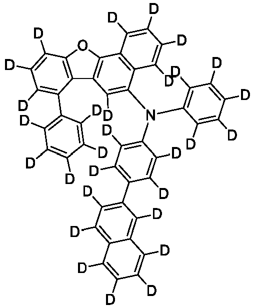 |
| 119 (89%) | 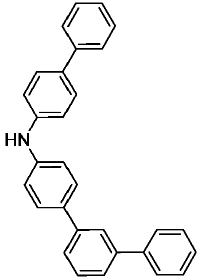 | 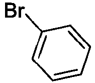 | 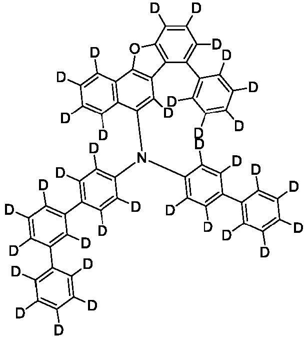 |
| 120 (82%) | 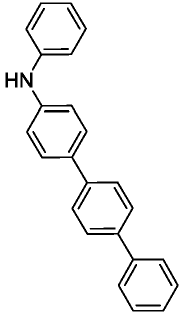 | 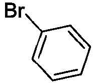 | 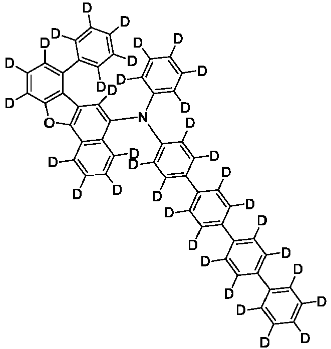 |

### <Preparation Example 12> Preparation of Compound 161

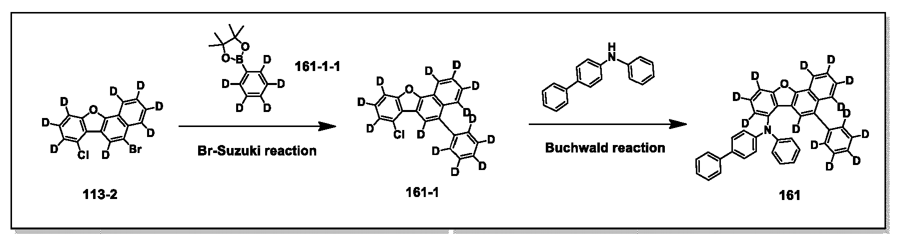

Preparation Example 12 can also be described as above. Compound A in the following Table 16 forms an intermediate by the same preparation method as in the preparation of Compound 1-1-1 in Preparation Example 1.

A target compound in the following Table 16 was obtained using the intermediate instead of Compound 161-1-1 in Preparation Example 12 and using Compound B in the following Table 16 instead of N-phenyl-[1,1'-biphenyl]-4-amine in Preparation Example 12.

**[Table 16]**

| Compound (yield) | Compound A | Compound B | Target compound |
|---|---|---|---|
| 161 (95%) | 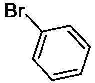 | 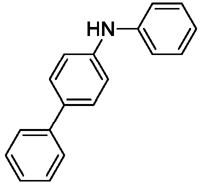 | 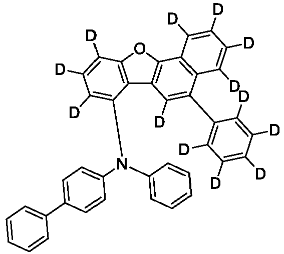 |
| 162 (88%) | 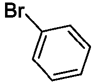 | 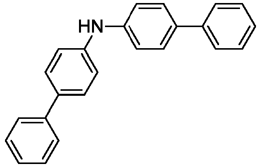 | 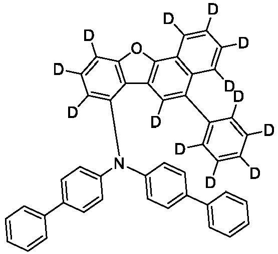 |
| 163 (71%) | 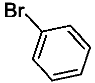 | 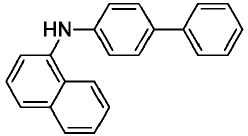 | 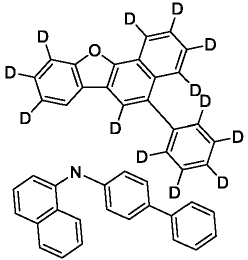 |
| 164 (73%) | 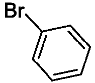 | 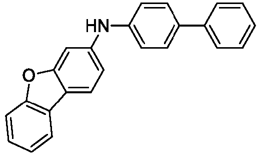 | 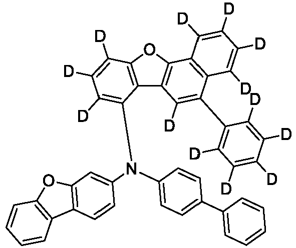 |
| 165 (80%) | 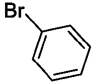 | 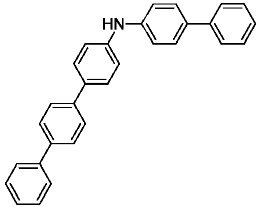 | 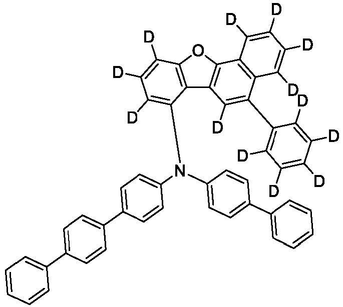 |
| 166 (70%) | 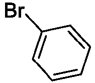 | 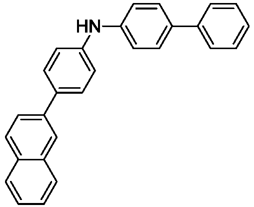 | 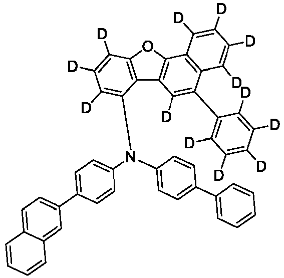 |
| 167 (90%) | 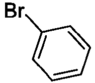 | 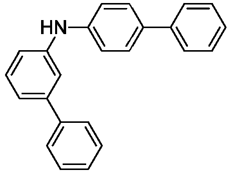 | 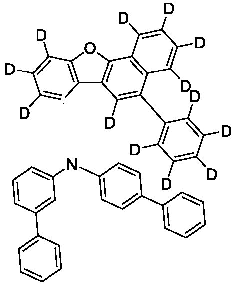 |
| 168 (83%) | 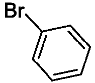 | 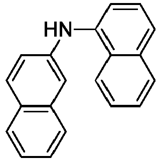 | 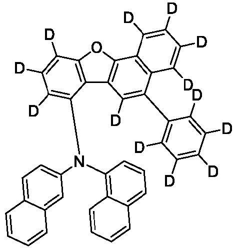 |
| 169 (74%) | 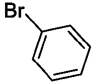 | 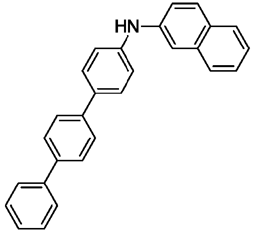 | 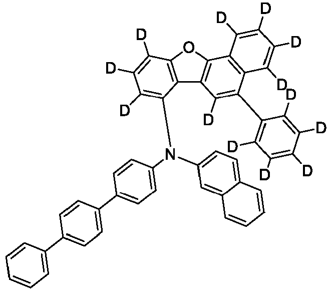 |
| 170 (75%) | 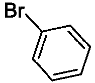 | 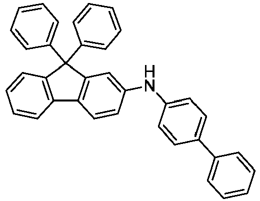 | 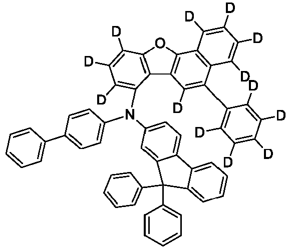 |
| 171 (81%) | 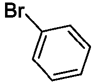 | 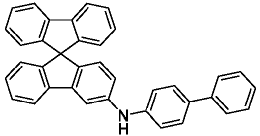 | 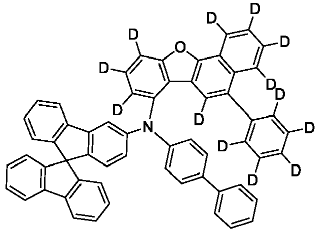 |
| 172 (90%) | 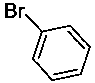 | 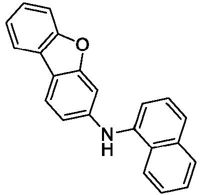 | 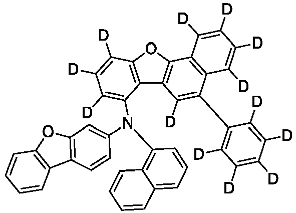 |
| 173 (83%) | 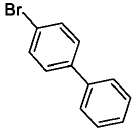 | 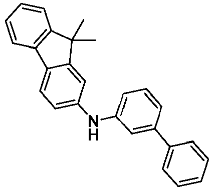 | 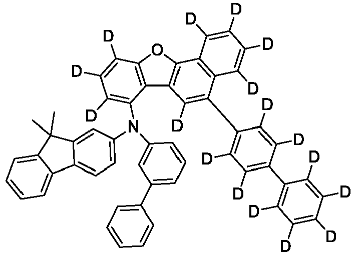 |
| 174 (85%) | 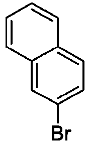 | 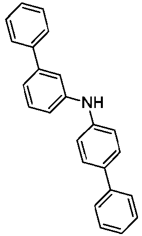 | 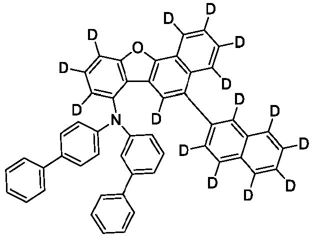 |
| 175 (78%) | 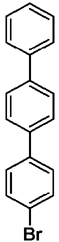 | 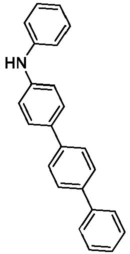 | 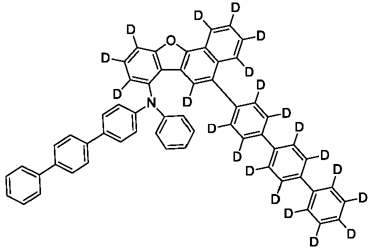 |
| 176 (81%) | 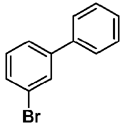 | 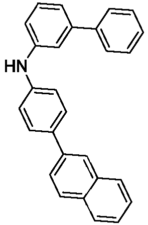 | 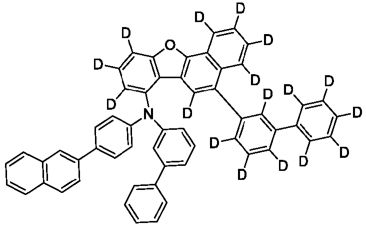 |

### <Preparation Example 13> Preparation of Compound 177

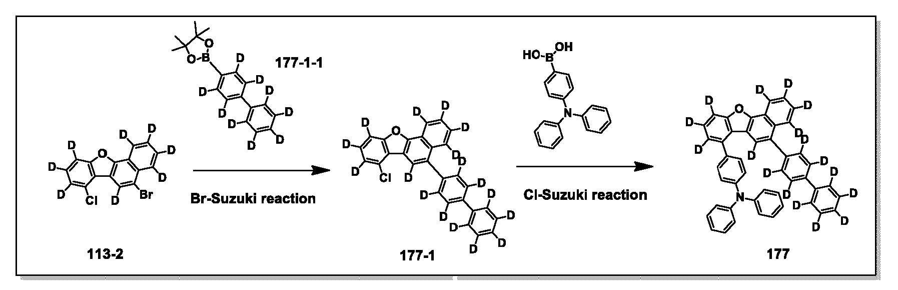

Preparation Example 13 can also be described above.

Compound A in the following Table 17 was used in the synthesis of an intermediate such as Compound 177-1-1 in Preparation Example 13, and Compound B in the following Table 17 was used instead of 4-(diphenylamino)phenylboronic acid in Preparation Example 13 to obtain a target compound in the following Table 17.

**[Table 17]**

| Compound (yield) | Compound A | Compound B | Target compound |
|---|---|---|---|
| 177 (78%) | 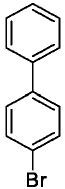 | 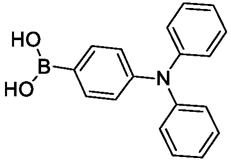 | 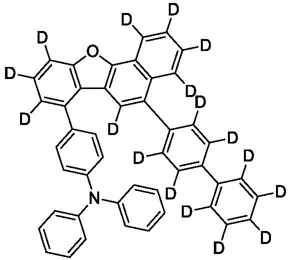 |
| 178 (81%) | 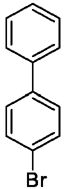 | 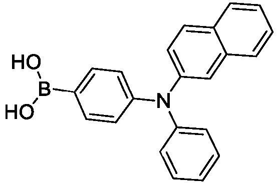 | 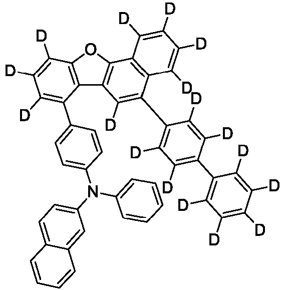 |
| 179 (83%) | 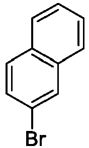 | 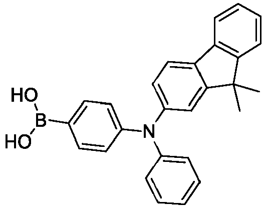 | 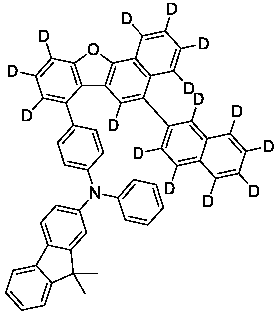 |
| 180 (85%) | 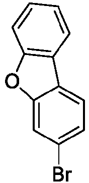 | 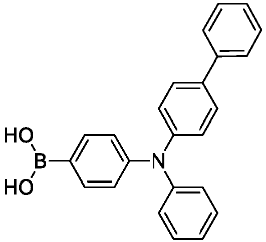 | 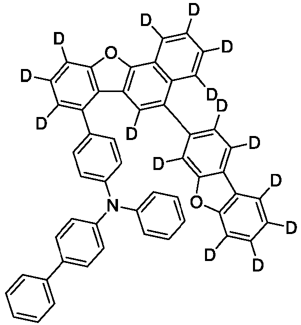 |

### <Preparation Example 14> Preparation of Compound 181

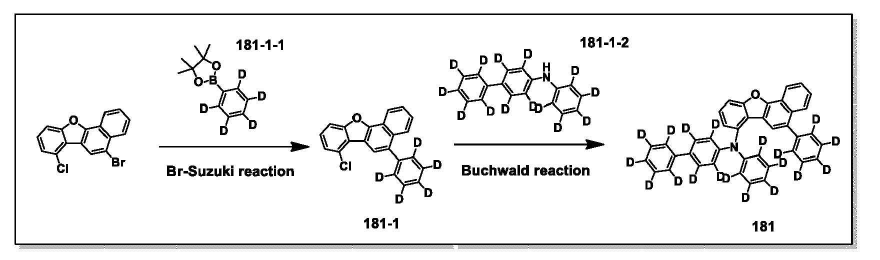

Preparation Example 14 can also be synthesized using intermediates having a history of previously being synthesized.

An intermediate that replaces Compound 181-1-1 of Preparation Example 14 was synthesized by Compound A in the following Table 18, an intermediate that replaces Compound 181-1-2 was synthesized by Compound B in the following Table 18, and a target compound in the following Table 18 was obtained by performing preparation in the same manner as in Preparation Example 14.

**[Table 18]**

| Compound (yield) | Compound A | Compound B | Target compound |
|---|---|---|---|
| 181 (72%) | 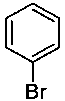 | 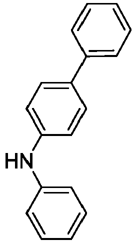 | 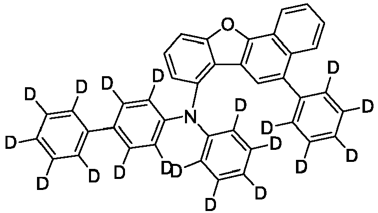 |
| 182 (69%) | 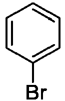 | 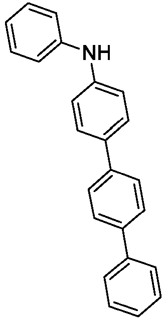 | 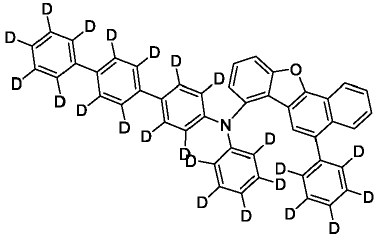 |
| 183 (73%) | 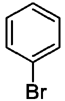 | 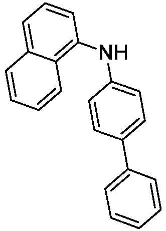 | 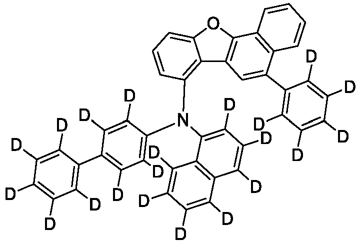 |
| 184 (75%) | 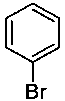 | 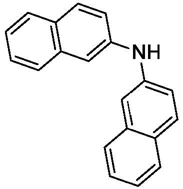 | 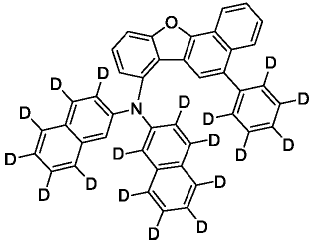 |
| 185 (80%) | 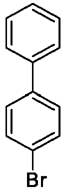 | 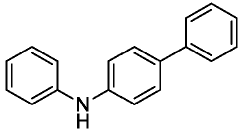 | 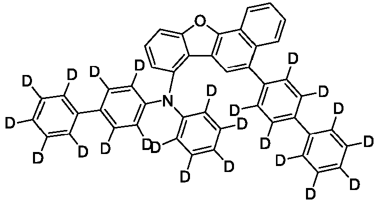 |
| 186 (90%) | 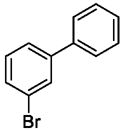 | 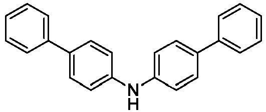 | 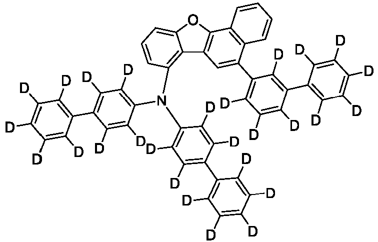 |
| 187 (89%) | 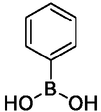 | 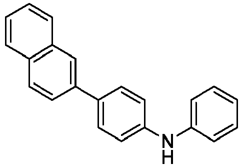 | 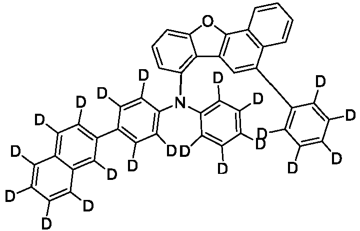 |
| 188 (80%) | 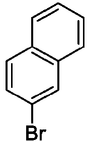 | 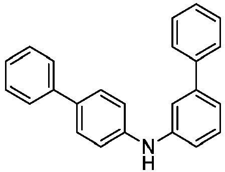 | 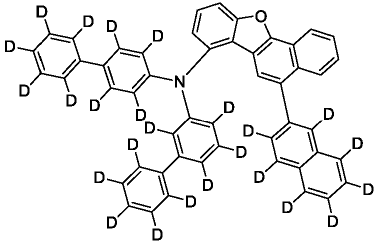 |

### <Preparation Example 15> Synthesis of Compound 189

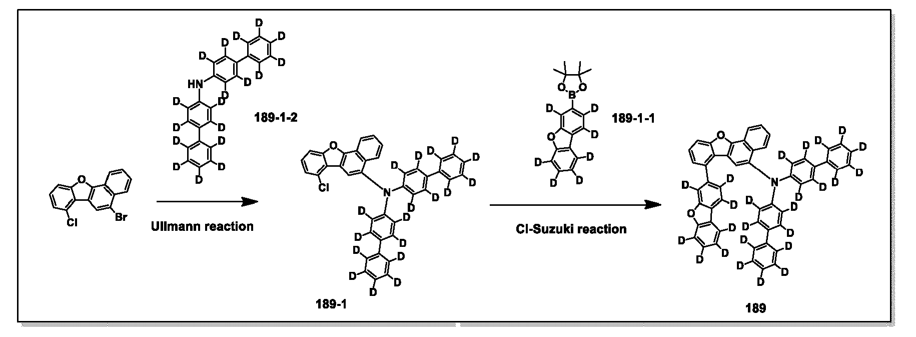

Preparation Example 15 is the same as the preparation method described in Prepration Example 2, and Compound 189-1-2 is prepared by the preparation method of Compound 121-1-1 in Preparation Example 8.

The intermediates that replace Compound 189-1-2 and Compound 189-1-1 in Preparation Example 15 are prepared using Compounds A and B in the following Table 19, and a target compound in the following Table 19 is produced instead of Compound 189 according to Preparation Example 15.

**[Table 19]**

| Compound (yield) | Compound B | Compound A | Target compound |
|---|---|---|---|
| 189 (75%) | 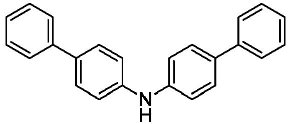 | 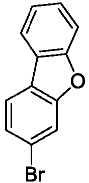 | 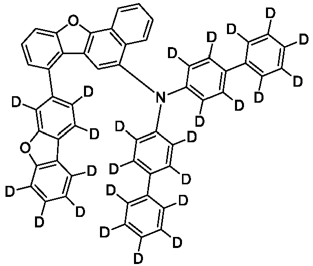 |
| 190 (73%) | 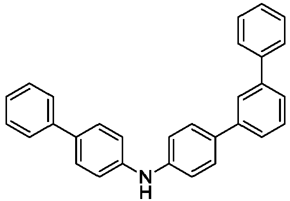 | 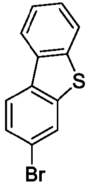 | 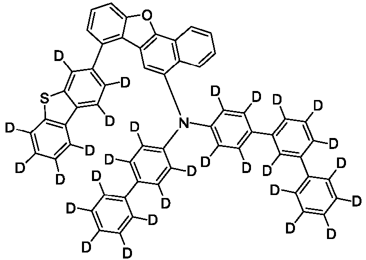 |
| 191 (70%) | 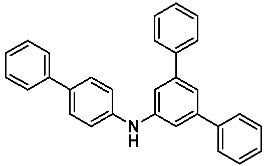 | 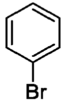 | 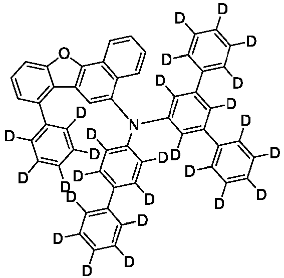 |
| 192 (82%) | 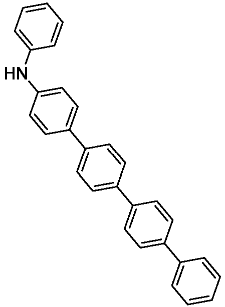 | 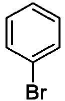 | 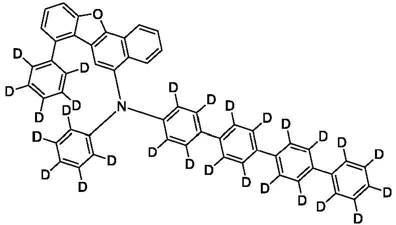 |
| 193 (85%) | 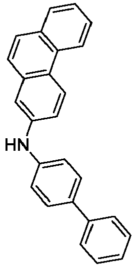 | 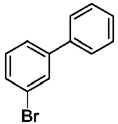 | 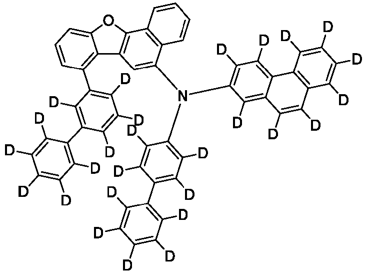 |
| 194 (76%) | 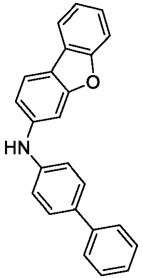 | 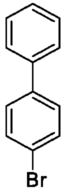 | 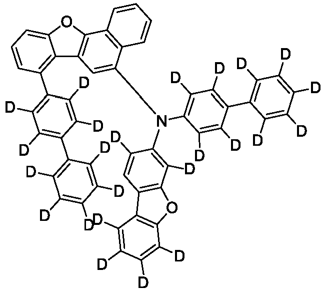 |
| 195 (77%) | 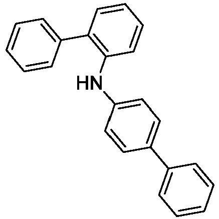 | 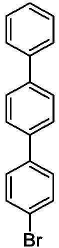 | 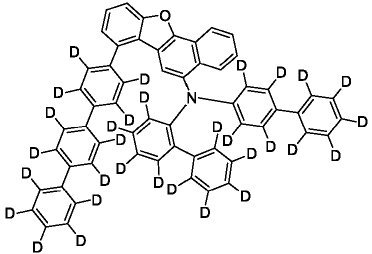 |
| 196 (80%) | 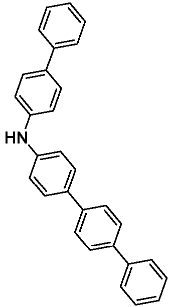 | 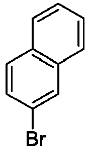 | 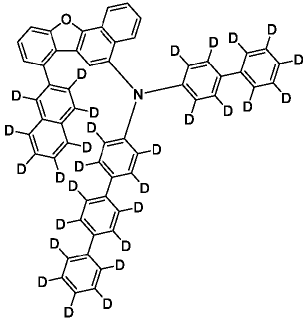 |

### <Preparation Example 16> Synthesis of Compound 197

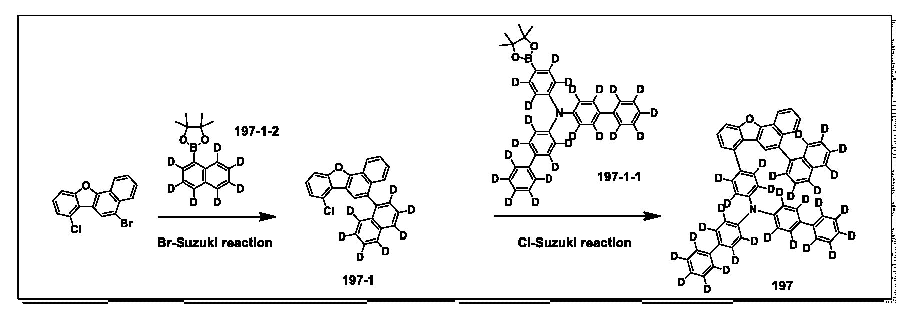

Preparation Example 16 has the same preparation method as that of Preparation Example 4, and Compounds 197-1-2 and 197-1-1 are both compounds having the above preparation history.

The intermediates that would replace Compound 197-1-2 and Compound 197-1-1 were obtained using Compounds A and B in the following Table 20 as starting materials, and a target compound in the following Table 20 was obtained instead of Compound 197 in the same manner as in Preparation Example 16.

**[Table 20]**

| Compound (yield) | Compound A | Compound B | Target compound |
|---|---|---|---|
| 197 (86%) | 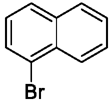 | 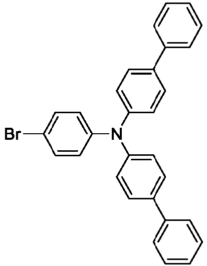 | 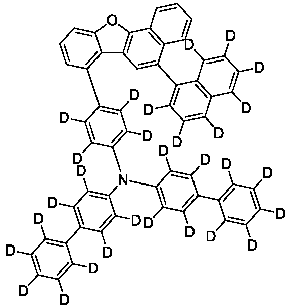 |
| 198 (71%) | 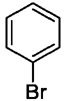 | 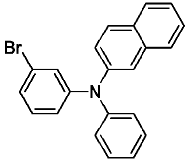 | 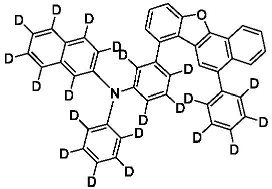 |
| 199 (91%) | 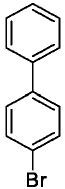 | 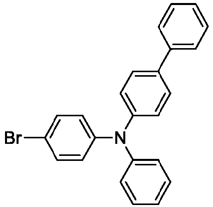 | 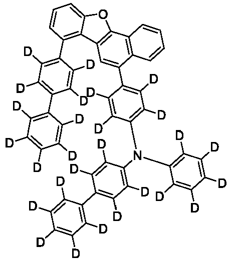 |
| 200 (80%) | 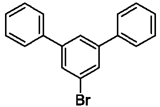 | 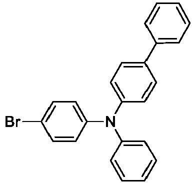 | 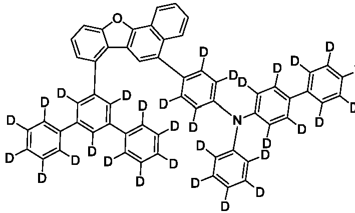 |

### <Preparation Example 17> Preparation of Compound 201

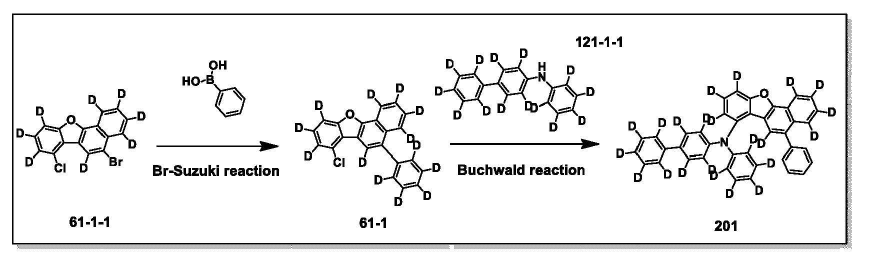

Preparation Example 17 can also be synthesized using intermediates having a history of being synthesized as described above.

A target compound in the following Table 21 was synthesized by performing synthesis in the same manner as in Preparation Example 17, except that an intermediate prepared using Compound A in the following Table 21 instead of phenylboronic acid, and using Compound B in the following Table 21 instead of Compound 121-1-1 was used in Preparation Example 17.

**[Table 21]**

| Compound (yield) | Compound A | Compound B | Target compound |
|---|---|---|---|
| 201 (85%) | 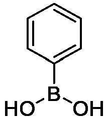 | 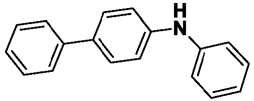 | 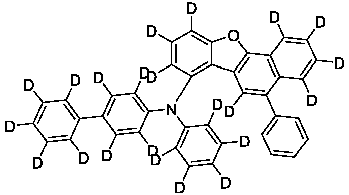 |
| 202 (82%) | 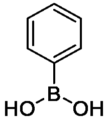 | 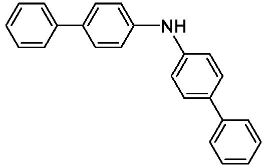 | 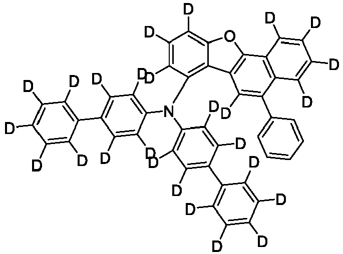 |
| 205 (80%) | 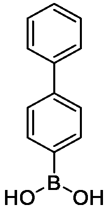 | 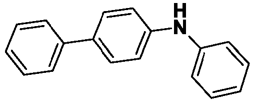 | 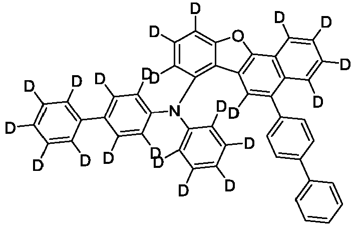 |
| 209 (75%) | 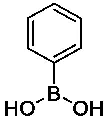 | 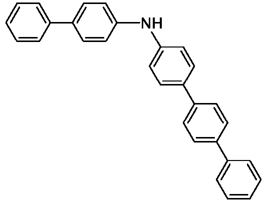 | 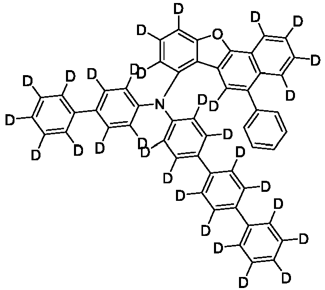 |
| 210 (80%) | 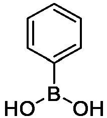 | 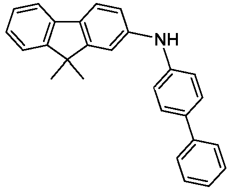 | 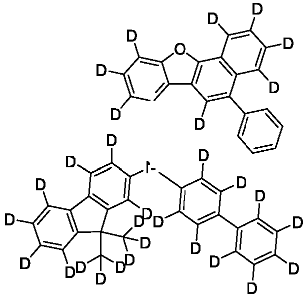 |
| 211 (78%) | 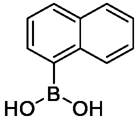 | 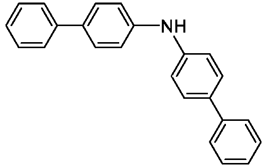 | 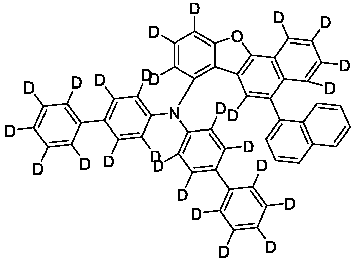 |

### <Preparation Example 18> Synthesis of Compound 213

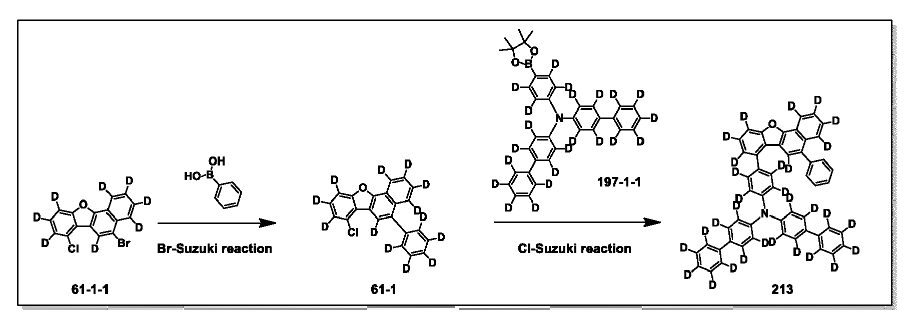

Preparation Example 18 can also be synthesized using intermediates having a history of being synthesized as described above.

A target compound in the following Table 22 was prepared by performing synthesis in the same manner as in Preparation Example 18, except that an intermediate prepared using Compound A in the following Table 22 instead of phenylboronic acid and using Compound B in the following Table 22 instead of Compound 197-1-1 was used in Preparation Example 18.

**[Table 22]**

| Compound (yield) | Compound A | Compound B | Target compound |
|---|---|---|---|
| 213 (86%) | 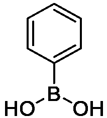 | 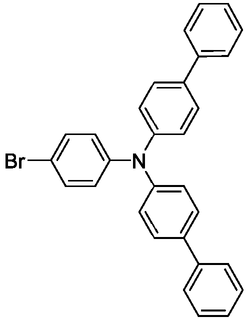 | 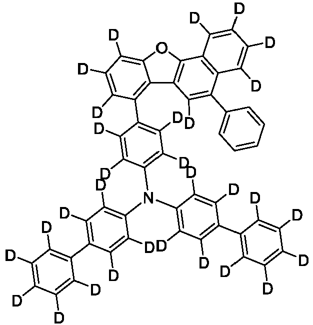 |
| 214 (84%) | 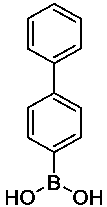 | 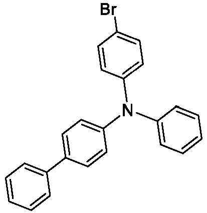 | 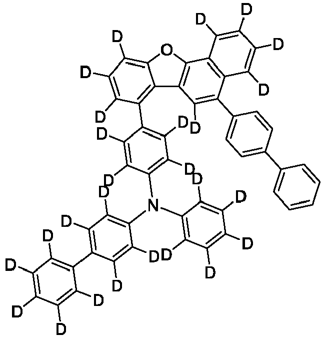 |
| 215 (85%) | 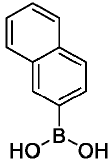 | 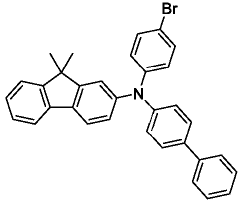 | 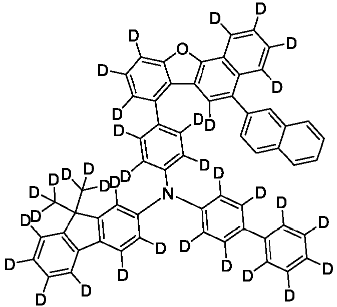 |

Heterocyclic compounds corresponding to Chemical Formula 1 other than the compounds described in Preparation Examples 1 to 18 and Tables 1 to 22 were also prepared in the same manner as in the above-described preparation examples.

The synthetic confirmation data of the compounds prepared above are as shown in the following Tables 23 and 24. Table 23 shows the measured values of ¹H NMR(CDCl₃, 200 MHz), and Table 24 shows the measured values of a field desorption mass spectrometer (FD-MS).

**[Table 23]**

| NO | ¹H NMR(CDCl₃, 200MHz) |
|---|---|
| 1 | 8.55(1H, d), 8.18(d, 1H), 6.63(s, 1H), 7.55(m, 2H), 7.20~7.13(m, 5H), 7.02(d, 1H). 6.81(m, 2H), 7.71~6.63(m, 4H), 6.33(d, 1H) |
| 3 | 8.55(d, 1H), 8.18(d, 1H), 6.63(s, 1H), 7.55~7.51(m, 9H), 7.25~7.20(m, 7H), 7.02(d, 1H), 6.81(m, 1H), 6.69~6.63(m, 4H), 6.33(d, 1H) |
| 4 | 8.55(d, 1H), 8.18(d, 1H), 7.71(s, 1H), 7.55~7.41(m, 6H), 7.13 (m, 1H), 7.02(d, 1H), 6.69(m, 4H), 6.33(d, 1H) |
| 5 | 8.55(m, 1H), 8.18(d, 1H), 7.89(m, 1H), 7.64~7.55(m, 5H), 7.38~7.32(m, 2H), 7.20~7.13(m, 3H), 7.02(d, 1H), 6.81(m, 1H), 6.63 (m, 2H), 6.33(m, 2H) |
| 13 | 8.18(d, 1H), 8.08(d, 1H), 7.87(d, 1H), 7.75(m, 1H), 7.68~7.55(m, 6H), 7.44~7.38(m, 2H), 7.28~7.20(m, 3H), 6.81~6.75(m, 2H), 6.63~6.53(m, 3H), 1.72(s, 6H) |
| 14 | 8.18(d, 1H), 8.08(d, 1H), 7.87(d, 1H), 7.75(d, 1H), 7.62~7.28(m, 15H), 7.28(m, 1H), 6.69~6.75(m, 3H), 6.58(m, 1H), 1.72(s, 6H) |
| 21 | 8.55(m, 1H), 8.18(m, 1H), 7.77~7.71(m, 5H), 7.55~7.36(m, 12H), 7.13(m, 1H), 7.02(m, 1H), 6.69(d, 2H), 6.33(d, 1H) |
| 24 | 8.55(m, 1H), 8.18(d, 1H), 6.63(s, 1H), 7.55~7.41(m, 9H), 7.71~7.13(m, 3H), 7.02(d, 1H), 6.81(t, 1H), 7.71~6.63(m, 4H), 6.33(d, 1H) |
| 29 | 8.55(m, 1H), 8.18(d, 1H), 7.71(s, 1H), 7.55~7.41(m, 16H), 7.13 (m, 1H), 7.02(d, 1H), 6.69(m, 4H), 6.63(d, 1H) |
| 30 | 8.55(d, 1H), 8.18(d, 1H), 7.87(d, 1H), 7.71(s, 1H), 7.55~7.38(m, 12H), 7.28(t, 1H), 7.13(t, 1H), 7.02(d, 1H), 6.75~6.69(m, 3H), 6.58(d, 1H), 6.75(d, 1H), 1.72(s, 6H) |
| 36 | 8.18(d, 1H), 8.08(d, 1H), 7.87(d, 1H), 7.75(d, 1H), 7.68~7.55(m, 6H), 7.44~7.38(m, 2H), 7.28~7.20(m, 3H), 6.84~6.75(m, 2H), 6.63~6.53(m, 3H), 1.72(s, 6H) |
| 39 | 8.18(d, 1H), 8.08(d, 1H), 7.87(d, 1H), 7.75(d, 1H), 7.68(s, 1H), 7.62~7.55(m, 7H), 7.44~7.28(m, 3H), 7.28(m, 2H), 6.75(s, 2H), 6.58(d, 2H), 1.72(s, 12H) |
| 41 | 8.55(m, 1H), 8.18(d, 1H), 7.75~.71(m, 2H), 7.62~7.54(m, 5H), 7.44(t, 1H), 7.20(m, 4H), 6.81(m, 2H), 6.69~6.63(m, 6H) |
| 43 | 8.55(d, 1H), 8.18(d, 1H), 7.75~7.71(m, 2H), 7.62~7.41(m, 19H), 6.89~6.88(d, 1H), 6.69(d, 4H), 6.59(d, 1H) |
| 47 | 8.55(d, 1H), 8.18(d, 1H), 7.75~7.71(s, d, 2H), 7.62~7.41(m, 20H), 6.69(d, 6H) |
| 48 | 8.55(d, 1H), 8.18(d, 1H), 7.87(d, 1H), 7.75~7.71(d, s. 2H), 7.62~7.54(m, 7H), 7.44~7.38(m, 2H), 7.28~7.20(m, 3H), 6.81~6.58(m, 7H), 1.72(s, 6H) |
| 51 | 8.55(d, 1H), 8.18(d, 1H), 7.87(d, 1H), 7.75~7.71(d, s, 2H), 7.62~7.54(m, 7H), 7.44~7.38(m, 2H), 7.28~7.20(m, 3H), 6.81~6.63 (m, 7H), 1.72(s, 6H) |
| 57 | 8.18(d, 1H), 7.87(d, 1H), 7.75~7.71(d, s, 2H), 7.62~7.55(m, 7H), 7.44~7.38(m, 2H), 7.28~7.20(m, 2H), 6.81~6.69(m, 7H), 1.72(s, 6H) |
| 61 | 7.54~7.41(m, 19H), 6.69(d, 4H) |
| 65 | 7.87(d, 1H), 7.52(d, 1H), 7.52~7.51(m, 14H), 7.28(t, 1H), 6.75(s, 1H), 6.69(d, 2H), 6.58(d, 1H), 1.72(s, 6H) |
| 101 | No proton |
| 102 | No proton |
| 105 | No proton |
| 107 | No proton |
| 109 | No proton |
| 117 | No proton |
| 121 | 8.18(d, 1H), 7.71(s, 1H), 7.55~7.41(m, 7H), 7.25(s, 4H), 7.13(t, 1H), 7.02(d, 1H), 6.33(d, 1H) |
| 133 | 8.18(d, 1H), 8.08(d, 1H), 7.93~7.87(m, 2H), 7.77~7.75(m, 2H), 7.68(s, 1H), 7.63~7.55(m, 6H), 7.44~7.38(m, 2H), 7.28 (m, 1H), 1.72(s, 6H) |
| 141 | 8.55(d, 1H), 8.18(d, 1H), 7.79(d, 2H), 7.71(s, 1H), 7.51~7.41(m, 5H), 7.13(t, 1H), 7.02(d, 1H), 6.33(d, 1H) |
| 142 | 8.55(d, 1H), 8.18(d, 1H), 7.79(d, 2H), 7.71(s, 1H), 7.55~7.41(m, 5H), 7.13(t, 1H), 7.02(d, 1H), 6.33(d, 1H) |
| 161 | 7.54~7.41(m, 7H), 7.20(t, 2H), 6.81(t, 1H), 6.69~6.63(m, 4H) |
| 162 | 7.54~7.41(m, 14H), 6.69(t, 4H) |
| 173 | 7.87(d, 1H), 7.62(d, 1H), 7.55~7.41(m, 8H), 6.89~6.88 (m, 2H), 6.75(s, 1H), 6.89~6.59(m, 2H), 1.72(s, 6H) |
| 179 | 7.87(d, 1H), 7.62(d, 1H), 7.55~7.54(m, 3H), 7.88(t, 1H), 7.28~7.20(m, 3H), 6.75~6.53(m, 7H), 1.72(s, 6H) |
| 180 | 7.54~7.51(m, 8H), 7.20(t, 2H), 6.81(t, 1H), 6.69~6.63(m, 6H) |
| 181 | 8.55(d, 1H), 8.18(d, 1H), 7.71(s, 1H), 7.55(d, 2H), 7.13(m, 1H), 7.02(t, 1H), 6.33(d, 1H) |
| 185 | 8.55(t, 1H), 8.18(d, 1H), 7.71(s, 1H), 7.55(m, 2H), 7.13(tm 1H), 7.02(d, 1H), 6.33(d, 1H) |
| 192 | 8.18(t, 1H), 8.08(d, 1H), 7.75~7.55(m, 5H), 7.44(t, 1H) |
| 197 | 8.55(m, 1H), 8.18(d, 1H), 7.75~7.71(m, 2H), 7.62~7.55(m, 3H), 7.44 (t, 1H) |
| 202 | 7.51~7.41(m, 5H) |
| 210 | 7.51~7.41(m, 5H) |
| 213 | 7.51~7.41(m, 5H) |

**[Table 24]**

| Compound | FD-MS | Compound | FD-MS |
|---|---|---|---|
| 1 | m/z= 466.21 (C34H18D5NO= 466.58) | 105 | m/z= 616.41 (C44D29NO= 616.89) |
| 3 | m/z= 618.27 (C46H26D5NO= 618.78) | 107 | m/z= 724.49 (C52D35NO= 725.06) |
| 5 | m/z= 556.22 (C40H20D5NO2= 556.66) | 109 | m/z= 564.38 (C40D27NO= 564.81) |
| 7 | m/z= 566.24 (C42H22D5NO= 566.70) | 111 | m/z= 812.52 (C59D37NO= 813.16) |
| 9 | m/z= 542.24 (C40H22D5NO= 542.68) | 113 | m/z= 696.46 (C50D33NO= 697.01) |
| 11 | m/z= 694.30 (C52H30D5NO= 694.87) | 115 | m/z= 644.94 (C46D31NO= 644.44) |
| 13 | m/z= 582.27 (C43H26D5NO= 582.74) | 117 | m/z= 644.94 (C46D31NO= 644.44) |
| 15 | m/z= 704.29 (C53H28D5NO= 704.87) | 119 | m/z= 724.49 (C52D35NO= 725.06) |
| 17 | m/z= 542.24 (C40H22D5NO= 542.68) | 121 | m/z= 627.33 (C46H17D14NO= 627.83) |
| 19 | m/z= 692.29 (C52H28D5NO= 692.86) | 123 | m/z= 601.31 (C44H15D14NO= 601.79) |
| 21 | m/z= 672.31 (C50H24D9NO= 672.86) | 125 | m/z= 703.36 (C52H21D14NO= 703.93) |
| 23 | m/z= 698.33 (C52H26D9NO= 698.90) | 127 | m/z= 641.31 (C46H15D14NO2= 641.81) |
| 25 | m/z= 684.28 (C50H24D7NO2= 684.83) | 129 | m/z= 651.33 (C48H17D14NO= 651.85) |
| 27 | m/z= 698.33 (C52H22D11NO= 698.89) | 131 | m/z= 679.36 (C50H17D16NO= 679.90) |
| 29 | m/z= 698.33 (C52H26D9NO= 698.90) | 133 | m/z= 747.42 (C55H21D18NO= 748.02) |
| 31 | m/z= 626.32 (C46H18D13NO= 626.82) | 135 | m/z= 623.30 (C46H21D10NO= 623.81) |
| 33 | m/z= 752.37 (C56H24D13NO= 752.98) | 137 | m/z= 761.43 (C56H15D22NO= 762.03) |
| 35 | m/z= 594.27 (C44H22D7NO= 594.75) | 139 | m/z= 601.31 (C44H15D14NO= 601.79) |
| 37 | m/z= 786.36 (C59H30D9NO= 787.00) | 141 | m/z= 551.30 (C40H13D14NO= 551.73) |
| 39 | m/z= 778.39 (C58H34D9NO= 779.02) | 143 | m/z= 551.30 (C40H13D14NO= 551.73) |
| 41 | m/z= 542.24 (C40H22D5NO= 542.68) | 145 | m/z= 551.30 (C40H13D14NO= 551.73) |
| 43 | m/z= 694.30 (C52H30D5NO= 694.87) | 147 | m/z= 603.33 (C44H13D16NO= 603.81) |
| 45 | m/z= 542.24 (C40H22D5NO= 542.68) | 149 | m/z= 675.41 (C49H13D22NO= 675.94) |
| 47 | m/z= 694.30 (C52H30D5NO= 694.87) | 151 | m/z= 655.35 (C48H13D18NO= 655.88) |
| 49 | m/z= 594.27 (C44H22D7NO= 594.75) | 153 | m/z= 631.35 (C46H13D18NO= 631.86) |
| 51 | m/z= 710.33 (C53H30D7NO= 710.91) | 155 | m/z= 631.35 (C46H13D18NO= 631.86) |
| 53 | m/z= 742.38 (C55H26D13NO= 742.98) | 157 | m/z= 711.41 (C52H13D22NO= 711.98) |
| 55 | m/z= 722.33 (C54H26D9NO= 722.92) | 159 | m/z= 655.35 (C48H13D18NO= 655.88) |
| 57 | m/z= 710.33 (C53H30D7NO= 710.91) | 161 | m/z= 550.29 (C40H8D19NO= 550.73) |
| 59 | m/z= 698.33 (C52H26D9NO= 698.90) | 163 | m/z= 600.31 (C44H16D13NO= 600.79) |
| 61 | m/z= 621.29 (C46H23D8NO= 621.79) | 165 | m/z= 702.35 (C52H22D13NO= 702.92) |
| 63 | m/z= 595.28 (C44H21D8NO= 595.76) | 167 | m/z= 626.32 (C46H18D13NO=626.84) |
| 65 | m/z= 661.32 (C49H27D8NO= 661.86) | 169 | m/z= 676.34 (C50H20D13NO= 676.88) |
| 67 | m/z= 545.26 (C40H19D8NO= 545.70) | 171 | m/z= 788.37 (C59H24D13NO= 789.01) |
| 69 | m/z= 701.35 (C52H31D8NO= 701.92) | 173 | m/z= 746.41 (C55H22D17NO= 747.01) |
| 71 | m/z= 569.26 (C42H19D8NO= 569.72) | 175 | m/z= 786.43 (C58H18D21NO= 787.07) |
| 73 | m/z= 621.29 (C46H23D8NO= 621.79) | 177 | m/z= 630.35 (C46H14D17NO= 630.85) |
| 75 | m/z= 545.26 (C40H19D8NO= 545.70) | 179 | m/z= 718.38 (C53H22D15NO= 718.96) |
| 77 | m/z= 621.29 (C46H23D8NO= 621.79) | 181 | m/z= 556.33 (C40H8D19NO= 556.77) |
| 79 | m/z= 621.29 (C46H23D8NO= 621.79) | 183 | m/z= 608.36 (C44H8D21NO=608.84) |
| 81 | m/z= 519.24 (C38H17D8NO= 519.66) | 185 | m/z= 636.38 (C46H8D23NO= 636.89) |
| 83 | m/z= 697.32 (C52H27D8NO= 697.89) | 187 | m/z= 608.36 (C44H8D21NO= 608.84) |
| 85 | m/z= 671.31 (C50H25D8NO= 671.85) | 189 | m/z= 728.41 (C52H8D25NO2= 728.98) |
| 87 | m/z= 711.30 (C52H25D8NO2= 711.87) | 191 | m/z= 716.44 (C52H8D27NO= 717.01) |
| 89 | m/z= 697.32 (C52H27D8NO= 697.89) | 193 | m/z= 740.44 (C54H8D27NO= 741.03) |
| 91 | m/z= 737.35 (C55H31D8NO= 737.95) | 195 | m/z= 796.50 (C58H8D31NO= 797.13) |
| 93 | m/z= 737.35 (C55H31D8NO= 737.95) | 197 | m/z= 768.47 (C56H8D29NO= 769.08) |
| 95 | m/z= 697.32 (C52H27D8NO= 697.89) | 199 | m/z= 716.44 (C52H8D27NO= 717.01) |
| 97 | m/z= 621.29 (C46H23D8NO= 621.79) | 202 | m/z= 639.40 (C46H5D26NO= 639.92) |
| 99 | m/z= 595.28 (C44H21D8NO= 595.76) | 210 | m/z= 683.46 (C49H5D30NO= 684.01) |
| 101 | m/z= 564.38 (C40D27NO= 564.81) | 213 | m/z= 719.46 (C52H5D30NO= 720.04) |
| 103 | m/z= 644.44 (C46D31NO= 644.94) | | |

### <Experimental Example 1>

### - Manufacture of organic light emitting device

### (1) Manufacture of organic light emitting device

A glass substrate, in which ITO was thinly coated to have a thickness of 1,500 Å, was ultrasonically washed with distilled water. When the washing with distilled water was finished, the glass substrate was ultrasonically washed with a solvent such as acetone, methanol, and isopropyl alcohol, dried and then was subjected to UVO treatment for 5 minutes using UV in a UV cleaning machine. Thereafter, the substrate was transferred to a plasma washing machine (PT), and then was subjected to plasma treatment in a vacuum state for an ITO work function and in order to remove a residual film, and was transferred to a thermal deposition apparatus for organic deposition.

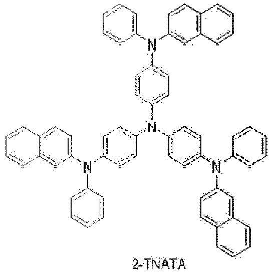

Subsequently, air in the chamber was evacuated until the degree of vacuum in the chamber reached 1.33x10⁻³ Pa(10⁻⁵ torr), and then a hole injection layer having a thickness of 600 Å was deposited on the ITO substrate by applying current to the cell to evaporate 2-TNATA. A compound described in the following Table 25 was put into another cell in the vacuum deposition device, and a hole transport layer having a thickness of 300 Å was deposited on the hole injection layer by applying current to the cell to evaporate the compound.

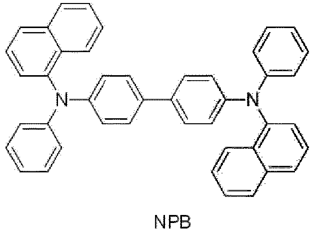

A light emitting layer was thermally vacuum deposited thereon as follows. The light emitting layer was deposited by depositing a compound of 9-[4-(4,6-diphenyl-1,3,5-triazin-2-yl)phenyl]-9'-phenyl-3,3'-Bi-9H-carbazole as a host to have a thickness of 400 Å and doping the deposited layer with a green phosphorescent dopant Ir(ppy)₃ at 7%. Thereafter, BCP as a hole blocking layer was deposited to have a thickness of 60 Å, and Alq₃ as an electron transport layer was deposited to have a thickness of 200 Å thereon. Finally, an organic light emitting device (hereinafter, referred to as Comparative Example 1) was manufactured by depositing lithium fluoride (LiF) to have a thickness of 10 Å on the electron transport layer to form an electron injection layer, and then depositing an aluminum (Al) negative electrode to have a thickness of 1,200 Å on the electron injection layer to form a negative electrode.

Meanwhile, all the organic compounds required for manufacturing an organic light emitting device were subjected to vacuum sublimed purification under 1.33×10⁻⁴ to 1.33×10⁻⁶Pa (10⁻⁶ to 10⁻⁸ torr) for each material, and used for the manufacture of the organic light emitting device.

### (2) Driving voltage and light emitting efficiency of organic light emitting device

For the organic light emitting devices of Examples 1 to 28 and Comparative Examples 1 to 5 manufactured as above, electroluminescent light emission (EL) characteristics were measured using M7000 manufactured by McScience Inc., and with the measurement results, a service life T₉₀ (unit: h, hour), which was the time when the luminance became 90% compared to the initial luminance when the standard luminance was 6,000 cd/m², was measured using a service life measurement apparatus (M6000) manufactured by McScience Inc.

The characteristics of the organic light emitting devices of the present invention shown with the measurement results are shown in the following Table 25.

**[Table 25]**

| | Compound | Driving voltage (V) | Efficiency (cd/A) | Service life (T₉₀) |
|---|---|---|---|---|
| Comparative Example 1 | Comparative Compound A | 4.65 | 115.8 | 174 |
| Comparative Example 2 | Comparative Compound B | 4.76 | 98.4 | 165 |
| Comparative Example 3 | Comparative Compound C | 4.81 | 110.7 | 180 |
| Comparative Example 4 | Comparative Compound D | 4.78 | 105.7 | 170 |
| Comparative Example 5 | Comparative Compound E | 4.83 | 107.8 | 175 |
| Example 1 | Compound 4 | 4.71 | 120.5 | 210 |
| Example 2 | Compound 13 | 4.22 | 132.8 | 208 |
| Example 3 | Compound 14 | 4.36 | 126.7 | 205 |
| Example 4 | Compound 24 | 4.59 | 131.1 | 220 |
| Example 5 | Compound 30 | 3.97 | 129.5 | 198 |
| Example 6 | Compound 36 | 4.28 | 141.6 | 186 |
| Example 7 | Compound 43 | 4.32 | 149.2 | 215 |
| Example 8 | Compound 47 | 3.85 | 145.4 | 209 |
| Example 9 | Compound 48 | 4.26 | 138.3 | 216 |
| Example 10 | Compound 51 | 4.14 | 129.7 | 221 |
| Example 11 | Compound 65 | 4.87 | 131.8 | 250 |
| Example 12 | Compound 101 | 4.33 | 150.3 | 276 |
| Example 13 | Compound 102 | 4.62 | 146.2 | 269 |
| Example 14 | Compound 105 | 3.86 | 142.5 | 271 |
| Example 15 | Compound 107 | 4.28 | 138.4 | 259 |
| Example 16 | Compound 109 | 4.34 | 127.5 | 278 |
| Example 17 | Compound 117 | 4.07 | 147.6 | 270 |
| Example 18 | Compound 121 | 4.39 | 149.1 | 255 |
| Example 19 | Compound 133 | 4.56 | 128.2 | 245 |
| Example 20 | Compound 141 | 4.73 | 127.3 | 239 |
| Example 21 | Compound 142 | 4.05 | 131.8 | 238 |
| Example 22 | Compound 162 | 4.12 | 126.9 | 240 |
| Example 23 | Compound 179 | 3.95 | 138.7 | 246 |
| Example 24 | Compound 181 | 3.84 | 141.4 | 222 |
| Example 25 | Compound 185 | 4.47 | 126.3 | 223 |
| Example 26 | Compound 197 | 4.65 | 137.2 | 249 |
| Example 27 | Compound 210 | 4.35 | 130.4 | 235 |
| Example 28 | Compound 213 | 4.11 | 139.4 | 243 |

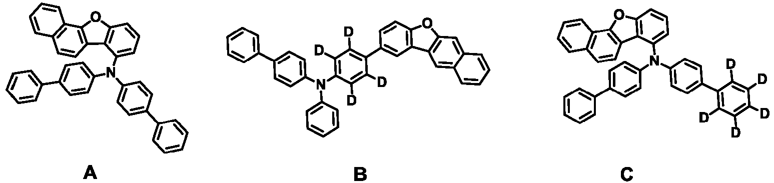

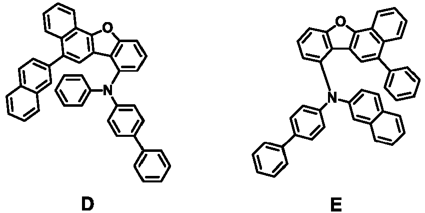

### <Experimental Example 2>

### (1) Manufacture of organic light emitting device

A glass substrate thinly coated with indium tin oxide (ITO) having a thickness of 1,500 Å was ultrasonically washed with distilled water. When the washing with distilled water was finished, the glass substrate was ultrasonically washed with a solvent such as acetone, methanol, and isopropyl alcohol, dried and then was subjected to UVO treatment for 5 minutes using UV in a UV cleaning machine. Thereafter, the substrate was transferred to a plasma washing machine (PT), and then was subjected to plasma treatment in a vacuum state in order to increase an ITO work function and remove a residual film, and was transferred to a thermal deposition apparatus for organic deposition.

Subsequently, air in the chamber was evacuated until the degree of vacuum in the chamber reached 1.33x10⁻⁴ Pa (10⁻⁶ torr), and then a hole injection layer having a thickness of 600 Å was deposited on the ITO substrate by applying current to the cell to evaporate 2-TNATA. A hole transport layer having a thickness of 300 Å was deposited on the hole injection layer by placing the following N,N'-bis(α-naphthyl)-N,N'-diphenyl-4,4'-diamine (NPB) in another cell in the vacuum deposition apparatus and applying current to the cell to evaporate NPB. Thereafter, the compound (one of Examples 29 to 56) or the comparative compound (one of Comparative Examples 6 to 10) represented by Chemical Formula 1 shown in the following Table 26 was deposited as a light emitting auxiliary layer to a thickness of 100 Å.

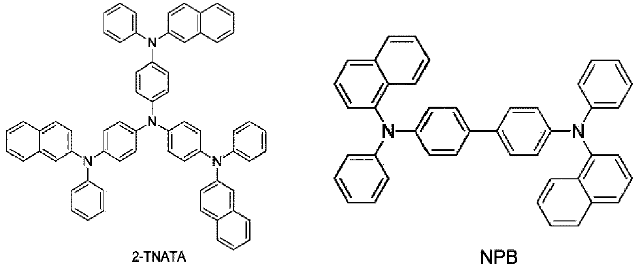

A light emitting layer was thermally vacuum deposited thereon as follows. The light emitting layer was deposited by depositing a compound of 9-[4-(4,6-diphenyl-1,3,5-triazin-2-yl)phenyl]-9'-phenyl-3,3'-Bi-9H-carbazole as a host to have a thickness of 400 Å and doping the deposited layer with [Ir(ppy)₃] as a green phosphorescent dopant by 7% of the deposited thickness of the light emitting layer. Thereafter, bathocuproine (BCP) was deposited as a hole blocking layer to have a thickness of 60 Å, and Alq₃ was deposited as an electron transport layer to have a thickness of 200 Å thereon. Finally, an organic light emitting device was manufactured by depositing lithium fluoride (LiF) to have a thickness of 10 Å on the electron transport layer to form an electron injection layer, and then depositing an aluminum (Al) negative electrode to have a thickness of 1,200 Å on the electron injection layer to form a negative electrode.

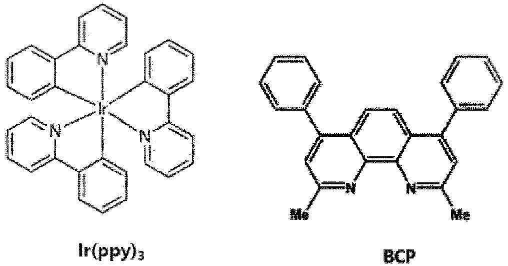

Meanwhile, all the organic compounds required for manufacturing an organic light emitting device were subjected to vacuum sublimed purification under 1.33×10⁻⁴ to 1.33×10⁻⁶ Pa (10⁻⁴ to 10⁻⁶ torr) for each material, and used for the manufacture of the organic light emitting device.

### (2) Driving voltage and light emitting efficiency of

### organic light emitting device

For the organic light emitting devices of Examples 29 to 56 and Comparative Examples 6 to 10 manufactured as above, electroluminescent light emission (EL) characteristics were measured using M7000 manufactured by McScience Inc., and with the measurement results, a service life T₉₀ (unit: h, hour), which was the time when the luminance became 90% compared to the initial luminance when the standard luminance was 6,000 cd/m², was measured using a service life measurement apparatus (M6000) manufactured by McScience Inc.

The characteristics of the organic light emitting devices of the present invention shown with the measurement results are shown in the following Table 26.

**[Table 26]**

| | Compound | Driving voltage (V) | Efficiency (cd/A) | Service life (T₉₀) |
|---|---|---|---|---|
| Comparative Example 6 | Comparative Compound A | 4.64 | 108.9 | 123 |
| Comparative Example 7 | Comparative Compound B | 5.68 | 95.6 | 138 |
| Comparative Example 8 | Comparative Compound C | 4.92 | 102.5 | 145 |
| Comparative Example 9 | Comparative Compound D | 4.82 | 101.0 | 140 |
| Comparative Example 10 | Comparative Compound E | 5.13 | 97.8 | 139 |
| Example 29 | Compound 4 | 3.52 | 130.9 | 215 |
| Example 30 | Compound 13 | 4.13 | 128.7 | 262 |
| Example 31 | Compound 14 | 3.61 | 119.5 | 223 |
| Example 32 | Compound 24 | 3.81 | 131.6 | 196 |
| Example 33 | Compound 30 | 4.50 | 140.1 | 257 |
| Example 34 | Compound 36 | 4.33 | 138.5 | 249 |
| Example 35 | Compound 43 | 3.51 | 119.4 | 235 |
| Example 36 | Compound 47 | 3.72 | 127.6 | 223 |
| Example 37 | Compound 48 | 4.03 | 136.9 | 238 |
| Example 38 | Compound 51 | 3.83 | 130.8 | 213 |
| Example 39 | Compound 65 | 3.94 | 134.5 | 224 |
| Example 40 | Compound 101 | 3.68 | 128.4 | 233 |
| Example 41 | Compound 102 | 4.14 | 135.8 | 245 |
| Example 42 | Compound 105 | 3.94 | 128.4 | 241 |
| Example 43 | Compound 107 | 4.24 | 127.8 | 215 |
| Example 44 | Compound 109 | 3.98 | 133.6 | 240 |
| Example 45 | Compound 117 | 3.84 | 133.6 | 236 |
| Example 46 | Compound 121 | 3.68 | 130.6 | 228 |
| Example 47 | Compound 133 | 3.59 | 142.5 | 223 |
| Example 48 | Compound 141 | 3.96 | 135.5 | 239 |
| Example 49 | Compound 142 | 3.71 | 138.4 | 242 |
| Example 50 | Compound 162 | 3.62 | 144.3 | 240 |
| Example 51 | Compound 179 | 4.24 | 138.9 | 235 |
| Example 52 | Compound 181 | 4.05 | 131.8 | 221 |
| Example 53 | Compound 185 | 3.81 | 135.5 | 240 |
| Example 54 | Compound 197 | 3.99 | 131.2 | 238 |
| Example 55 | Compound 210 | 4.03 | 134.6 | 216 |
| Example 56 | Compound 213 | 3.89 | 138.9 | 229 |

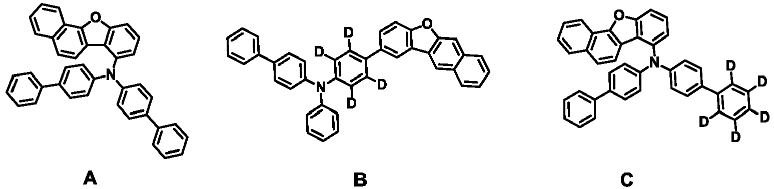

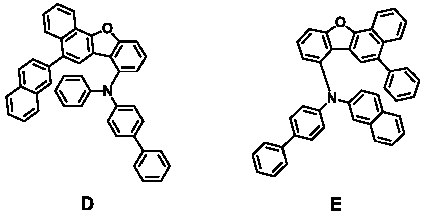

When the heterocyclic compound represented by Chemical Formula 1 is used for an organic light emitting device, the driving voltage of the device could be lowered, the light efficiency of the device could be improved, and the service life characteristics of the device could be improved due to the thermal stability of the compound.

The deuterium content of the heterocyclic compound of the present invention satisfies 1% to 100%, and since the single bond dissociation energy of carbon and deuterium is higher than the single bond dissociation energy of carbon and hydrogen, the stability of the total molecules is enhanced as the deuterium content of the heterocyclic compound of Chemical Formula 1 according to the present application satisfies the above range, so that it could be confirmed that there was an effect that the service life of the device was improved.

## Claims

1. A heterocyclic compound represented by the following Chemical Formula 1: 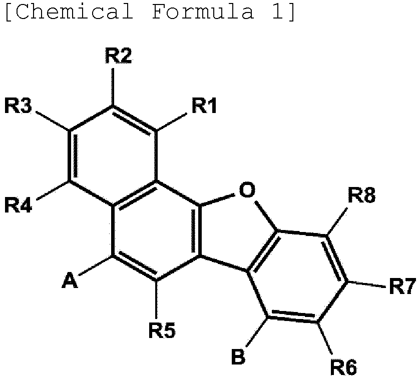 in Chemical Formula 1,
R1 to R8 are the same as or different from each other, and are each independently selected from the group consisting of hydrogen; deuterium; a halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; and -SiRR'R", or two or more adjacent groups are bonded to each other to form a substituted or unsubstituted C6 to C60 aliphatic or aromatic hydrocarbon ring, or a substituted or unsubstituted C2 to C60 aliphatic or aromatic hetero ring,
A and B are each independently represented by the following Chemical Formula 1-1; or the following Chemical Formula 1-2, 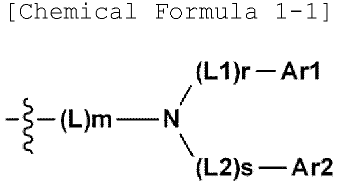 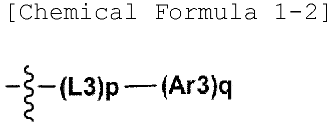
in Chemical Formulae 1-1 and 1-2,
L and L1 to L3 are the same as or different from each other, and are each independently a direct bond; a substituted or unsubstituted C6 to C60 arylene group; or a substituted or unsubstituted C2 to C60 heteroarylene group,
Ar1 to Ar3 are the same as or different from each other, and are each independently a substituted or unsubstituted C1 to C60 alkyl group; a substituted or unsubstituted C6 to C60 aryl group; a substituted or unsubstituted C2 to C60 heteroaryl group; -P(=O)RR'; or - SiRR'R",
p, m, r and s are each an integer from 0 to 4, q is an integer from 1 to 6,
when p, m, r, s and q are each 2 or higher, substituents in the parenthesis are the same as or different from each other,
a deuterium content of the heterocyclic compound of Chemical Formula 1 is 1% to 100%, and
R, R' and R" are the same as or different from each other, and are each independently a substituted or unsubstituted C1 to C60 alkyl group; a substituted or unsubstituted C6 to C60 aryl group; or a substituted or unsubstituted C2 to C60 heteroaryl group.

2. The heterocyclic compound of claim 1, wherein Chemical Formula 1 is represented by the following Chemical Formula 2 or the following Chemical Formula 3: 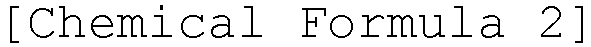 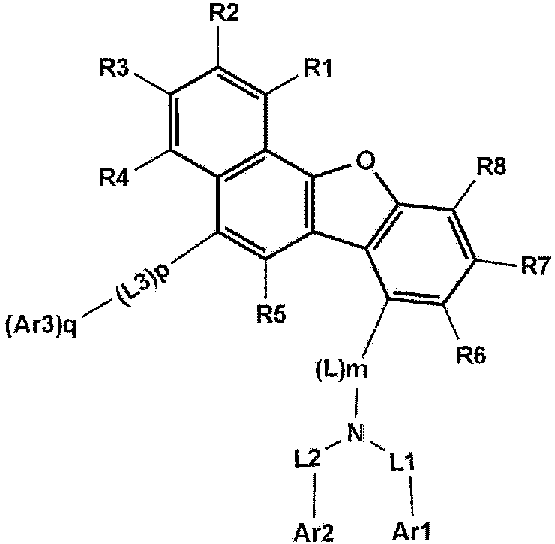 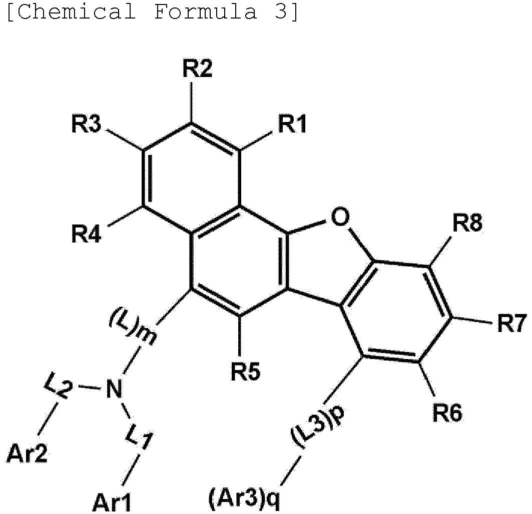 in Chemical Formulae 2 and 3,
the definition of each substituent is the same as the definition in Chemical Formula 1.

3. The heterocyclic compound of claim 1, wherein the deuterium content of the heterocyclic compound of Chemical Formula 1 is 10% to 80%.

4. The heterocyclic compound of claim 1, wherein Chemical Formula 1 is expressed while being divided into the units of the following Structural Formulae A to C, and
the deuterium content of the following Structural Formula A; the following Structural Formula B; the following Structural Formula C; the following Structural Formula A and the following Structural Formula B; the following Structural Formula A and the following Structural Formula C; the following Structural Formula B and the following Structural Formula C; or the following Structural Formulae A to C in Chemical Formula 1 is 1% to 100%: 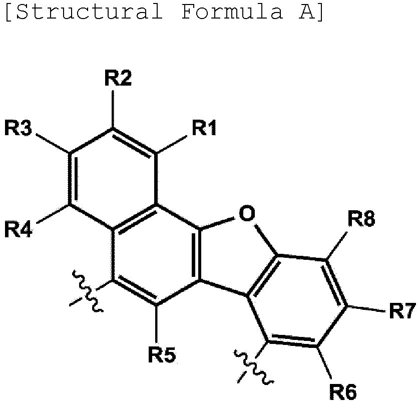 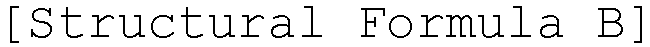 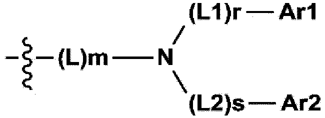 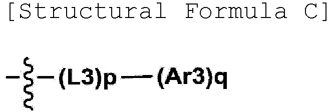
in Structural Formulae A to C, the definition of each substituent is the same as the definition in Chemical Formula 1, and 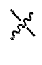 means a position at which Structural Formulae A to C are linked to each other.

5. The heterocyclic compound of claim 1, wherein Chemical Formula 1 is represented by any one of the following compounds:

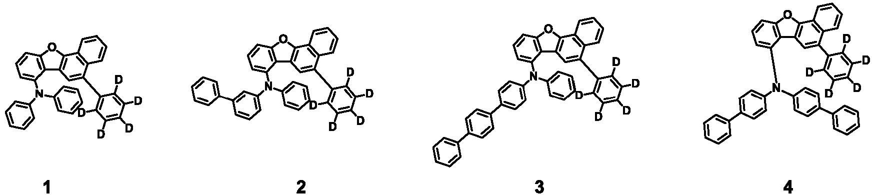

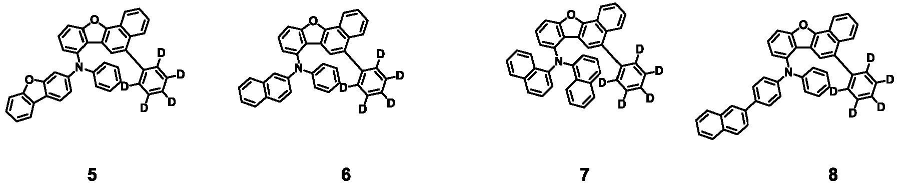

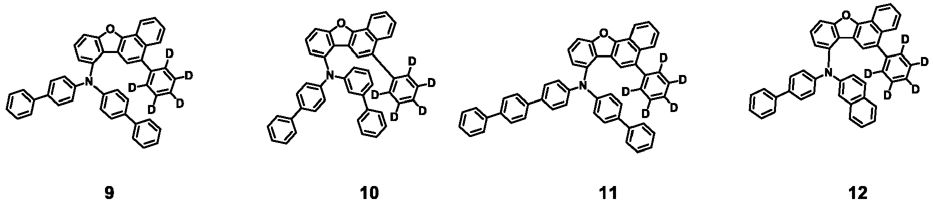

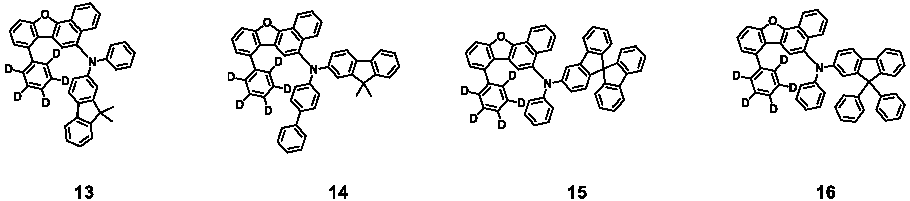

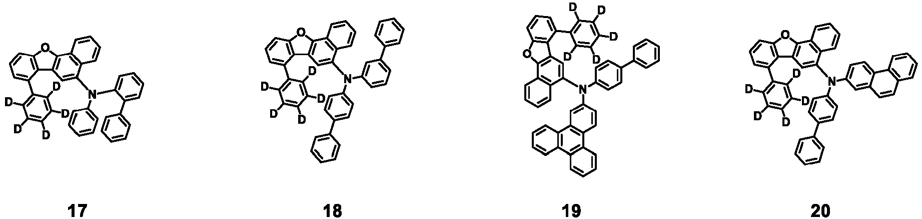

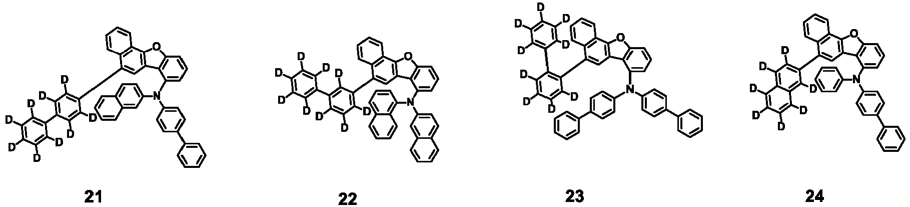

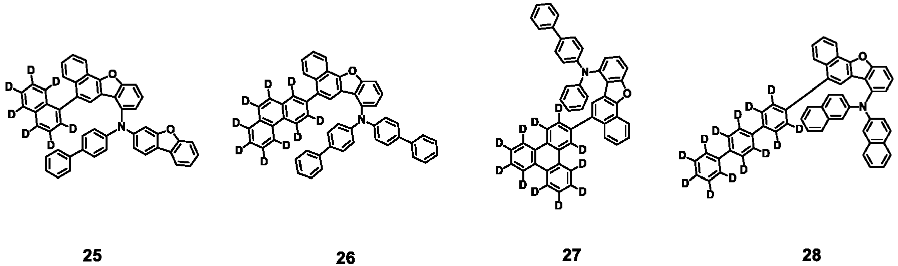

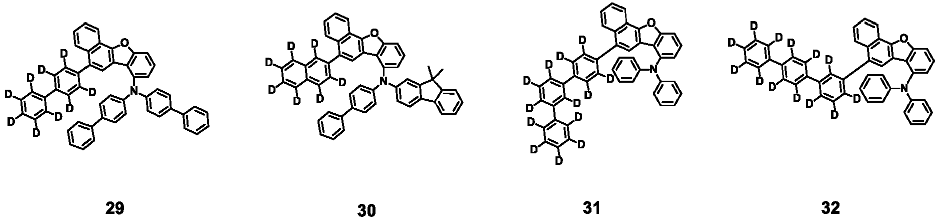

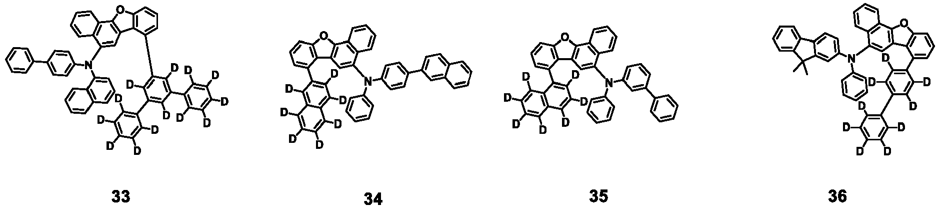

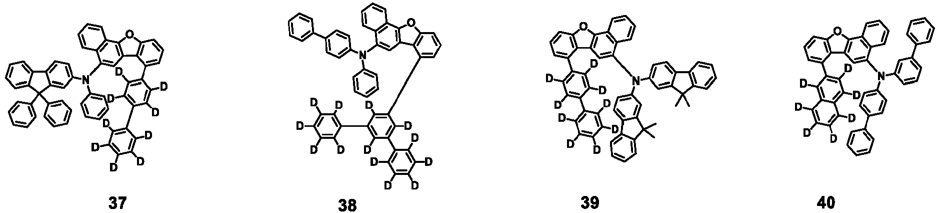

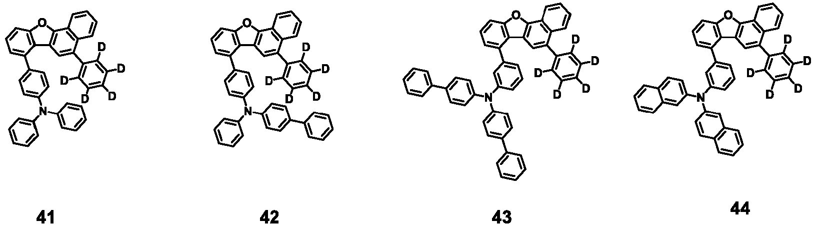

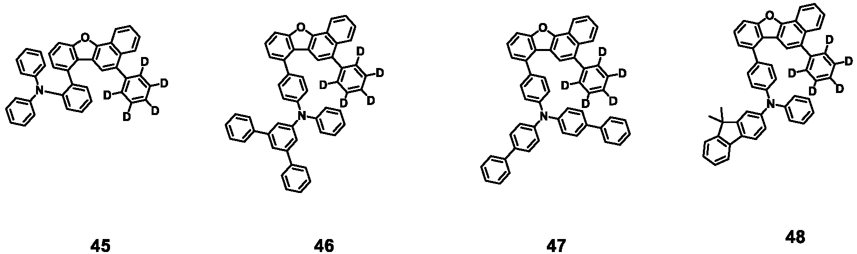

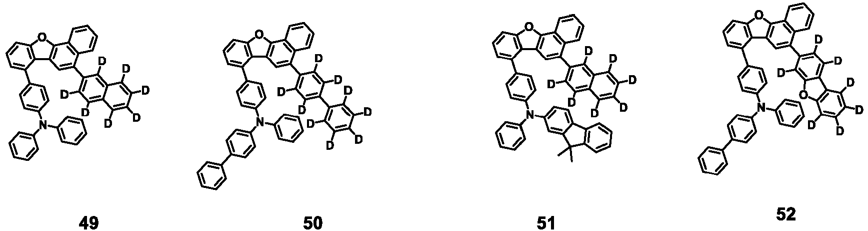

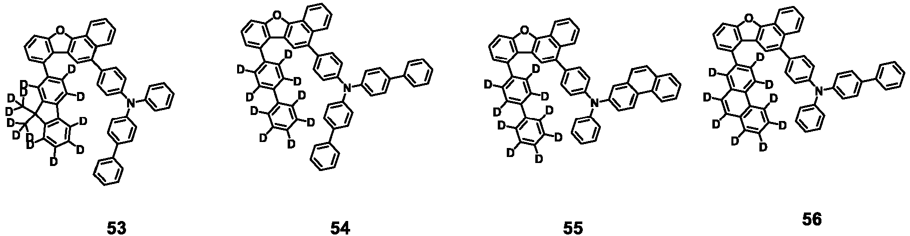

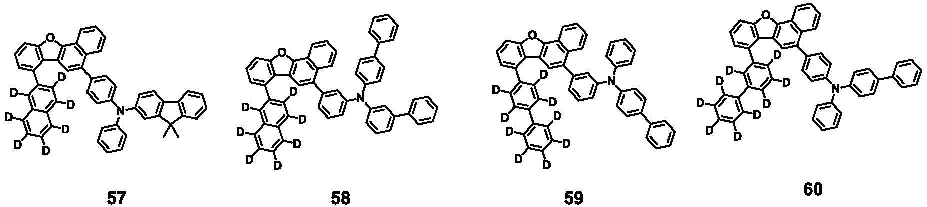

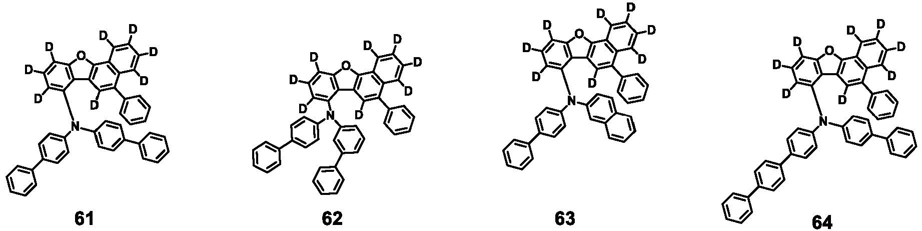

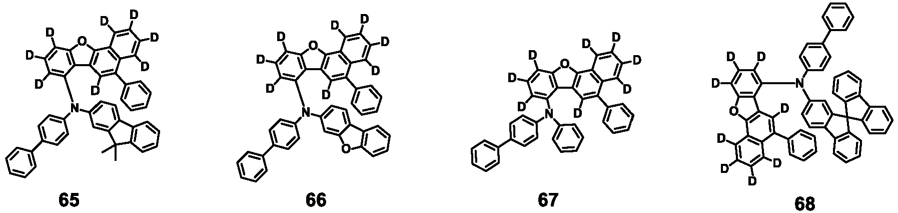

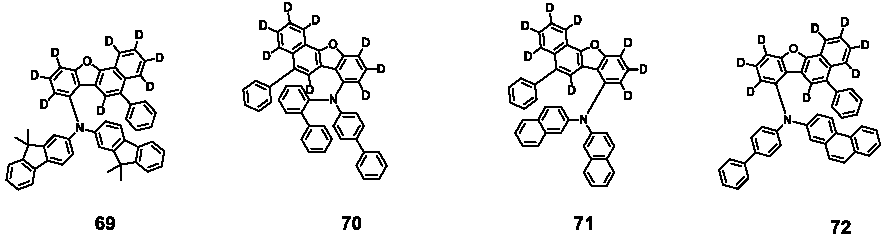

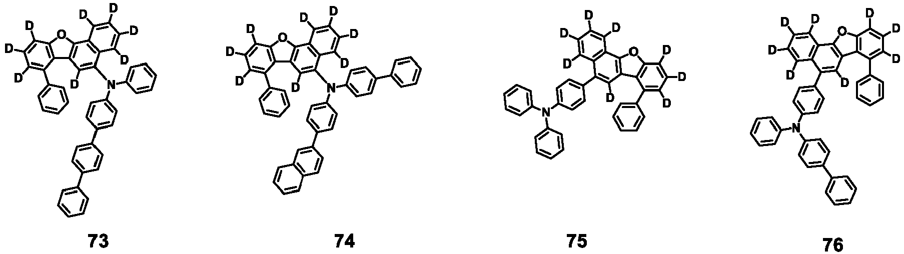

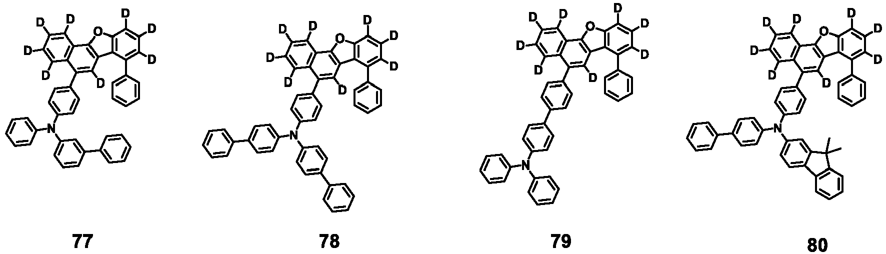

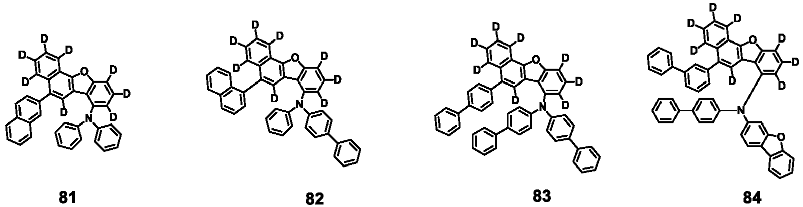

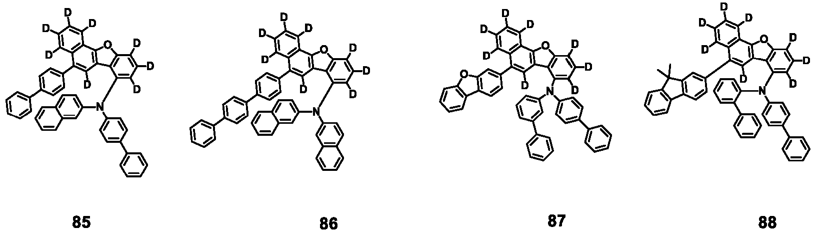

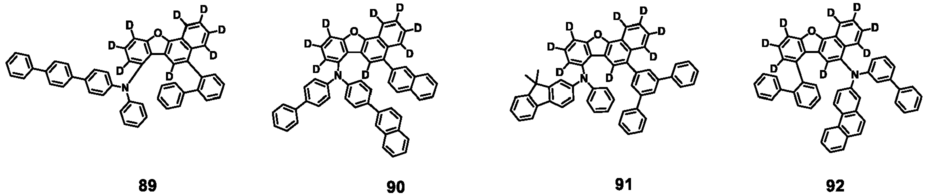

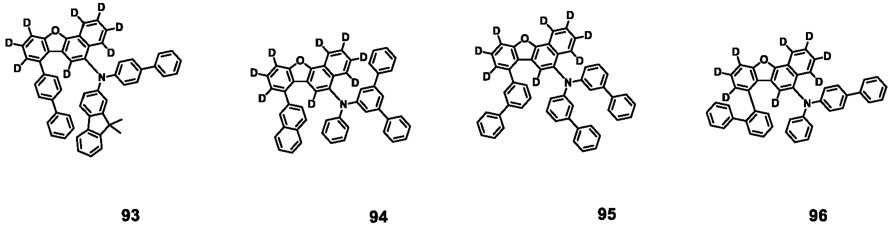

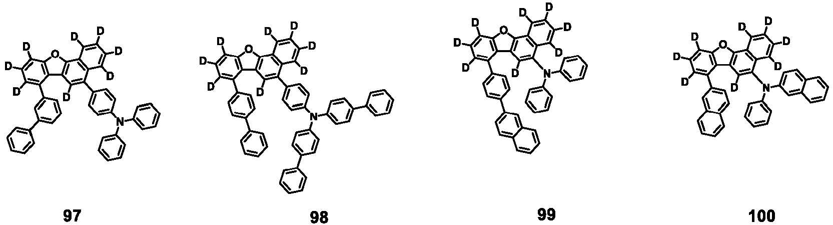

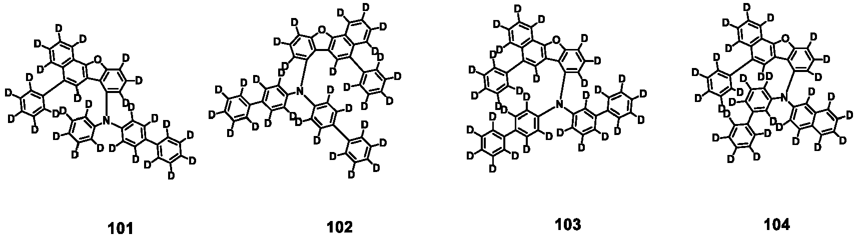

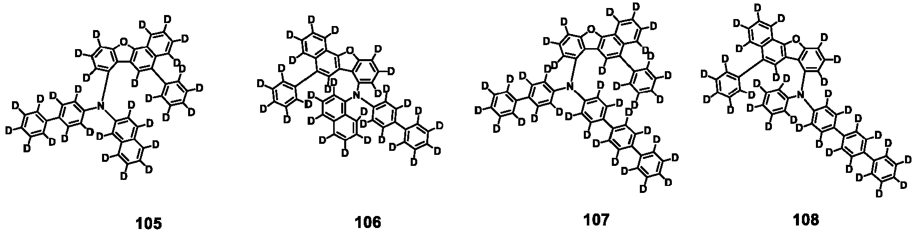

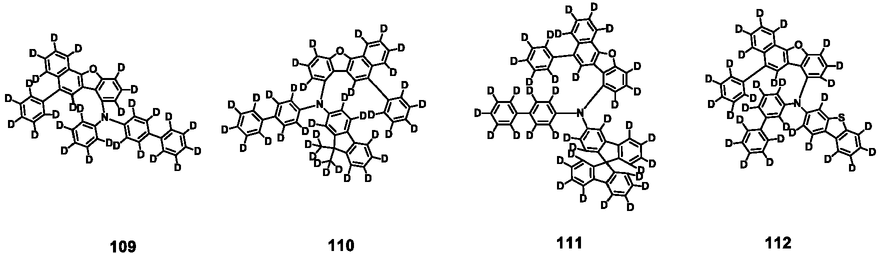

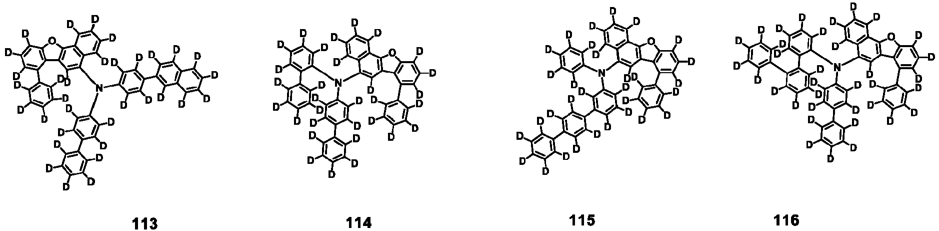

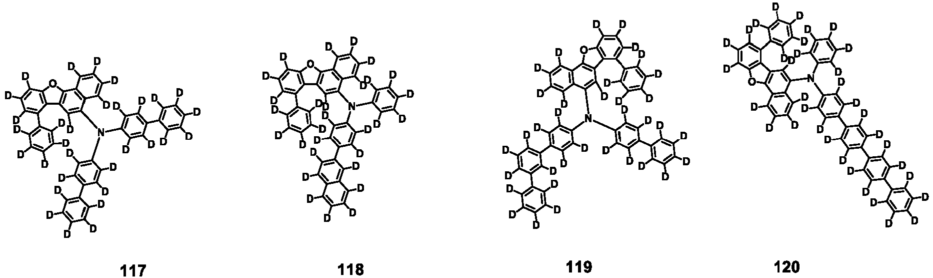

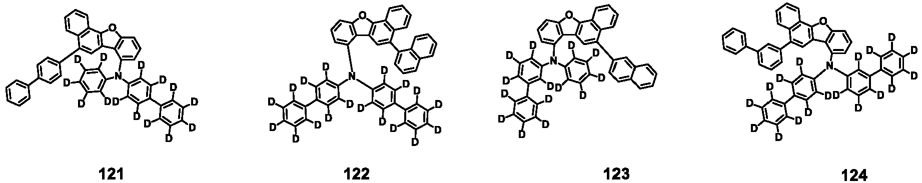

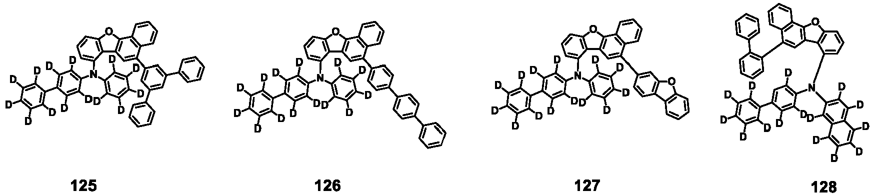

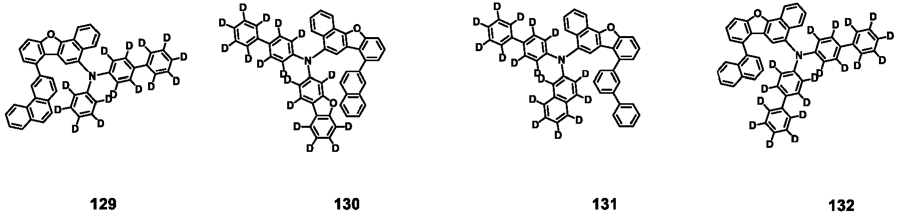

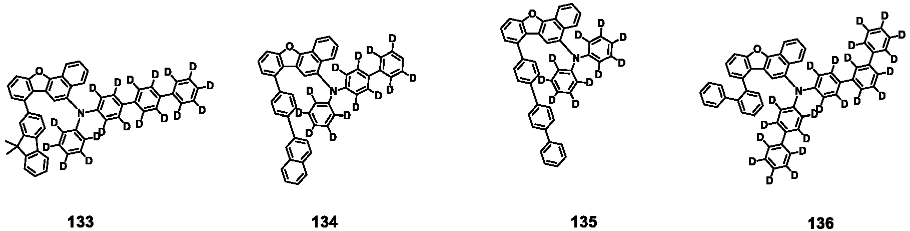

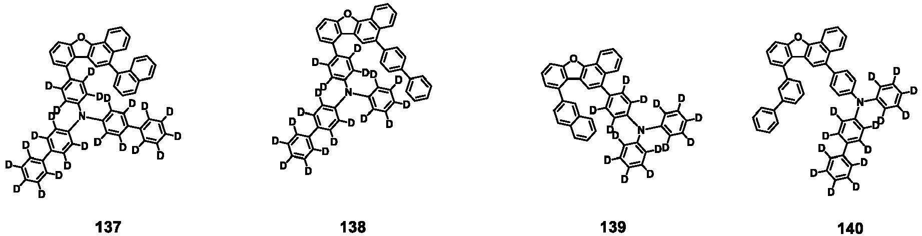

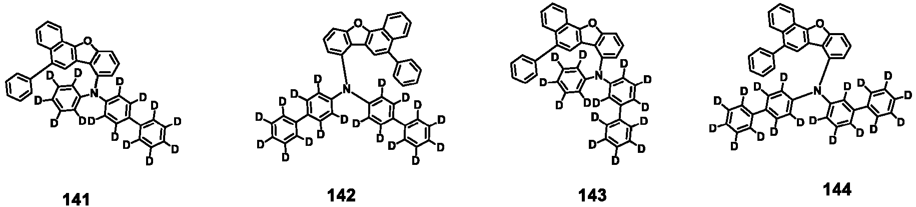

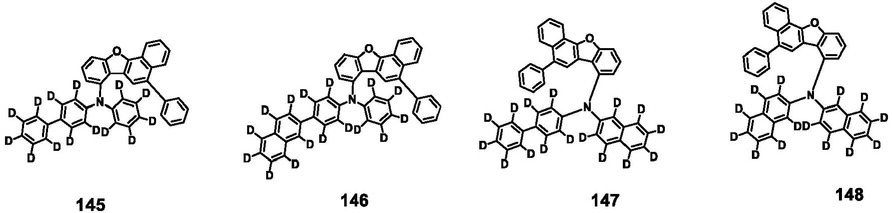

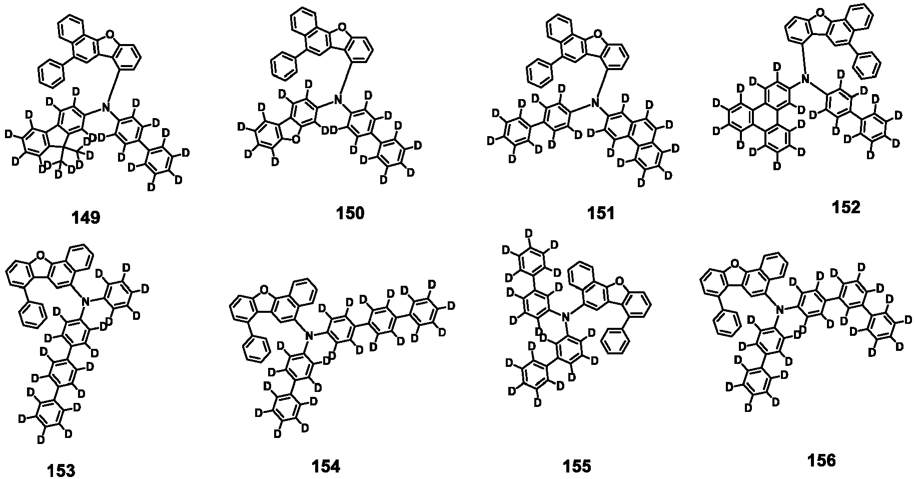

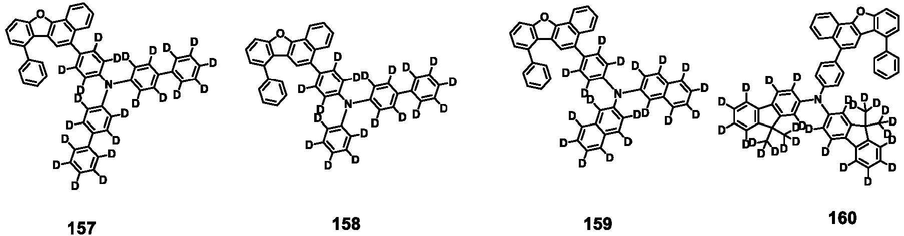

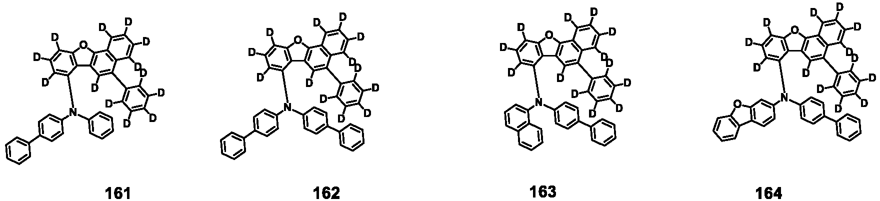

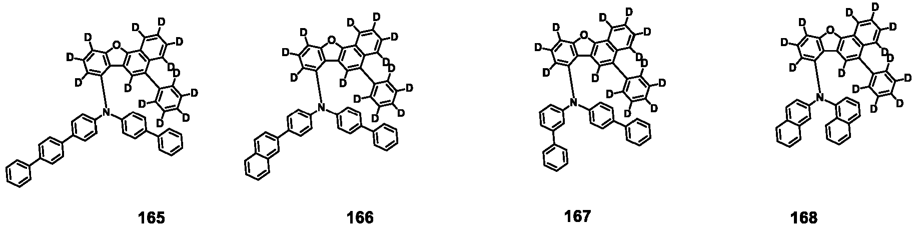

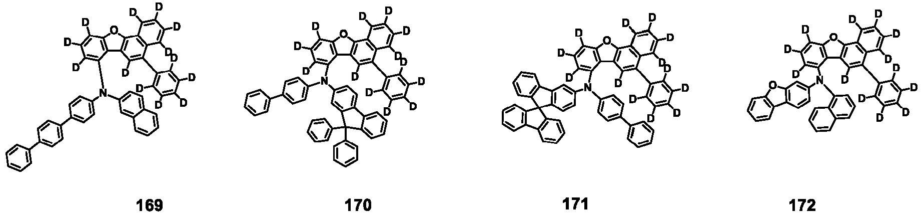

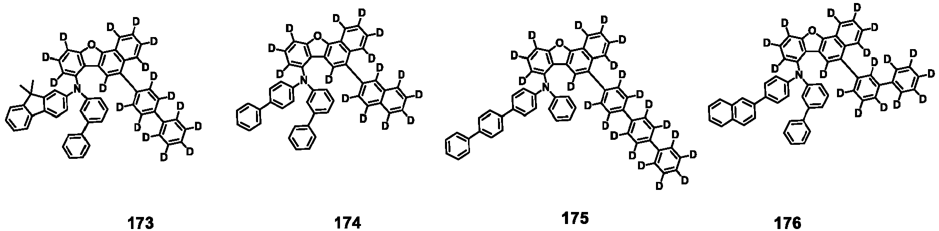

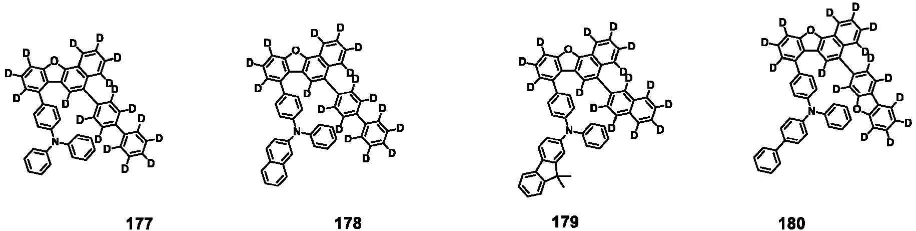

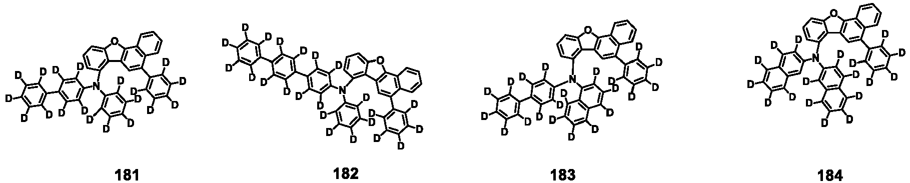

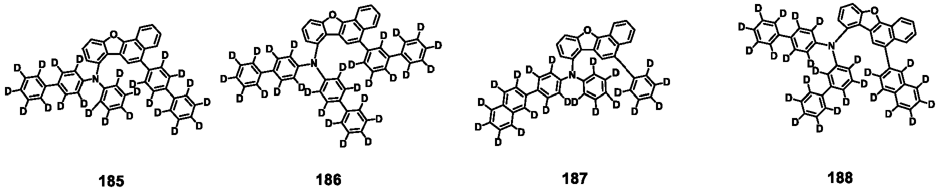

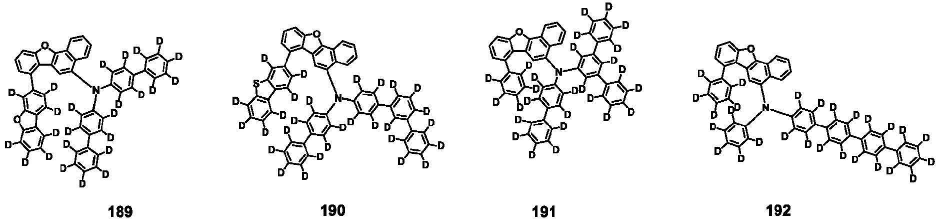

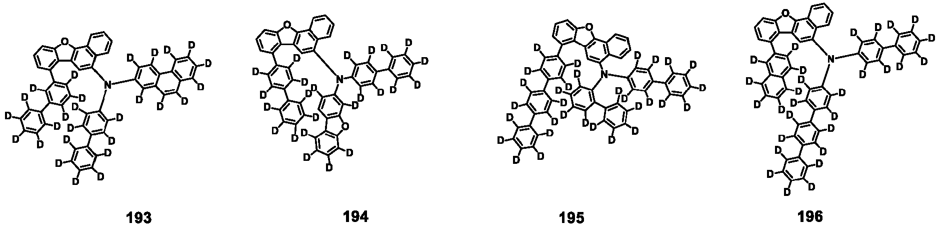

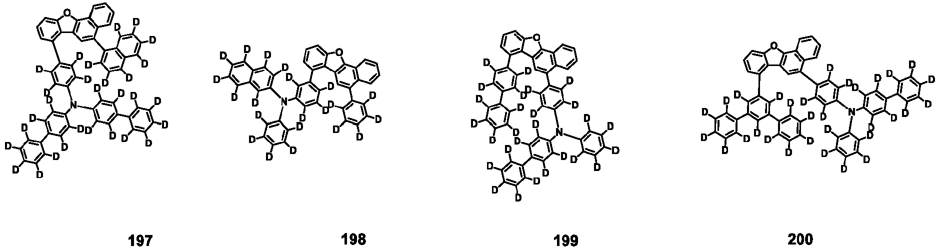

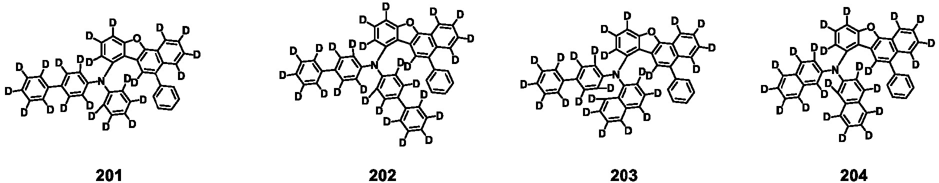

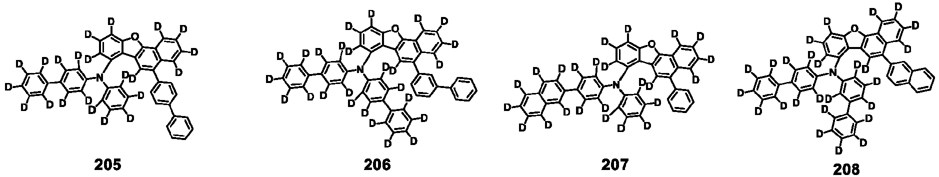

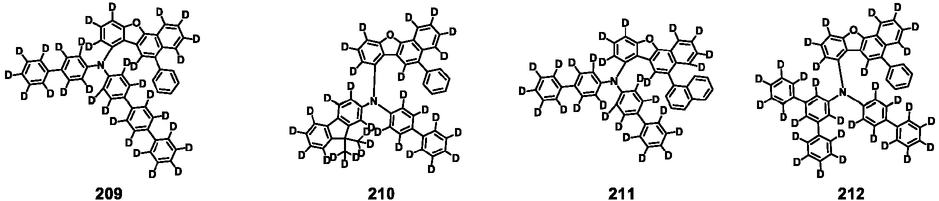

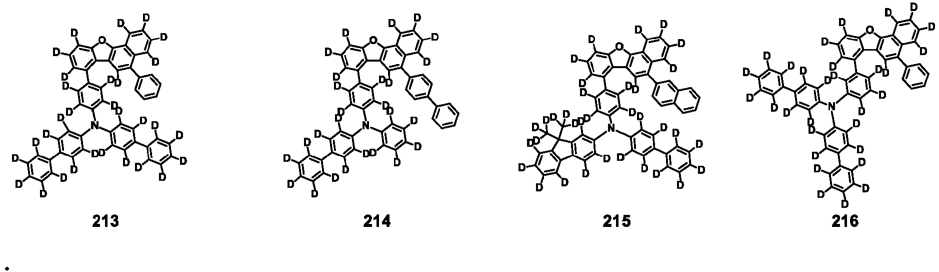

6. An organic light emitting device comprising:
a first electrode;
a second electrode provided to face the first electrode; and
an organic material layer having one or more layers provided between the first electrode and the second electrode,
wherein one or more layers of the organic material layer comprise the heterocyclic compound according to any one of claims 1 to 5.

7. The organic light emitting device of claim 6, wherein the organic material layer comprises a light emitting auxiliary layer, and the light emitting auxiliary layer comprises the heterocyclic compound.

8. The organic light emitting device of claim 6, wherein the organic material layer comprises an electron injection layer or an electron transport layer, and the electron transport layer or the electron injection layer comprises the heterocyclic compound.

9. The organic light emitting device of claim 6, wherein the organic material layer comprises an electron blocking layer or a hole blocking layer, and the electron blocking layer or the hole blocking layer comprises the heterocyclic compound.

10. The organic light emitting device of claim 6, wherein the organic material layer comprises a hole transport layer, and the hole transport layer comprises the heterocyclic compound.

11. The organic light emitting device of claim 6, further comprising one or more layers selected from the group consisting of a light emitting layer, a hole injection layer, a hole transport layer, an electron injection layer, an electron transport layer, an electron blocking layer and a hole blocking layer.

## Patentansprüche

1. Heterocyclische Verbindung, dargestellt durch die folgende chemische Formel 1: 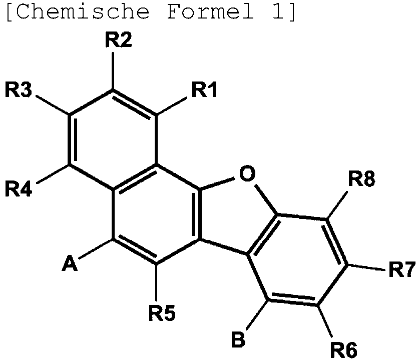 wobei in der chemischen Formel 1
R1 bis R8 gleich oder verschieden voneinander sind und jeweils unabhängig ausgewählt sind aus der Gruppe bestehend aus Wasserstoff; Deuterium; einem Halogen; einer Cyanogruppe; einer substituierten oder unsubstituierten C1 - bis C60 - Alkylgruppe; einer substituierten oder unsubstituierten C2 - bis C60 -Alkenylgruppe; einer substituierten oder unsubstituierten C2 - bis C60 -Alkinylgruppe; einer substituierten oder unsubstituierten C1 - bis C60 - Alkoxygruppe; einer substituierten oder unsubstituierten C3 - bis C60 -Cycloalkylgruppe; einer substituierten oder unsubstituierten C2 - bis C60 -Heterocycloalkylgruppe; einer substituierten oder unsubstituierten C6 - bis C60 -Arylgruppe; einer substituierten oder unsubstituierten C2 - bis C60 - Heteroarylgruppe; -P(=O)RR'; und -SiRR'R", oder zwei oder mehr benachbarte Gruppen aneinander gebunden sind, um einen substituierten oder unsubstituierten aliphatischen oder aromatischen C6 - bis C60 -Kohlenwasserstoffring oder einen substituierten oder unsubstituierten aliphatischen oder aromatischen C2 - bis C60 -Heteroring zu bilden,
A und B jeweils unabhängig dargestellt sind durch die folgende chemische Formel 1-1; oder die folgende chemische Formel 1-2, 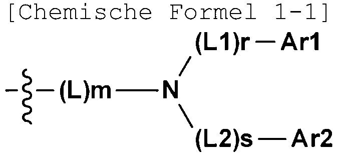 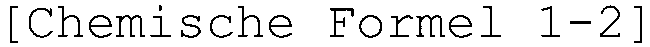 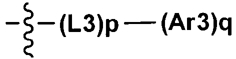
wobei in den chemischen Formeln 1-1 und 1-2
L und L1 bis L3 gleich oder verschieden voneinander sind und jeweils unabhängig eine direkte Bindung; eine substituierte oder unsubstituierte C6 - bis C60 -Arylengruppe; oder eine substituierte oder unsubstituierte C2 - bis C60 - Heteroarylengruppe sind,
Ar1 bis Ar3 gleich oder verschieden voneinander sind und jeweils unabhängig eine substituierte oder unsubstituierte C1 - bis C60 -Alkylgruppe; eine substituierte oder unsubstituierte C6 - bis C60 -Arylgruppe; eine substituierte oder unsubstituierte C2 - bis C60 -Heteroarylgruppe; - P(=O)RR'; oder -SiRR'R" sind,
p, m, r und s jeweils eine ganze Zahl von 0 bis 4 sind, q eine ganze Zahl von 1 bis 6 ist,
wenn p, m, r, s und q jeweils 2 oder höher sind, Substituenten in den Klammern gleich oder verschieden voneinander sind,
ein Deuteriumgehalt der heterocyclischen Verbindung der chemischen Formel 1 1 % bis 100 % beträgt, und
R, R' und R" gleich oder verschieden voneinander sind und jeweils unabhängig eine substituierte oder unsubstituierte C1 - bis C60 -Alkylgruppe; eine substituierte oder unsubstituierte C6 - bis C60 -Arylgruppe; oder eine substituierte oder unsubstituierte C2 - bis C60 - Heteroarylgruppe sind.

2. Heterocyclische Verbindung nach Anspruch 1, wobei die chemische Formel 1 durch die folgende chemische Formel 2 oder die folgende chemische Formel 3 dargestellt ist: 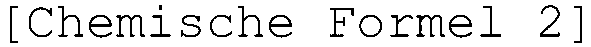 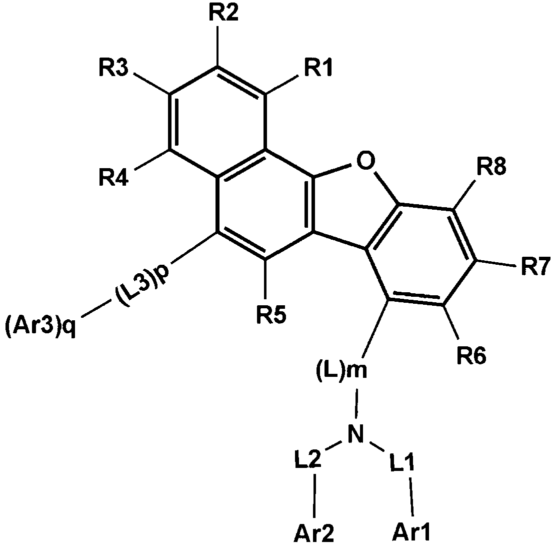 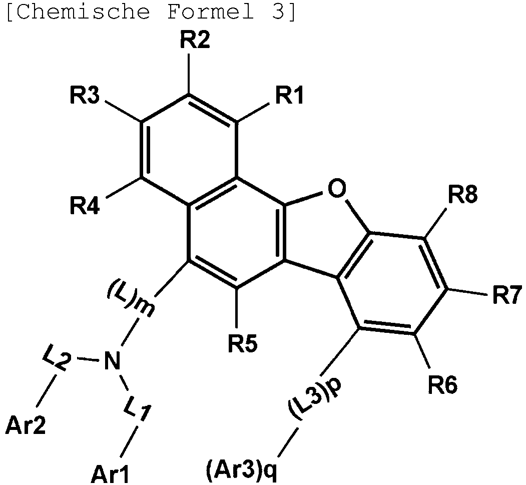
wobei in den chemischen Formeln 2 und 3
die Definition jedes Substituenten die gleiche ist wie die Definition in der chemischen Formel 1.

3. Heterocyclische Verbindung nach Anspruch 1, wobei der Deuteriumgehalt der heterocyclischen Verbindung der chemischen Formel 1 10 % bis 80 % beträgt.

4. Heterocyclische Verbindung nach Anspruch 1, wobei die chemische Formel 1 ausgedrückt wird, während sie in die Einheiten der folgenden Strukturformeln A bis C unterteilt ist, und
der Deuteriumgehalt der folgenden Strukturformel A; der folgenden Strukturformel B; der folgenden Strukturformel C; der folgenden Strukturformel A und der folgenden Strukturformel B; der folgenden Strukturformel A und der folgenden Strukturformel C; der folgenden Strukturformel B und der folgenden Strukturformel C; oder der folgenden Strukturformeln A bis C in der chemischen Formel 1 1 % bis 100 % beträgt: 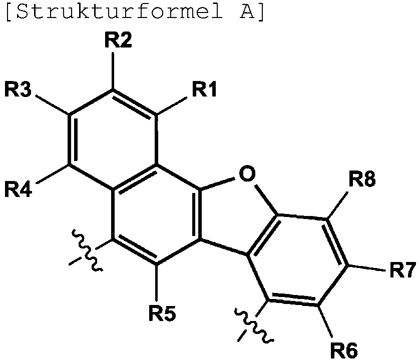 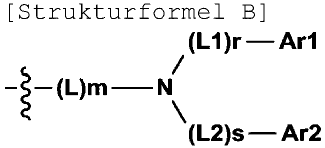 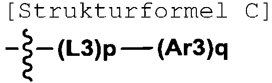
wobei in den Strukturformeln A bis C die Definition jedes Substituenten die gleiche ist wie die Definition in der chemischen Formel 1, und 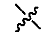 eine Position bedeutet, an der die Strukturformeln A bis C miteinander verknüpft sind.

5. Heterocyclische Verbindung nach Anspruch 1, wobei die chemische Formel 1 durch eine der folgenden Verbindungen dargestellt ist:

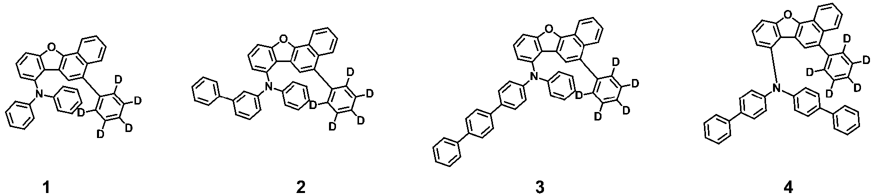

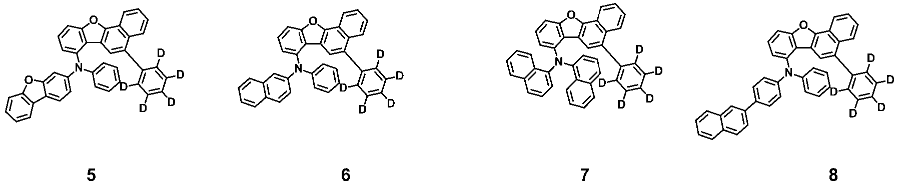

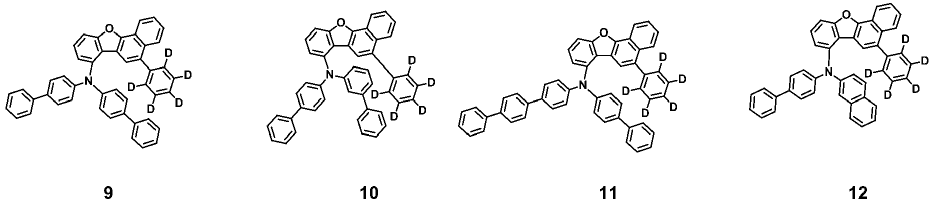

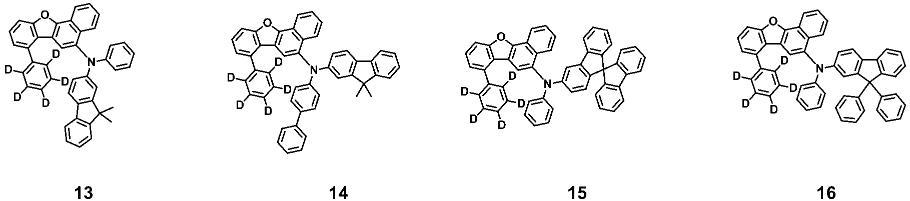

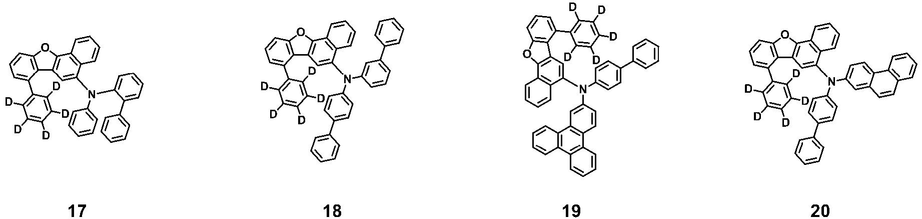

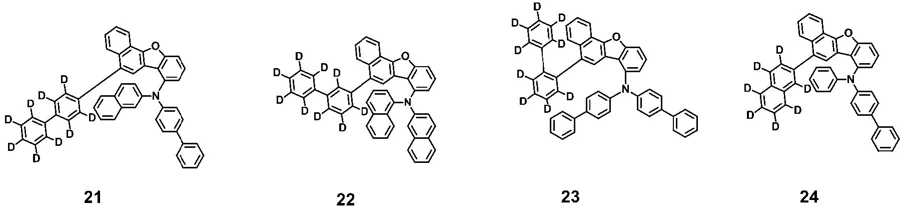

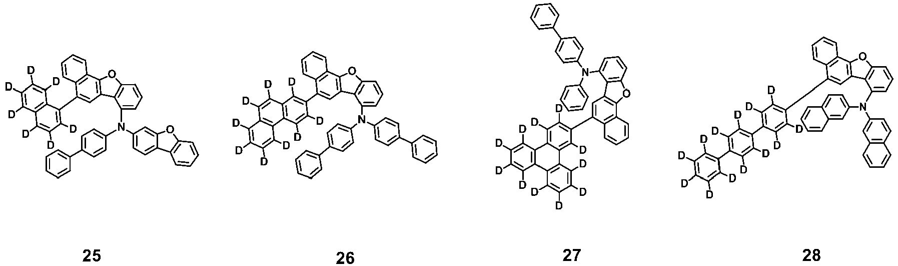

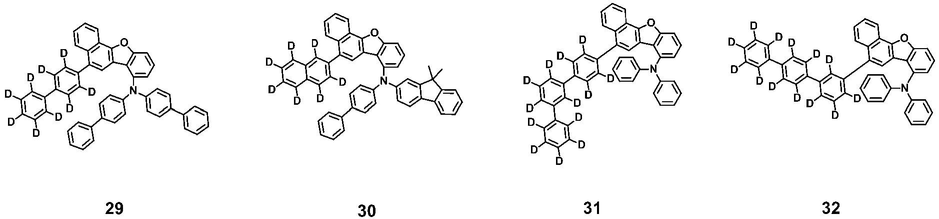

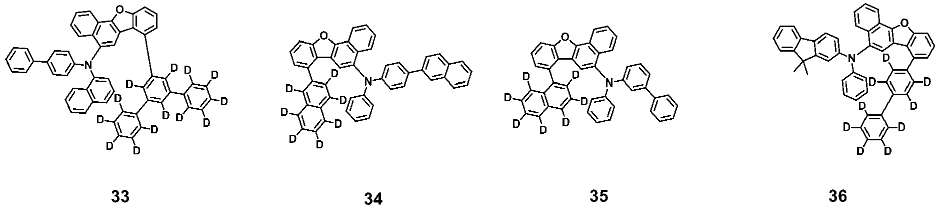

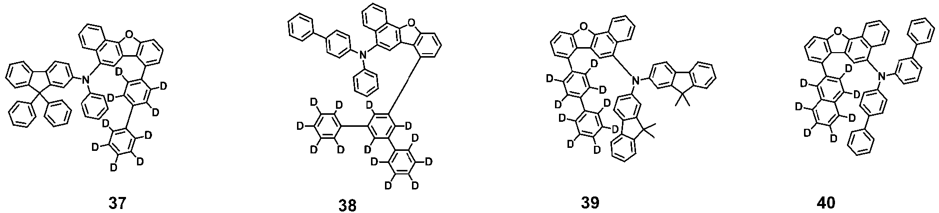

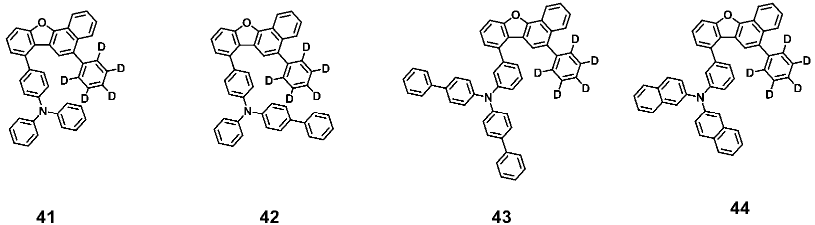

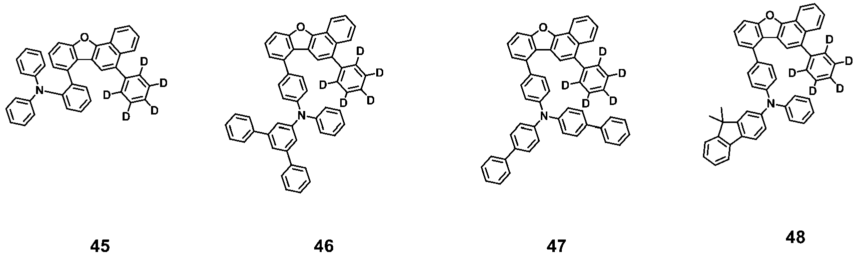

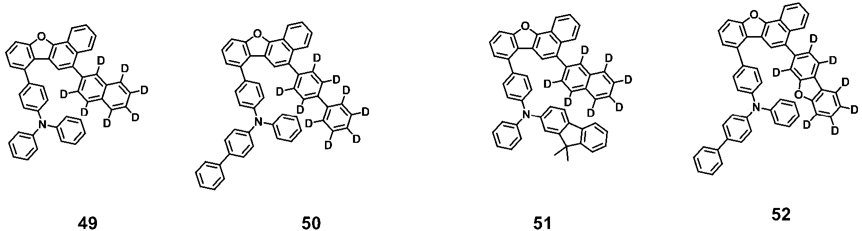

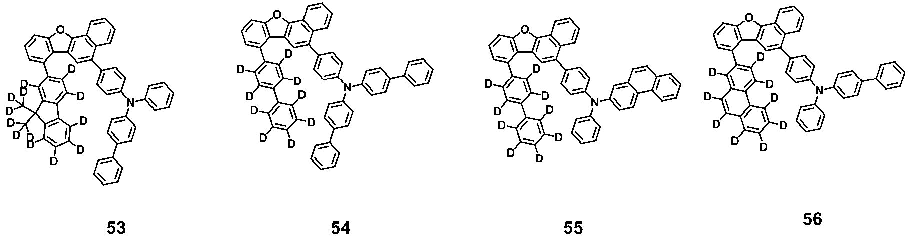

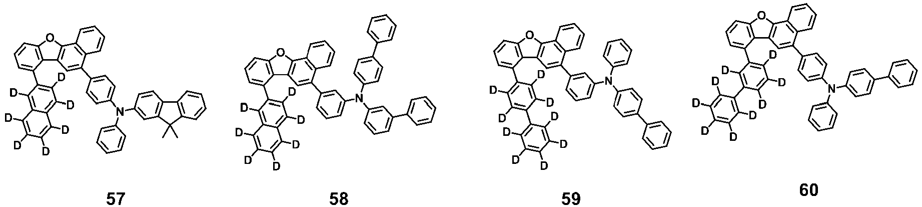

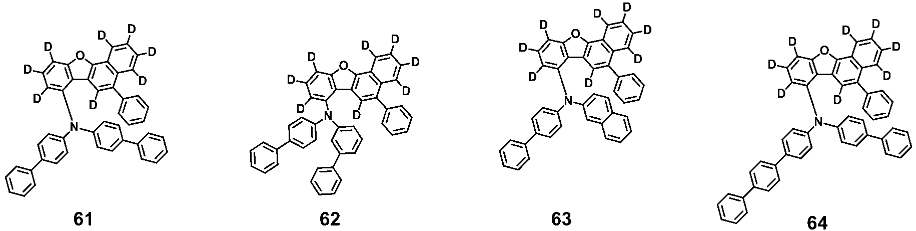

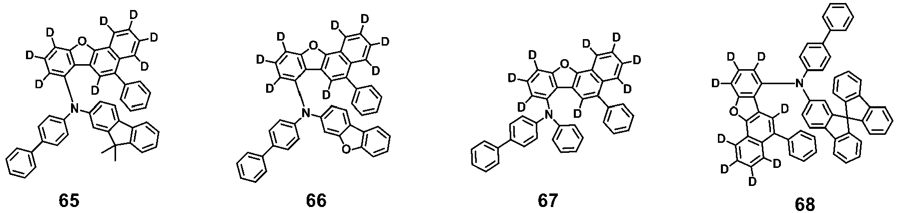

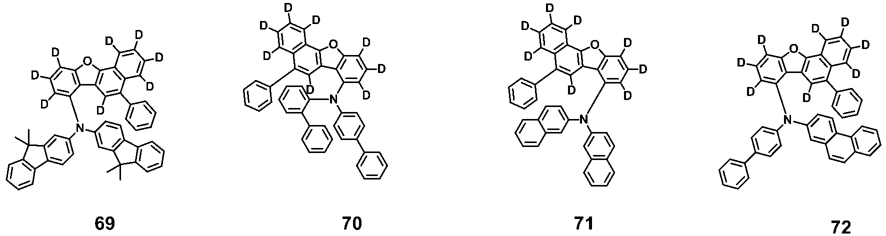

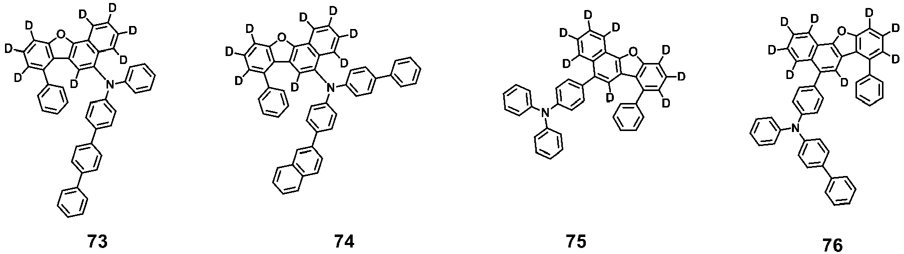

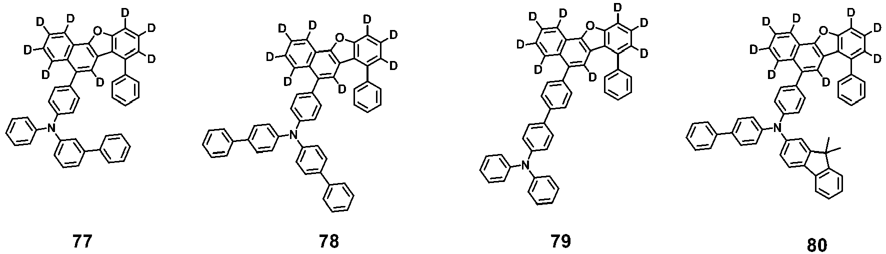

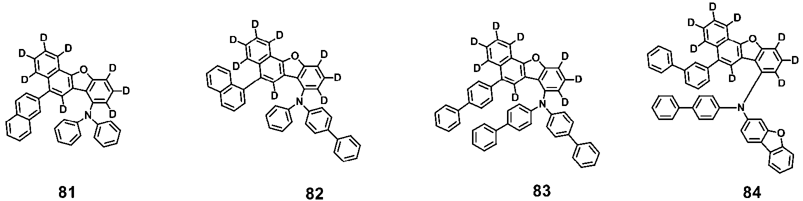

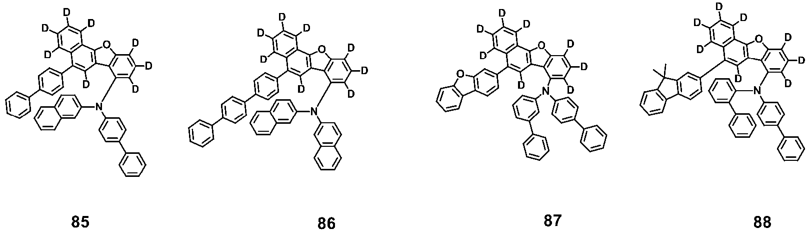

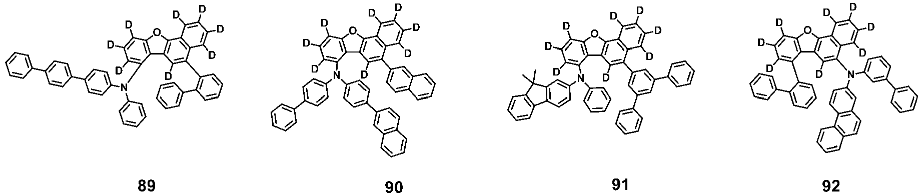

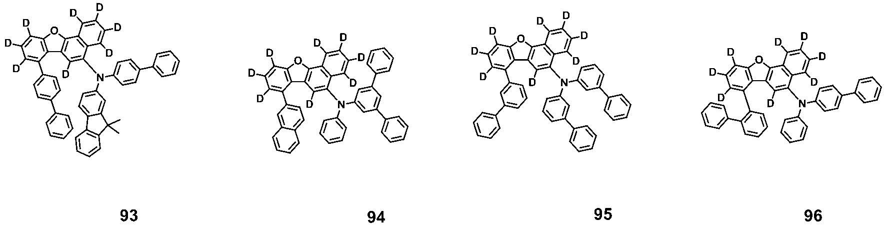

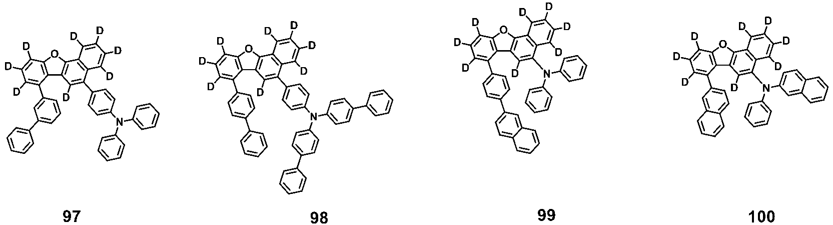

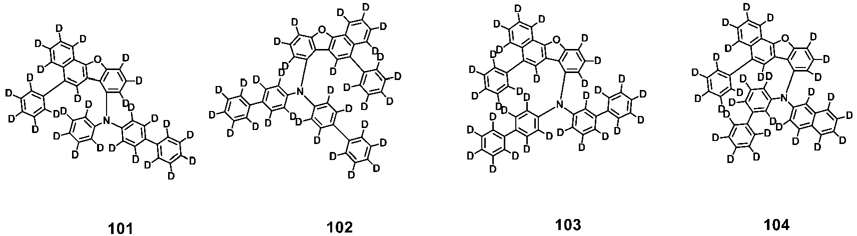

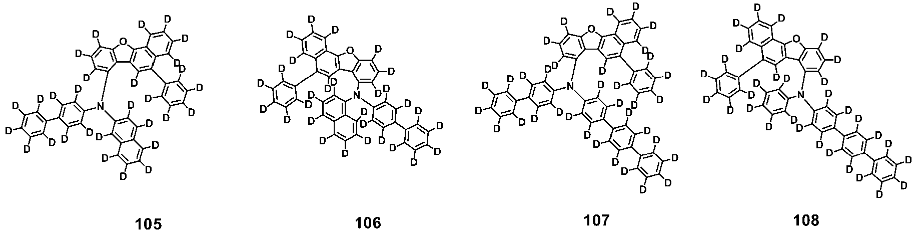

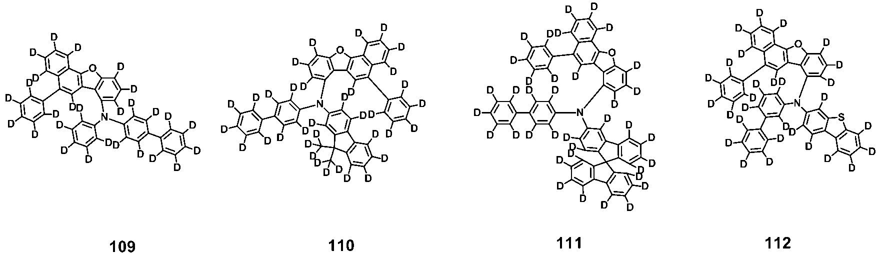

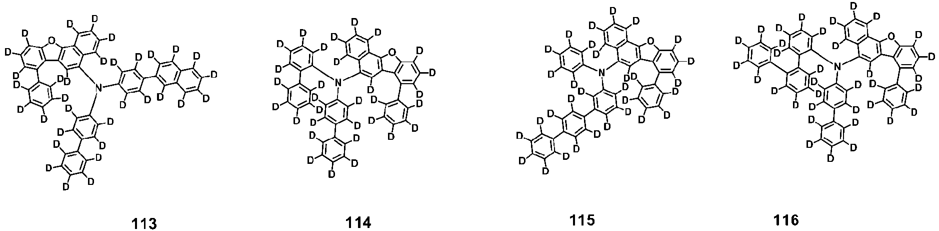

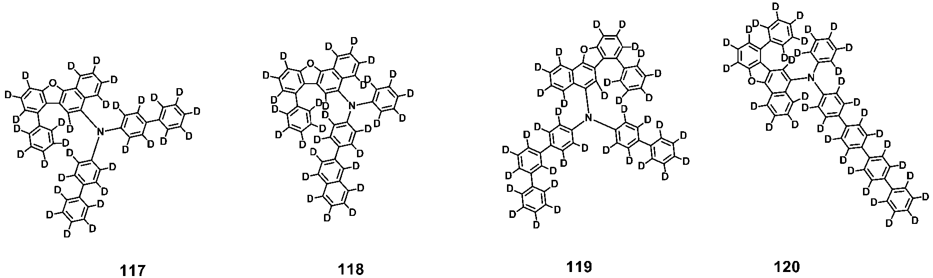

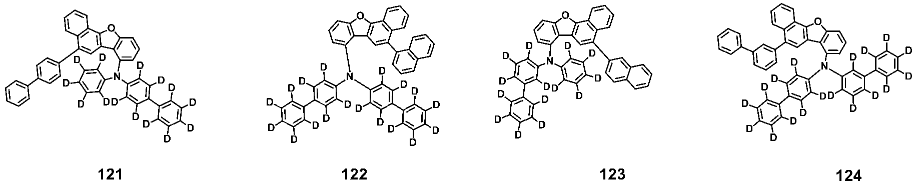

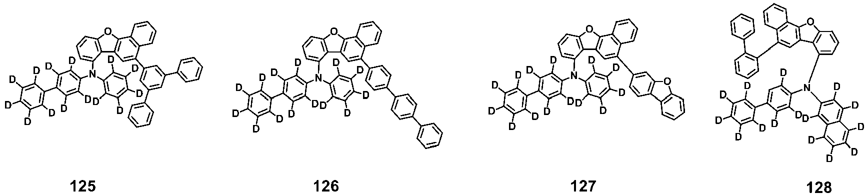

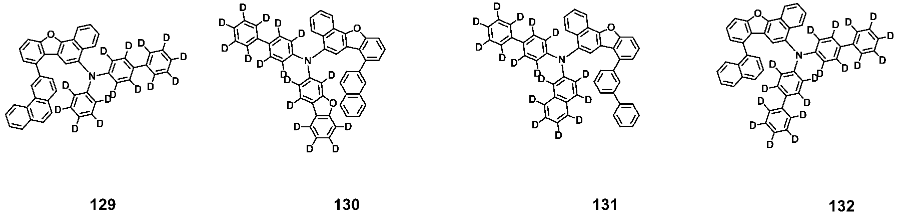

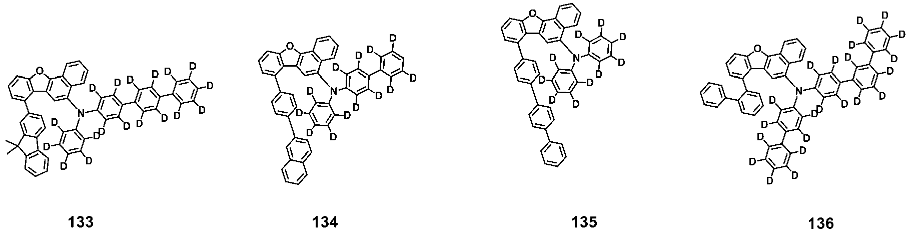

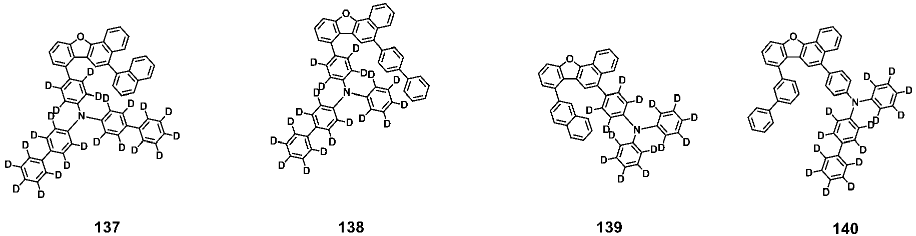

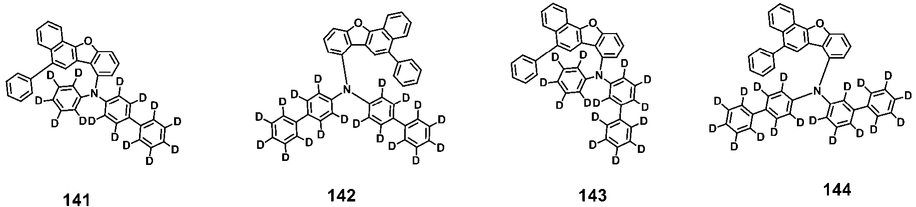

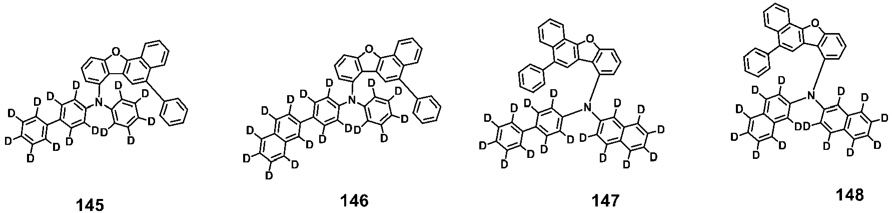

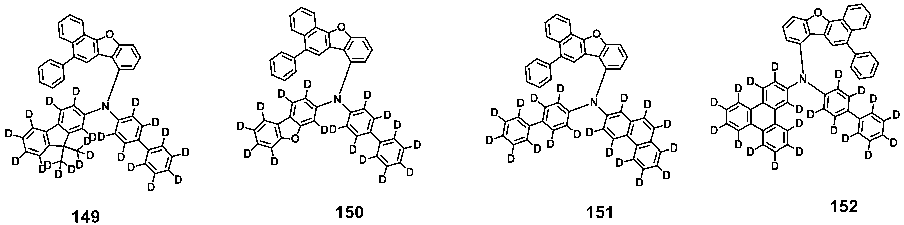

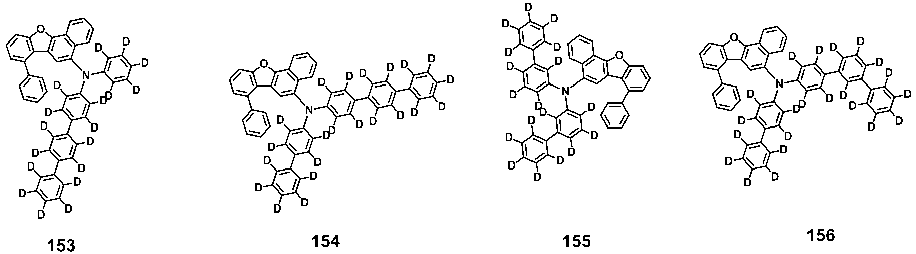

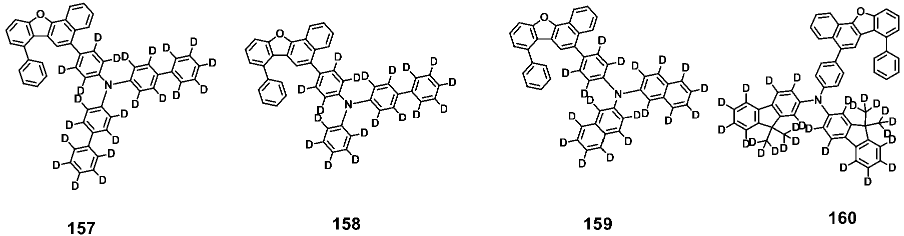

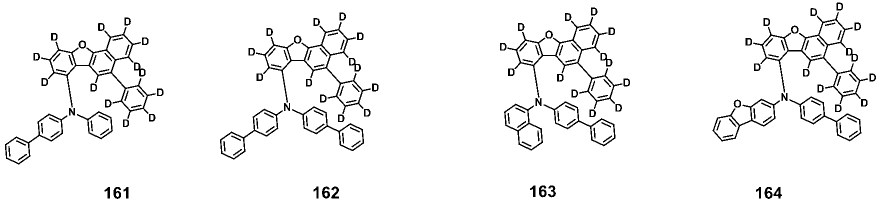

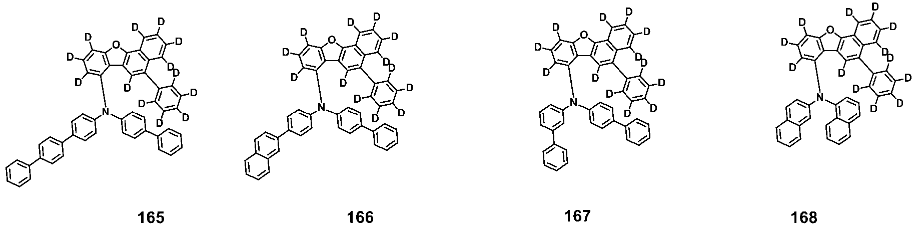

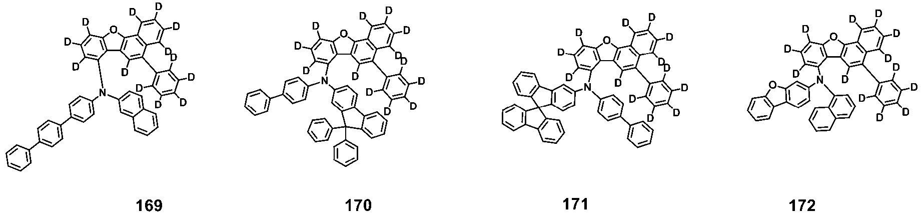

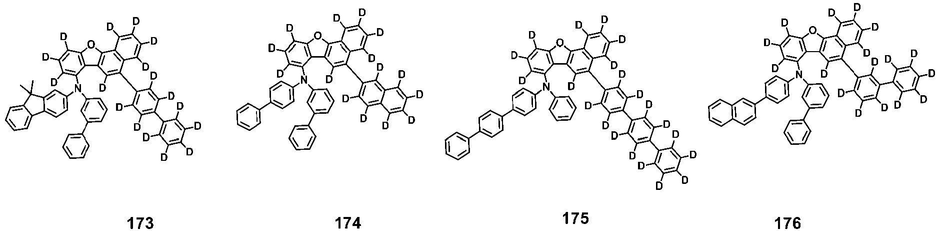

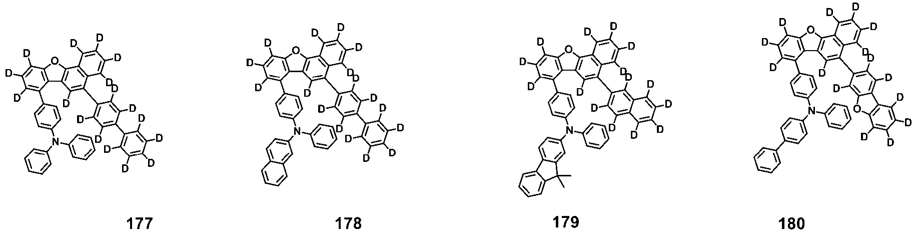

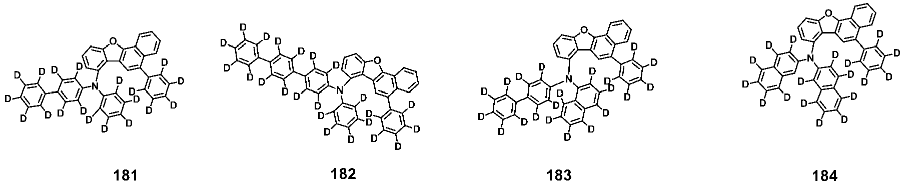

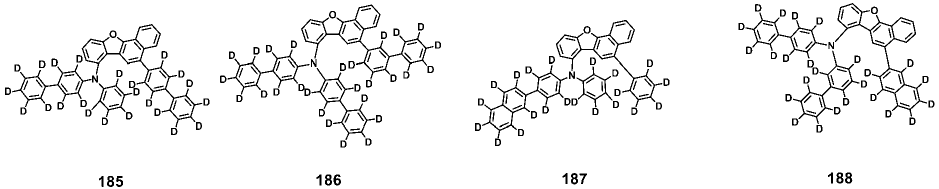

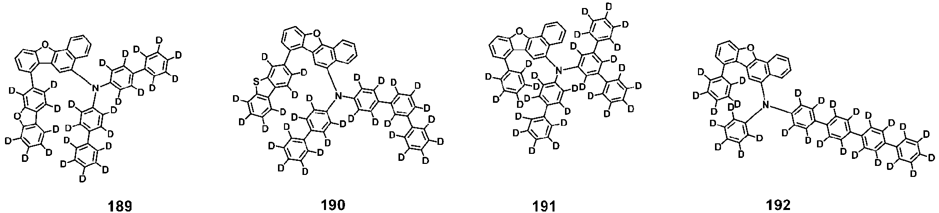

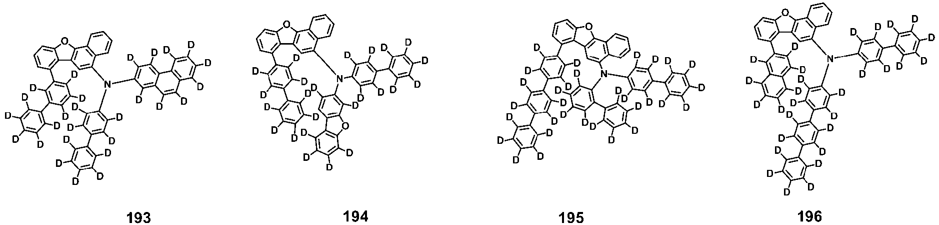

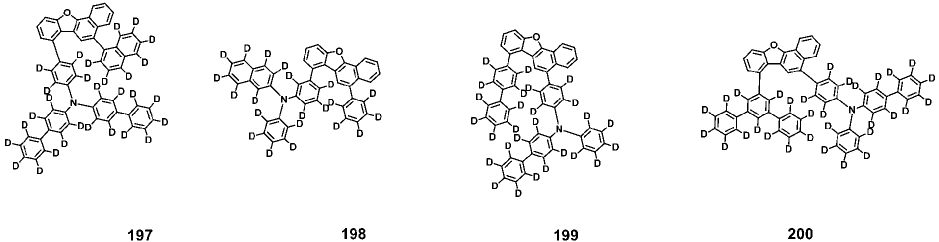

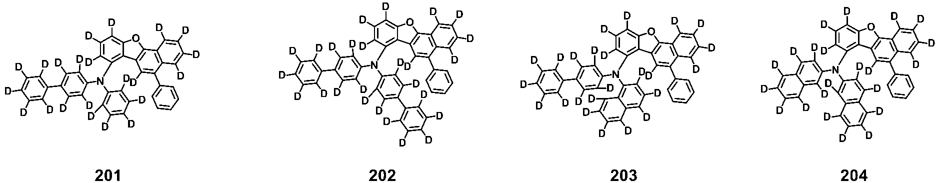

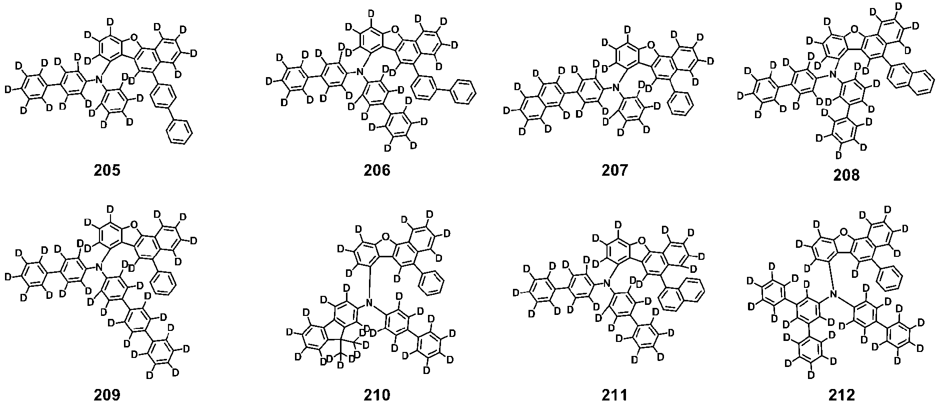

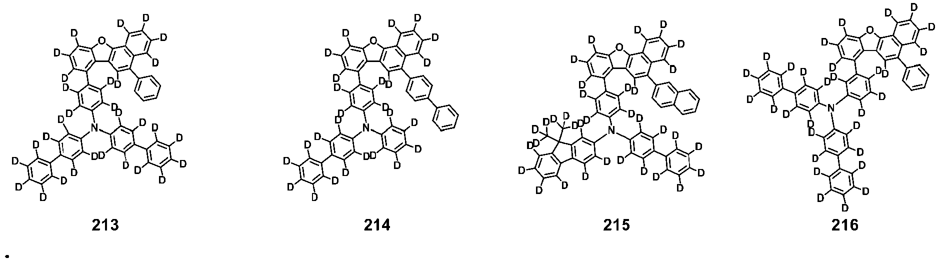

6. Organische lichtemittierende Vorrichtung, umfassend:
eine erste Elektrode;
eine zweite Elektrode, die so vorgesehen ist, dass sie der ersten Elektrode zugewandt ist; und
eine organische Materialschicht mit einer oder mehreren Schichten, die zwischen der ersten Elektrode und der zweiten Elektrode vorgesehen sind,
wobei eine oder mehrere Schichten der organischen Materialschicht die heterocyclische Verbindung nach einem der Ansprüche 1 bis 5 umfassen.

7. Organische lichtemittierende Vorrichtung nach Anspruch 6, wobei die organische Materialschicht eine lichtemittierende Hilfsschicht umfasst und die lichtemittierende Hilfsschicht die heterocyclische Verbindung umfasst.

8. Organische lichtemittierende Vorrichtung nach Anspruch 6, wobei die organische Materialschicht eine Elektroneninjektionsschicht oder eine Elektronentransportschicht umfasst und die Elektronentransportschicht oder die Elektroneninjektionsschicht die heterocyclische Verbindung umfasst.

9. Organische lichtemittierende Vorrichtung nach Anspruch 6, wobei die organische Materialschicht eine Elektronenblockierschicht oder eine Lochblockierschicht umfasst und die Elektronenblockierschicht oder die Lochblockierschicht die heterocyclische Verbindung umfasst.

10. Organische lichtemittierende Vorrichtung nach Anspruch 6, wobei die organische Materialschicht eine Lochtransportschicht umfasst und die Lochtransportschicht die heterocyclische Verbindung umfasst.

11. Organische lichtemittierende Vorrichtung nach Anspruch 6, ferner umfassend eine oder mehrere Schichten, die aus der Gruppe ausgewählt sind, die aus einer lichtemittierenden Schicht, einer Lochinjektionsschicht, einer Lochtransportschicht, einer Elektroneninjektionsschicht, einer Elektronentransportschicht, einer Elektronenblockierschicht und einer Lochblockierschicht besteht.

## Revendications

1. Composé hétérocyclique représenté par la Formule chimique ci-après 1 : 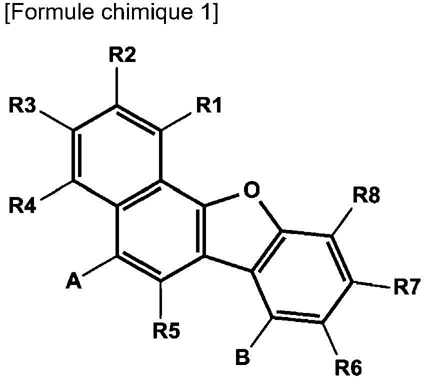 dans la Formule chimique 1,
R1 à R8 sont identiques ou différents les uns des autres, et sont chacun indépendamment sélectionnés dans le groupe constitué d'un hydrogène ; un deutérium ; un halogène ; un groupe cyano ; un groupe alkyle en C1 à C60 non substitué ou substitué ; un groupe alkényle en C2 à C60 non substitué ou substitué ; un groupe alkynyle en C2 à C60 non substitué ou substitué ; un groupe alkoxy en C1 à C60 non substitué ou substitué ; un groupe cycloalkyle en C3 à C60 non substitué ou substitué ; un groupe hétérocycloalkyle en C2 à C60 non substitué ou substitué ; un groupe aryle en C6 à C60 non substitué ou substitué ; un groupe hétéroaryle en C2 à C60 non substitué ou substitué ; -P(=O)RR' ; et -SiRR'R", ou deux groupes adjacents ou plus sont liés les uns avec les autres pour former un cycle hydrocarboné aromatique ou aliphatique en C6 à C60 non substitué ou substitué, ou un hétérocycle aromatique ou aliphatique en C2 à C60 non substitué ou substitué,
A et B sont chacun indépendamment représentés par la Formule chimique ci-après 1-1 ; ou la Formule chimique ci-après 1-2, 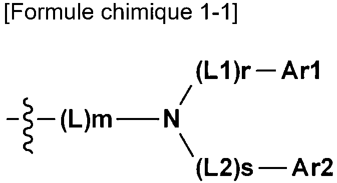 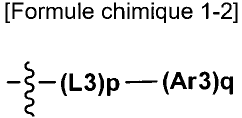
dans les Formules chimiques1-1 et 1-2,
L et L1 à L3 sont identiques ou différents les uns des autres, et sont chacun indépendamment une liaison directe ; un groupe arylène en C6 à C60 non substitué ou substitué ; ou un groupe hétéroarylène en C2 à C60 non substitué ou substitué,
Ar1 à Ar3 sont identiques ou différents les uns des autres, et sont chacun indépendamment un groupe alkyle en C1 à C60 non substitué ou substitué ; un groupe aryle en C6 à C60 non substitué ou substitué ; un groupe hétéroaryle en C2 à C60 non substitué ou substitué ; -P(=O)RR' ; ou -SiRR'R",
p, m, r et s sont chacun un nombre entier de 0 à 4, q est un nombre entier de 1 à 6,
quand p, m, r, s et q sont chacun 2 ou plus, les substituants entre parenthèses sont identiques ou différents les uns des autres,
une teneur en deutérium du composé hétérocyclique de la Formule chimique 1 est 1 % à 100 %, et
R, R' et R" sont identiques ou différents les uns des autres, et sont chacun indépendamment un groupe alkyle en C1 à C60 non substitué ou substitué ; un groupe aryle en C6 à C60 non substitué ou substitué ; ou un groupe hétéroaryle en C2 à C60 non substitué ou substitué.

2. Composé hétérocyclique selon la revendication 1, dans lequel la Formule chimique 1 est représentée par la Formule chimique 2 ci-après ou la Formule chimique 3 ci-après : 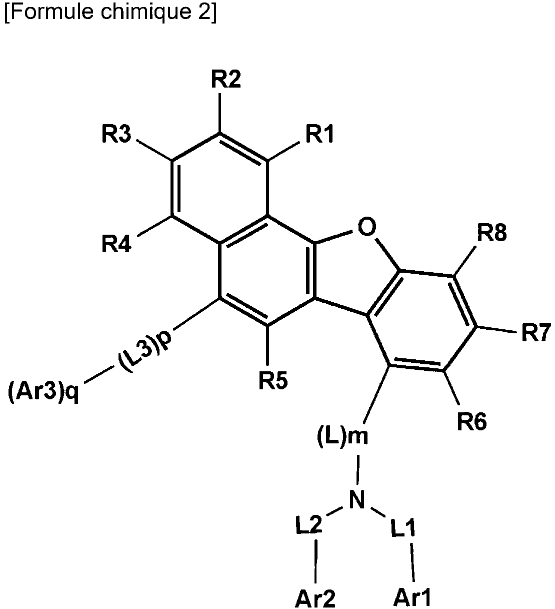 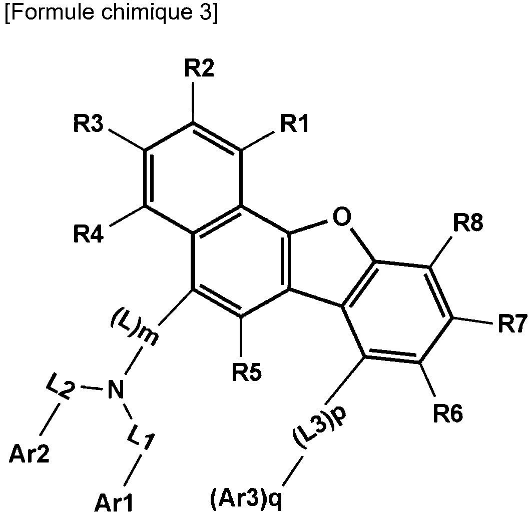
dans les Formules chimiques 2 et 3,
la définition de chaque substituant est identique à la définition dans la Formule chimique 1.

3. Composé hétérocyclique selon la revendication 1, dans lequel la teneur en deutérium du composé hétérocyclique de la Formule chimique 1 est 10 % à 80 %.

4. Composé hétérocyclique selon la revendication 1, dans lequel la Formule chimique 1 est exprimée tout en étant divisée en unités des Formules structurelles A à C ci-après, et
la teneur en deutérium de la Formule structurelle A ci-après ; la Formule structurelle B ci-après ; la Formule structurelle C ci-après ; la Formule structurelle A ci-après et la Formule structurelle B ci-après ; la Formule structurelle A ci-après et la Formule structurelle C ci-après ; la Formule structurelle B ci-après et la Formule structurelle C ci-après ; ou les Formules structurelles A à C ci-après dans la Formule chimique 1 est 1 % à 100 % : 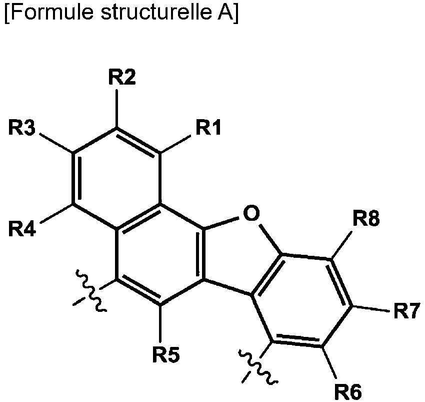 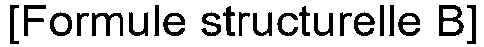 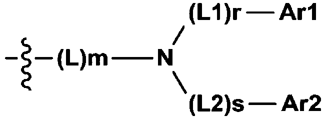 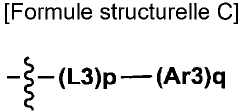
Dans les Formules structurelles A à C, la définition de chaque substituant est identique à la définition dans la Formule chimique 1, et 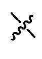 indique une position où les Formules structurelles A à C sont liées les unes aux autres.

5. Composé hétérocyclique selon la revendication 1, dans lequel la Formule chimique 1 est représentée par l'un des composés suivants :

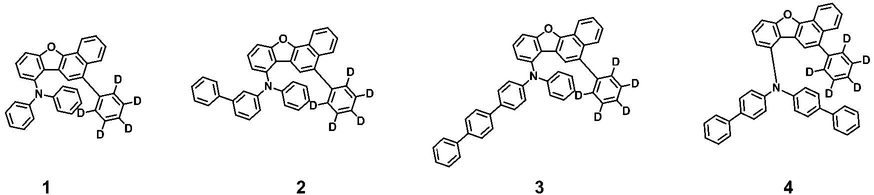

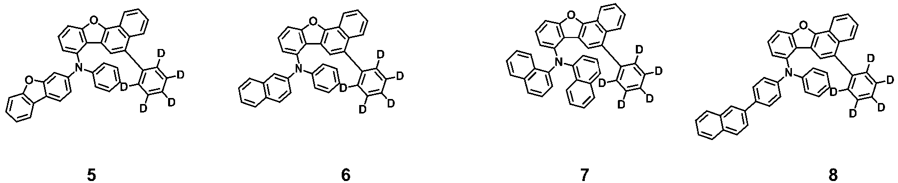

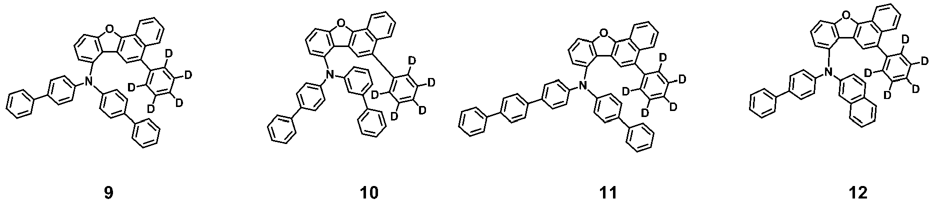

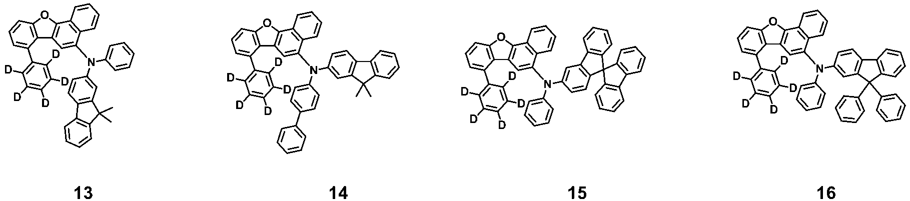

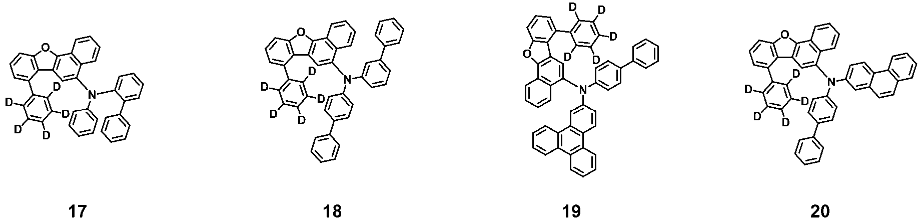

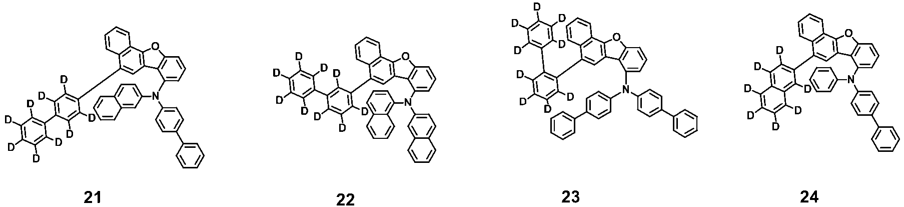

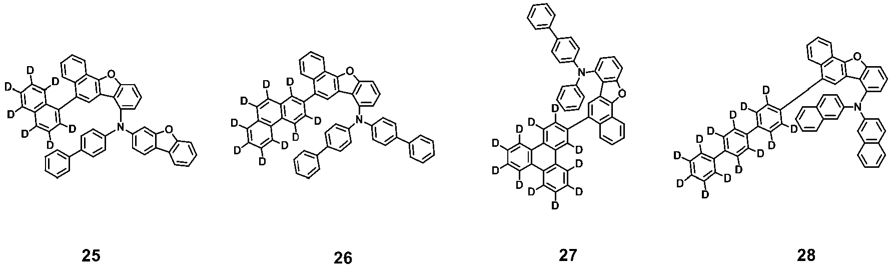

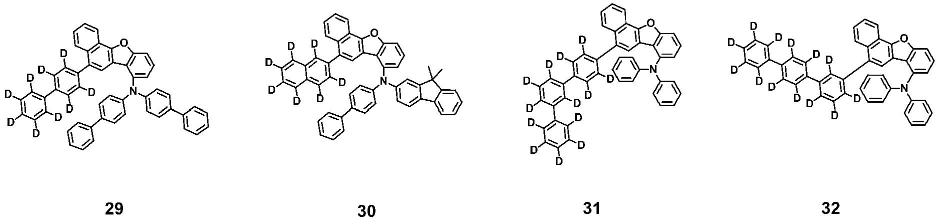

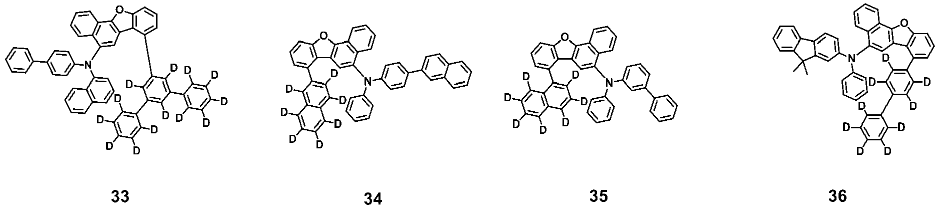

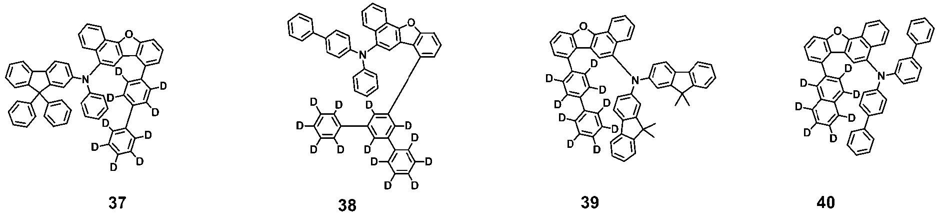

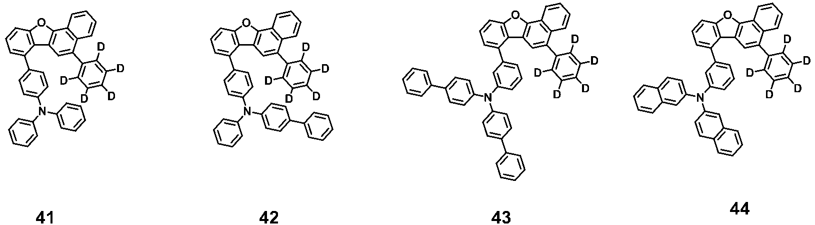

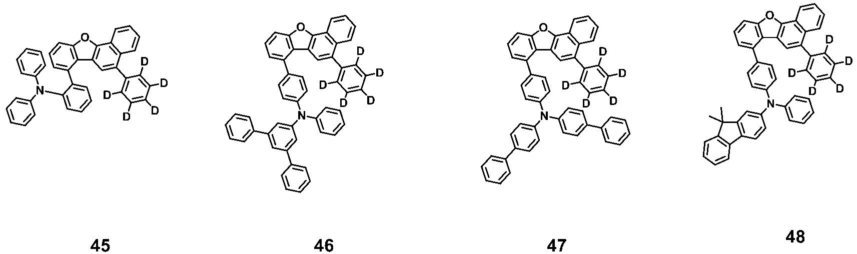

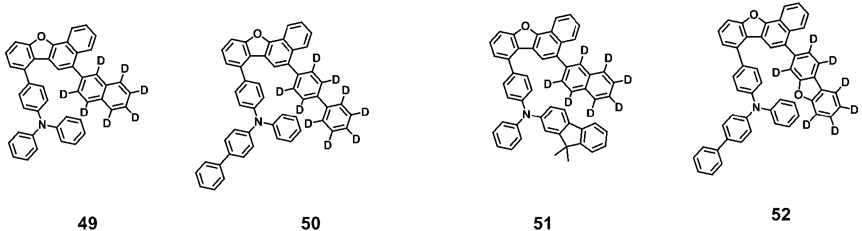

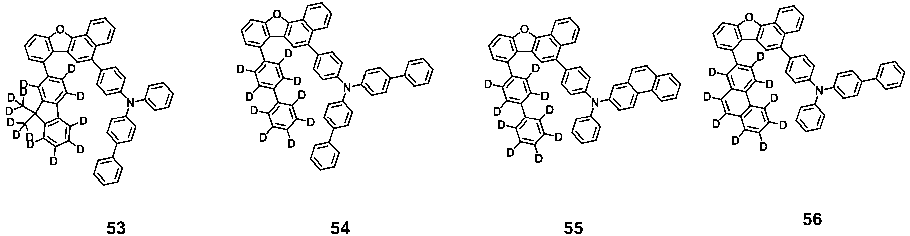

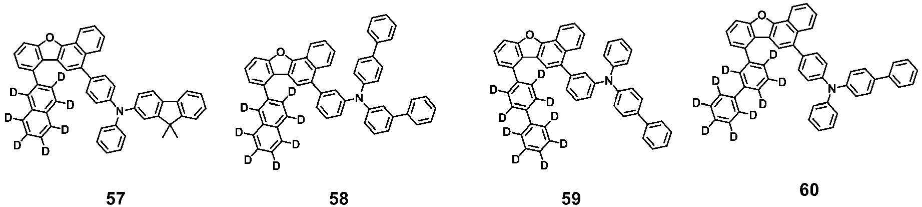

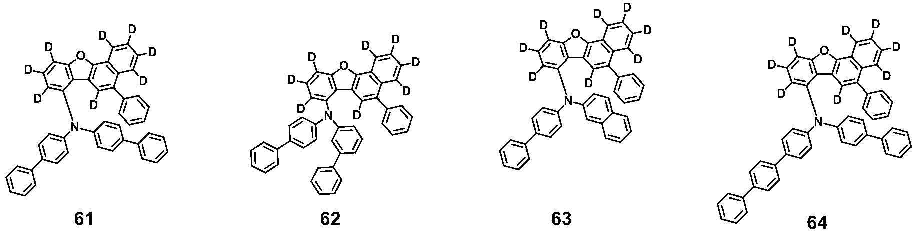

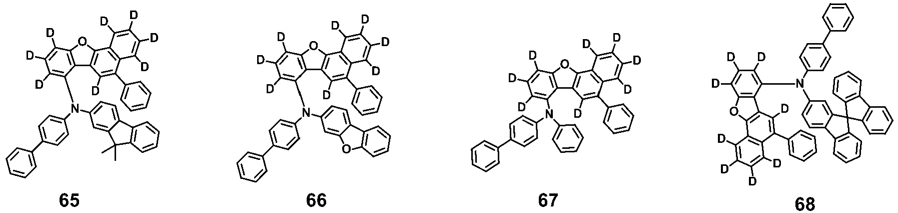

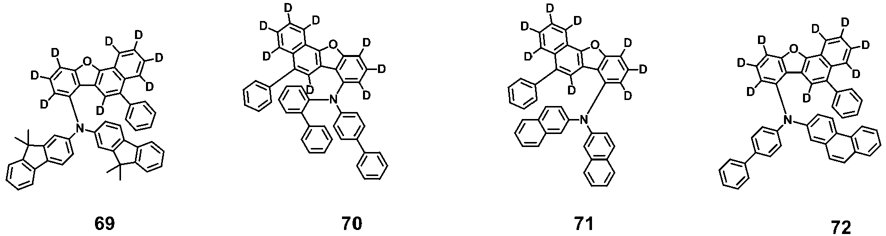

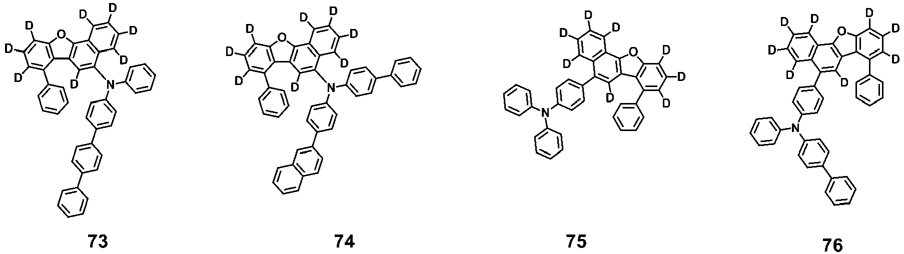

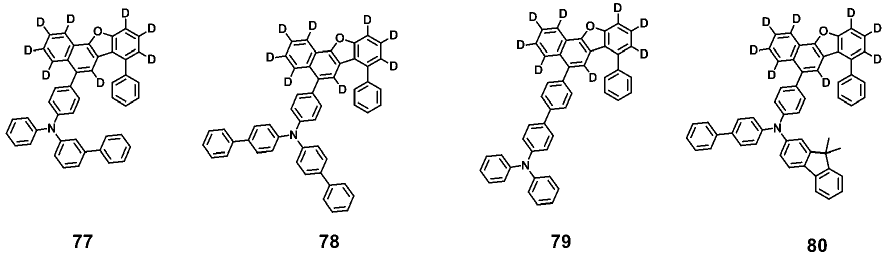

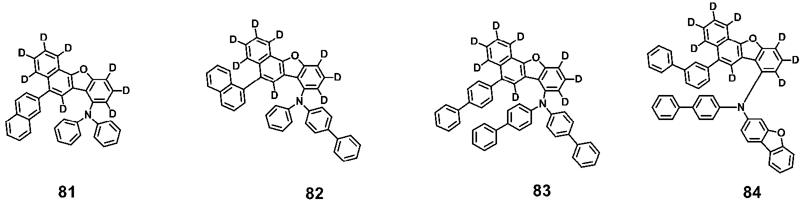

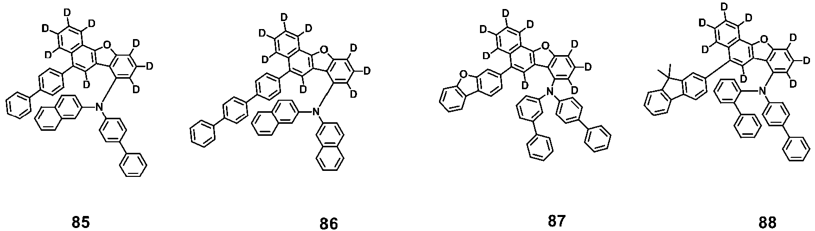

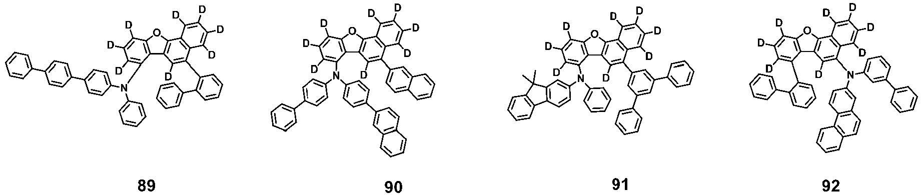

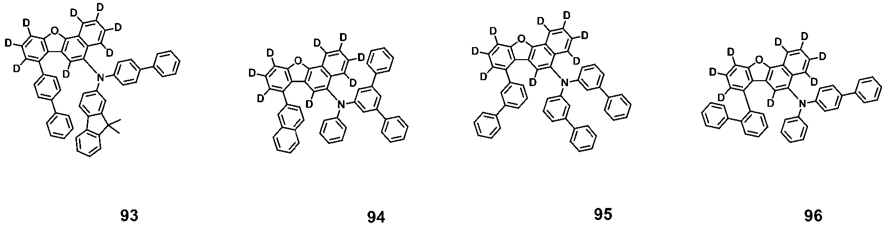

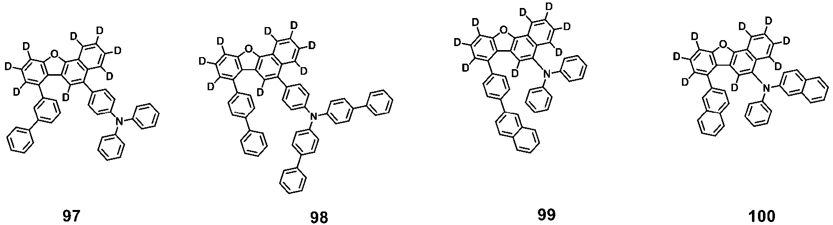

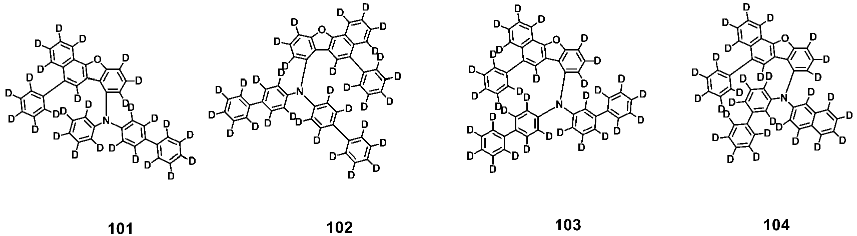

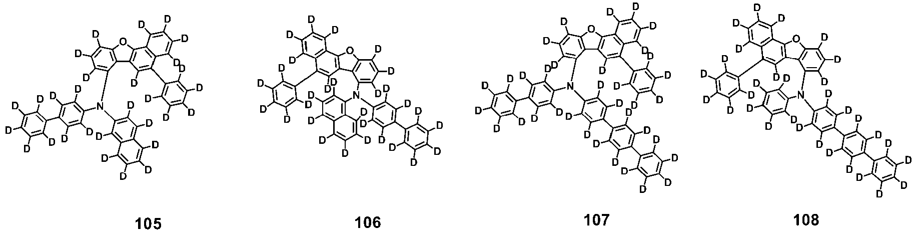

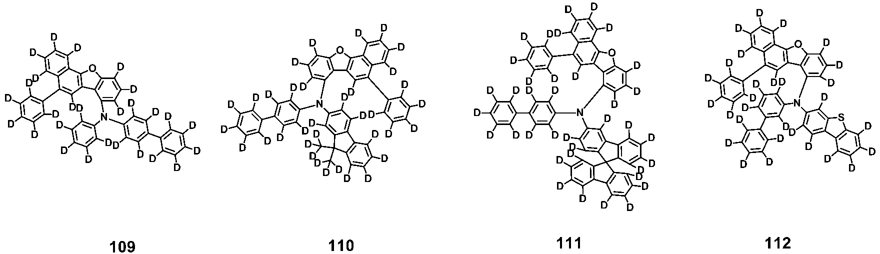

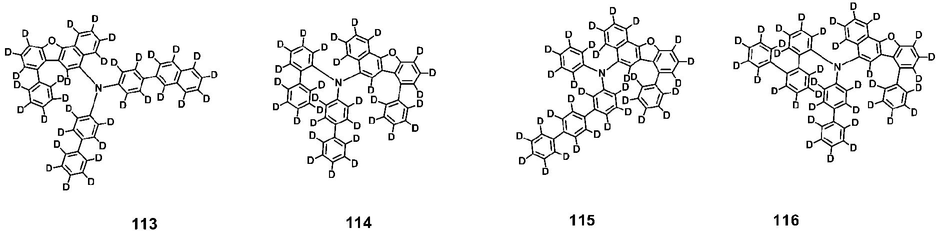

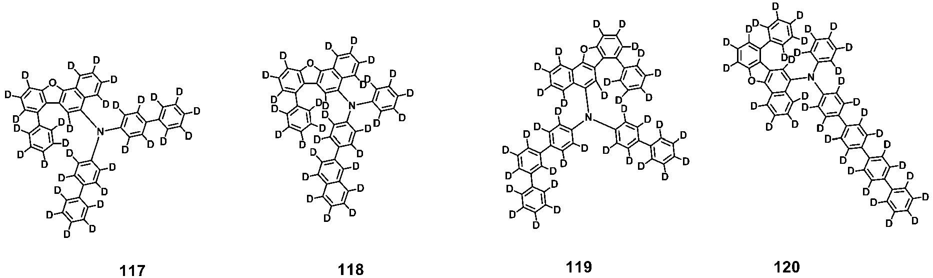

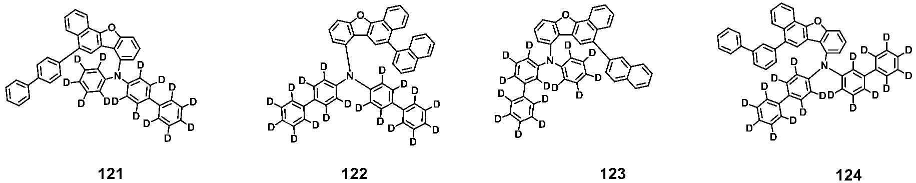

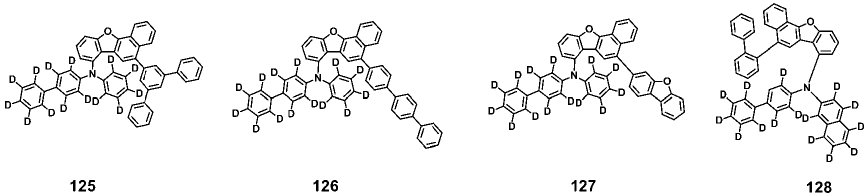

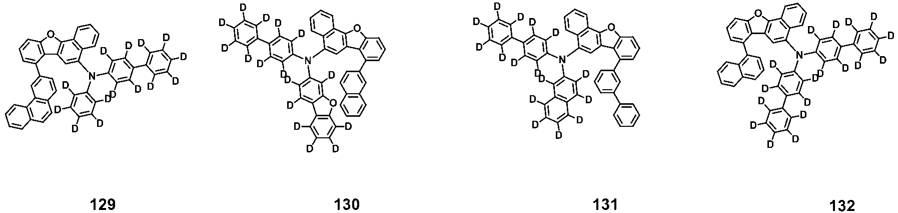

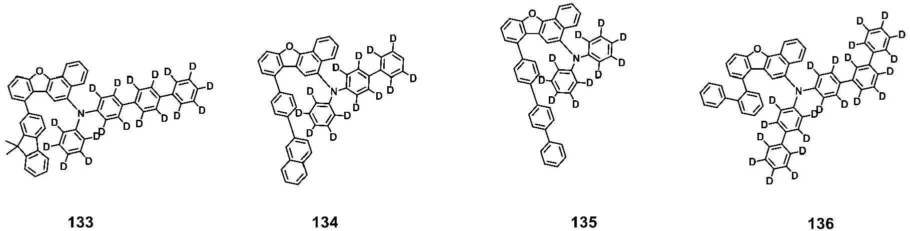

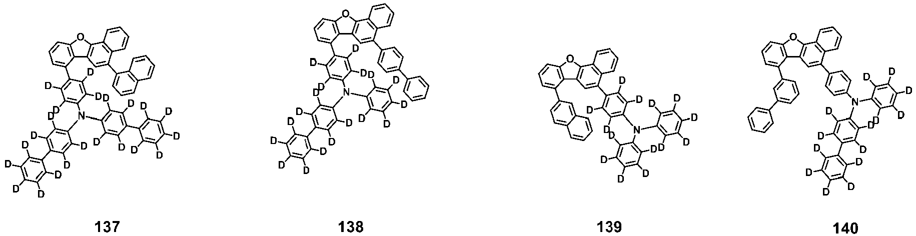

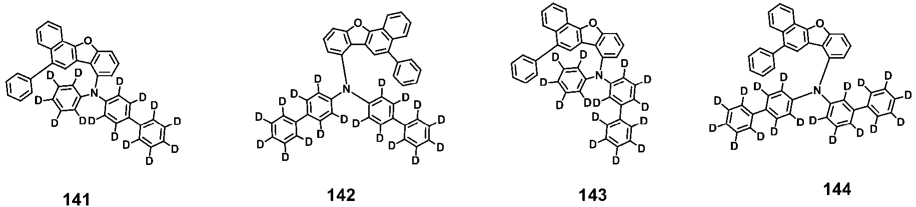

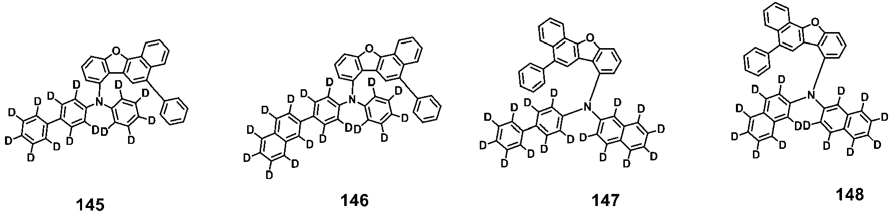

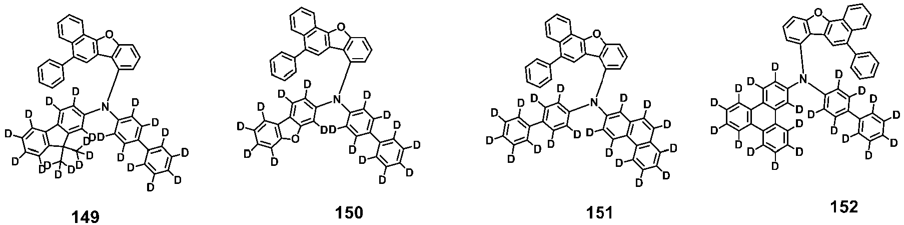

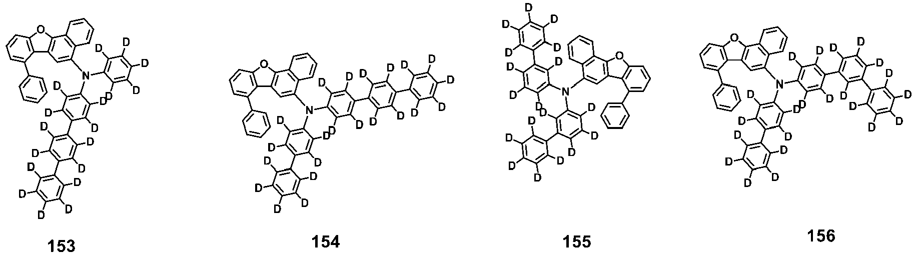

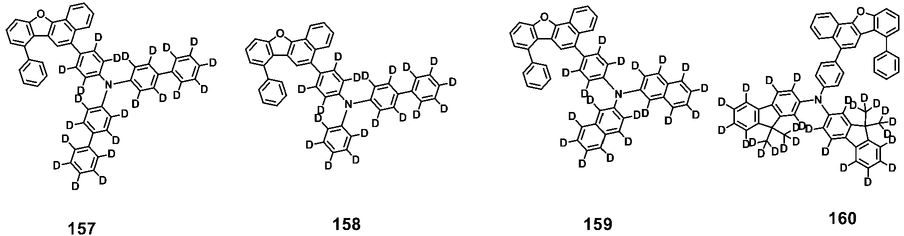

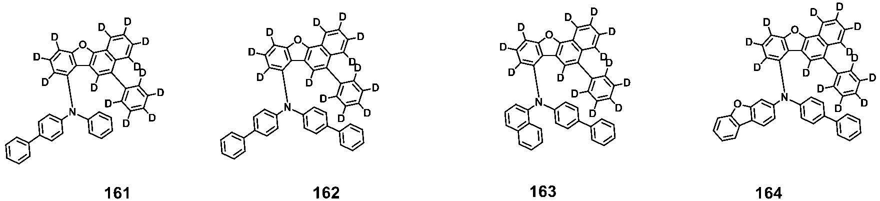

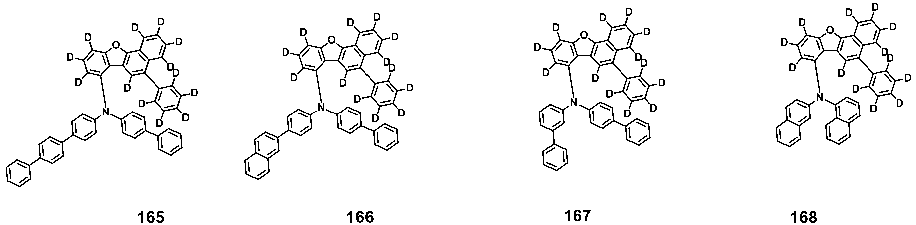

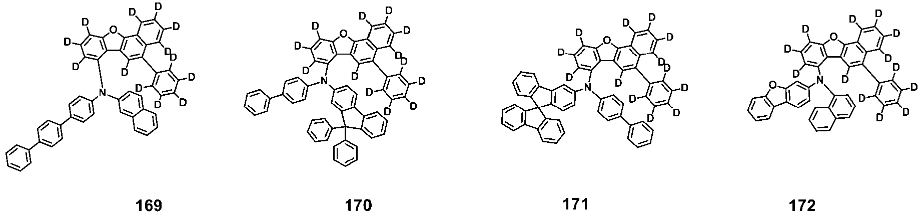

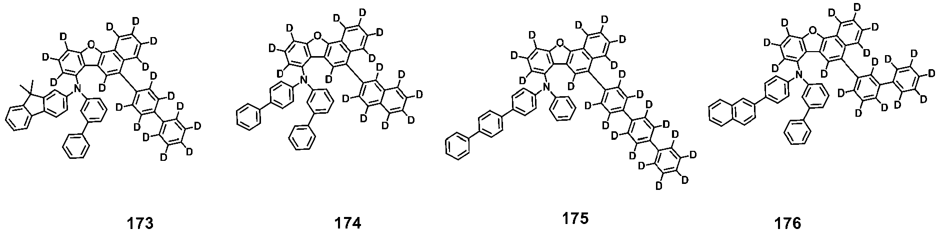

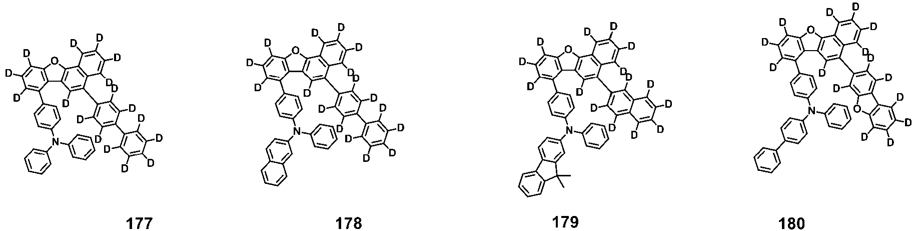

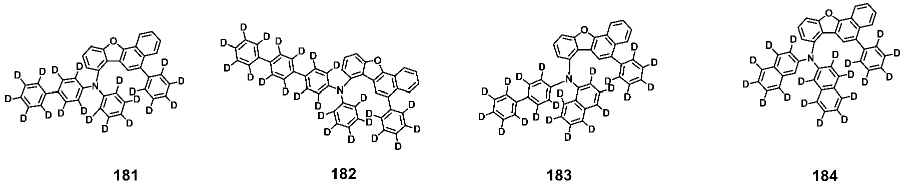

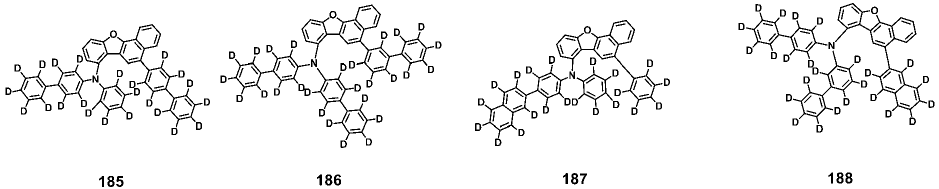

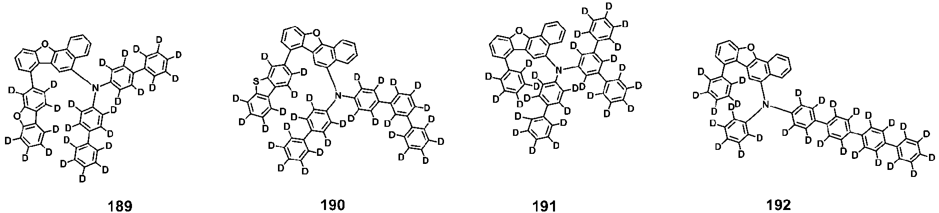

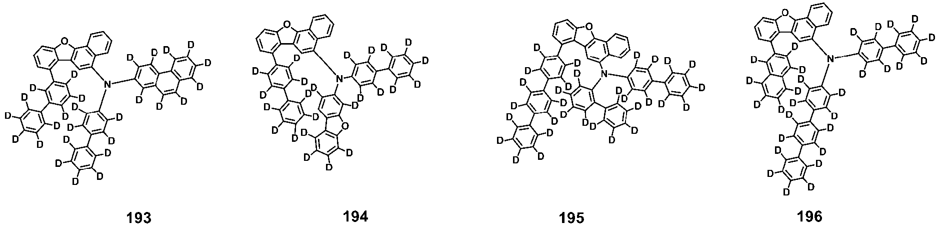

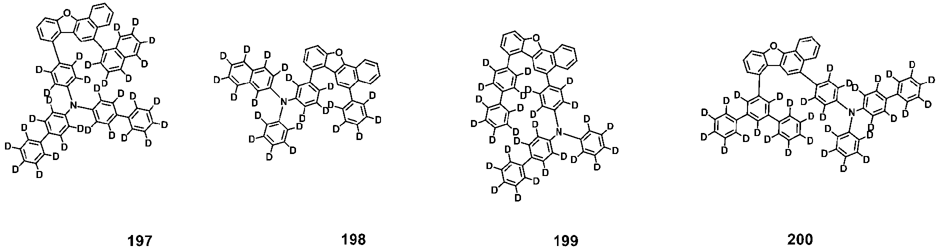

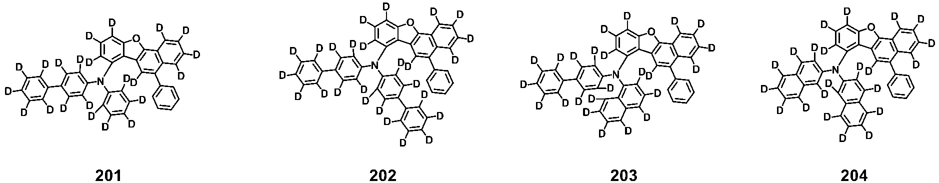

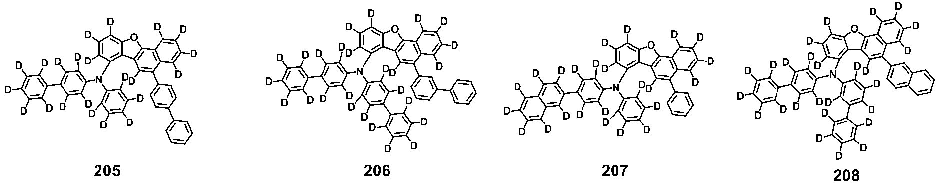

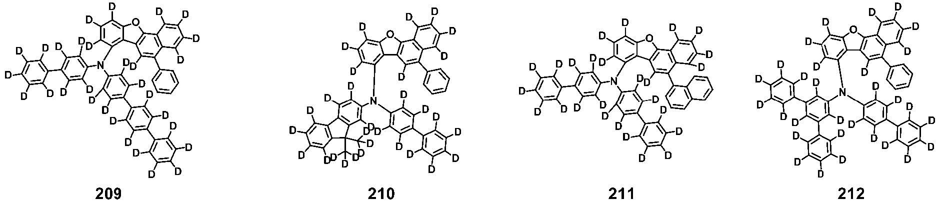

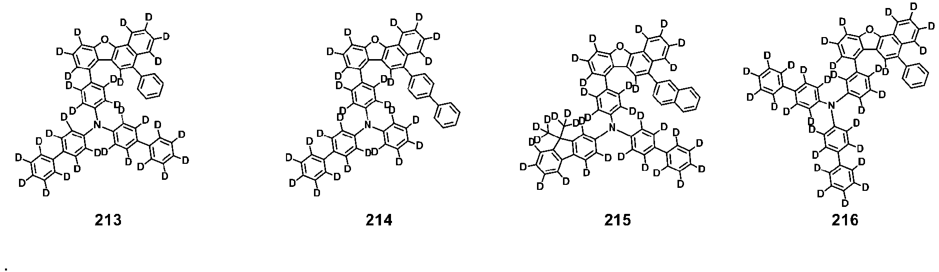

6. Dispositif électroluminescent organique comprenant :
une première électrode ;
une deuxième électrode fournie pour être face à la première électrode ; et
une couche de matériau organique ayant une ou plusieurs couches fournies entre la première électrode et la deuxième électrode,
dans lequel une ou plusieurs couches de la couche de matériau organique comprennent le composé hétérocyclique selon l'une quelconque des revendications 1 à 5.

7. Dispositif électroluminescent organique selon la revendication 6, dans lequel la couche de matériau organique comprend une couche électroluminescente auxiliaire, et la couche électroluminescente auxiliaire comprend le composé hétérocyclique.

8. Dispositif électroluminescent organique selon la revendication 6, dans lequel la couche de matériau organique comprend une couche d'injection d'électrons ou une couche de transfert d'électrons, et la couche de transfert d'électrons ou la couche d'injection d'électrons comprend le composé hétérocyclique.

9. Dispositif électroluminescent organique selon la revendication 6, dans lequel la couche de matériau organique comprend une couche de blocage d'électrons ou une couche de blocage de trous, et la couche de blocage d'électrons ou la couche de blocage de trous comprend le composé hétérocyclique.

10. Dispositif électroluminescent organique selon la revendication 6, dans lequel la couche de matériau organique comprend une couche de transfert de trous, et la couche de transfert de trous comprend le composé hétérocyclique.

11. Dispositif électroluminescent organique selon la revendication 6, comprenant en outre une ou plusieurs couches sélectionnées dans le groupe constitué d'une couche électroluminescente, une couche d'injection de trous, une couche de transfert de trous, une couche d'injection d'électrons, une couche de transfert d'électrons, une couche de blocage d'électrons et une couche de blocage de trous.
